(19) Europäisches Patentamt
European Patent Office
Office européen des brevets

(11) EP 3 880 284 B1

(12) # EUROPEAN PATENT SPECIFICATION

(45) Date of publication and mention of the grant of the patent:
**25.02.2026 Bulletin 2026/09**

(21) Application number: **19883367.5**

(22) Date of filing: **13.11.2019**

(51) International Patent Classification (IPC):
***A61M 16/16*** *(2006.01)* ***A61M 16/10*** *(2006.01)*
***A61M 16/00*** *(2006.01)*

(52) Cooperative Patent Classification (CPC):
**A61M 16/1045; A61M 16/109; A61M 16/1095; A61M 16/16;** A61M 16/0069; A61M 16/024; A61M 16/1065; A61M 16/107; A61M 16/161; A61M 2016/0027; A61M 2016/0033; A61M 2205/18; A61M 2205/3368; A61M 2205/42; A61M 2205/502;
(Cont.)

(86) International application number:
**PCT/IB2019/059741**

(87) International publication number:
**WO 2020/100054 (22.05.2020 Gazette 2020/21)**

(54) **HUMIDIFICATION WITH HME**

BEFEUCHTUNG MIT HME

HUMIDIFICATION AVEC ECH

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2018 US 201862767149 P**

(43) Date of publication of application:
**22.09.2021 Bulletin 2021/38**

(73) Proprietor: **ResMed Pty Ltd**
**Bella Vista, New South Wales 2153 (AU)**

(72) Inventors:
- **BATH, Andrew Roderick**
  **Bella Vista, New South Wales 2153 (AU)**
- **LAU, Wei Liang**
  **Bella Vista, New South Wales 2153 (AU)**
- **EBERL, Lorenz**
  **Bella Vista, New South Wales 2153 (AU)**
- **SHORT, Skye Kimberley**
  **Bella Vista, New South Wales 2153 (AU)**
- **STANISLAS, Luke Andrew**
  **Bella Vista, New South Wales 2153 (AU)**

(74) Representative: **Vossius & Partner**
**Patentanwälte Rechtsanwälte mbB**
**Siebertstraße 3**
**81675 München (DE)**

(56) References cited:

| | |
|---|---|
| **EP-A1- 3 360 594** | **WO-A1-2014/138820** |
| **WO-A1-2017/004664** | **WO-A1-2018/035579** |
| **WO-A1-91/19527** | **GB-A- 2 277 689** |
| **US-A1- 2012 304 985** | **US-A1- 2016 175 552** |
| **US-A1- 2018 001 047** | **US-A1- 2018 264 222** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
A61M 2205/702

## Description

2 BACKGROUND OF THE TECHNOLOGY

2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use. The invention relates to a CPAP system.

2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See "Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient $CO_2$ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapy

Various therapies, such as Continuous Positive Airway Pressure (CPAP) therapy, Non-invasive ventilation (NIV) and Invasive ventilation (IV) have been used to treat one or more of the above respiratory disorders.

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.3 Treatment Systems

These therapies may be provided by a treatment system or device. Such systems and devices may also be used to screen, diagnose, or monitor a condition without treating it.

A treatment system may comprise a Respiratory Pressure Therapy Device (RPT device), an air circuit, a humidifier, a patient interface, and data management.

Another form of treatment system is a mandibular repositioning device.

#### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

#### *2.2.3.1.1 Seal-forming structure*

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Limited, describe examples of nasal pillows masks: International Patent Application WO2004/073,778 (describing amongst other things aspects of the ResMed Limited SWIFT™ nasal pillows), US Patent Application 2009/0044808 (describing amongst other things aspects of the ResMed Limited SWIFT™ LT nasal pillows); International Patent Applications WO 2005/063,328 and WO 2006/130,903 (describing amongst other things aspects of the ResMed Limited MIRAGE LIBERTY™ full-face mask); International Patent Application WO 2009/052,560 (describing amongst other things aspects of the ResMed Limited SWIFT™ FX nasal pillows).

#### *2.2.3.1.2 Positioning and stabilising*

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressurised. Examples of RPT devices include a CPAP device and a ventilator.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

Table of noise output levels of prior RPT devices (one specimen only, measured using test method specified in ISO 3744 in CPAP mode at 10 $cmH_2O$).

| RPT Device name | A-weighted sound pressure level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

#### 2.2.3.3 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air.

A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient’s surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

#### 2.2.3.4 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient’s compliance, a provider of the RPT device, such as a health care provider, may manually obtain data describing the patient’s therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient’s therapy that would benefit from communication of therapy data to a third party or external system.

Existing processes to communicate and manage such data can be one or more of costly, time-consuming, and error-prone.

#### 2.2.3.5 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient’s teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient’s mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC™ MRD are designed to retain the mandible in a forward position. This device also reduces or minimises dental and temporo-mandibular joint (TMJ) side effects. Thus, it is configured to minimises or prevent any movement of one or more of the teeth.

**2.2.3.6 Vent technologies**

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

Table of noise of prior masks (ISO 17510-2:2007, 10 $cmH_2O$ pressure at 1m)

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage™ | nasal | 36 (3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage™ SoftGel | nasal | 29 (3) | 22 (3) | 2008 |
| ResMed Mirage™ FX | nasal | 26 (3) | 18 (3) | 2010 |
| ResMed Mirage Swift™ (*) | nasal pillows | 37 | 29 | 2004 |
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |
| (* one specimen only, measured using test method specified in ISO 3744 in CPAP mode at 10 $cmH_2O$) | | | | |

Sound pressure values of a variety of objects are listed below

| Object | A-weighted sound pressure dB(A) | Notes |
|---|---|---|
| Vacuum cleaner: Nilfisk<br>Walter Broadly Litter Hog: B+ Grade | 68 | ISO 3744 at 1m distance |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

WO 2017/004664 A1 relates to a system for heating a respiratory conduit positioned between a PAP device and a patent interface, wherein the system comprises: a first thermistor encapsulated within the conduit proximal to a first end of the conduit which is proximal to the patient interfaces; a second thermistor encapsulated within the conduit proximal to a second end of the conduit which is proximal to the PAP device; a heater is encapsulated within the conduit; and wherein the first and second thermistor are electrically isolated from the heater within the conduit.

EP 3 360 594 A1 relates to a system for humidifying ventilation gas, the system comprising: a ventilator; a patient circuit in fluid communication with the ventilator at a proximal end and fluidly connected to a patient airway at a distal end, wherein the patient circuit has an inner diameter of approximatelymm; a fluid reservoir; a humidification device; and a channel between the humidification device and a distal end of the patient circuit.

US 2012/304985 A1 relates to a continuous positive airway pressure (CPAP) system providing positive airway pressure therapy.

GB 2277689 A relates to a filter or heat and moisture exchange device (HME) has a housing with a separate heaterretained on the housing by a couplingengaging a flangeon the heater. The heater has a helical wire heating element and a layer of insulating materialon an outside surface and warms the internal surface of the wall of the housing to reduce condensation.

WO 2018/035579 A1 relates to an apparatus for treating a respiratory disorder in a patient, the apparatus comprising: a respiratory pressure therapy device configured to generate a flow of air for treating the respiratory disorder, said flow of air being at a positive pressure with respect to ambient pressure; an air circuit adapted to transport said flow of air generated by said respiratory pressure therapy device to a patient interface, said air circuit having a proximal end connectable to said respiratory pressure therapy device and a distal end connectable to said patient interface; a nebuliser module located at or adjacent to said proximal end of said air circuit, said nebuliser module adapted to nebulise a liquid to form a nebula of said liquid, and to admit said nebula into said flow of air generated by said respiratory pressure therapy device; and a vaporiser located at said distal end of said air circuit, said vaporiser adapted to receive said nebula and further adapted to vaporise said nebula to form a humidified flow of air.

## 3 BRIEF SUMMARY OF THE TECHNOLOGY

The invention is set out in the appended claims. The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

Another aspect of the present technology relates to a CPAP system for providing air at positive pressure for respiratory therapy to a patient includes an RPT device configured to supply a flow of air at a therapeutic pressure, a patient interface forming a plenum chamber pressurizable to the therapeutic pressure, the patient interface including a seal-forming structure constructed and arranged to form a seal with a region of a patient's face surrounding an entrance to a patient's airways, an air delivery tube configured to pass the flow of air at the therapeutic pressure from the RPT device to the patient interface, a heat and moisture exchanger (HME) constructed and arranged to retain moisture from a flow of expiratory air from the patient, wherein retained moisture from the HME is returned to the flow of air for humidification, a controllable heater configured and arranged to heat the HME and/or heat incoming air to the HME, and a controller configured to adjust heating of the controllable heater to adjust the moisture returned from the HME to the flow of air for humidification.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

### 4.1 TREATMENT SYSTEMS

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

### 4.3 PATIENT INTERFACE

Fig. 3A shows a patient interface in the form of a nasal mask in accordance with one form of the present technology.

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.

Fig. 3C shows a schematic of a cross-section through a structure at a point. An outward normal at the point is indicated. The curvature at the point has a positive sign, and a relatively small magnitude when compared to the magnitude of the curvature shown in Fig. 3B.

Fig. 3D shows a schematic of a cross-section through a structure at a point. An outward normal at the point is indicated. The curvature at the point has a value of zero.

Fig. 3E shows a schematic of a cross-section through a structure at a point. An outward normal at the point is indicated. The curvature at the point has a negative sign, and a relatively small magnitude when compared to the magnitude of the curvature shown in Fig. 3F.

Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

Fig. 3G shows a cushion for a mask that includes two pillows. An exterior surface of the cushion is indicated. An edge of the surface is indicated. Dome and saddle regions are indicated.

Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

Fig. 3I shows the surface of a structure, with a one dimensional hole in the surface. The illustrated plane curve forms the boundary of a one dimensional hole.

Fig. 3J shows a cross-section through the structure of Fig.3I. The illustrated surface bounds a two dimensional hole in the structure of Fig. 3I.

Fig. 3K shows a perspective view of the structure of Fig. 3I, including the two dimensional hole and the one dimensional hole. Also shown is the surface that bounds a two dimensional hole in the structure of Fig. 3I.

Fig. 3L shows a mask having an inflatable bladder as a cushion.

Fig. 3M shows a cross-section through the mask of Fig. 3L, and shows the interior surface of the bladder. The interior surface bounds the two dimensional hole in the mask.

Fig. 3N shows a further cross-section through the mask of Fig. 3L. The interior surface is also indicated.

Fig. 3O illustrates a left-hand rule.

Fig. 3P illustrates a right-hand rule.

Fig. 3Q shows a left ear, including the left ear helix.

Fig. 3R shows a right ear, including the right ear helix.

Fig. 3S shows a right-hand helix.

Fig. 3T shows a view of a mask, including the sign of the torsion of the space curve defined by the edge of the sealing membrane in different regions of the mask.

Fig. 3U shows a view of a plenum chamber 3200 showing a sagittal plane and a mid-contact plane.

Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.

Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face" when the plenum chamber is in position for use. In Fig. 3X the plenum chamber 3200 is that of a nasal mask, and the superior point 3220 sits approximately on the sellion, while the inferior point 3230 sits on the lip superior.

4.4 BREATHING WAVEFORMS

Fig. 4 shows a model typical breath waveform of a person while sleeping.

4.5 RPT DEVICE AND HUMIDIFIER

Fig. 5A shows an exploded perspective view of an RPT device 4000 in accordance with one form of the present technology.

Fig. 5B shows a perspective view of an RPT device 4000 comprising an outlet muffler 4124 in accordance with one form of the present technology.

Fig. 5C shows a perspective view of an RPT device 4000 with an integrated humidifier 5000 comprising a water reservoir 5110 in accordance with one form of the present technology.

Fig. 5D is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

Fig. 5E is a schematic diagram of the electrical components of an RPT device in accordance with one form of the present technology.

Fig. 5F is a schematic diagram of the algorithms implemented in an RPT device in accordance with one form of the present technology.

Fig. 5G shows a schematic of a humidifier in accordance with one form of the present technology.

4.6 CPAP SYSTEM WITH HME AND HEATED AIR DELIVERY TUBE

Fig. 6 is a perspective view of a CPAP system including an RPT device, a heated air delivery tube, a patient interface, and an adaptor with an HME according to an example of the present technology.

Fig. 7 is a top view showing the heated air delivery tube and the adaptor according to an example of the present technology.

Fig. 8 is a cross-sectional view of the heated air delivery tube and the adaptor according to an example of the present technology.

Fig. 9 is a cross-sectional view showing a thermistor provided within a cuff of the heated air delivery tube according to an example of the present technology.

Fig. 10 is a top view of the patient interface and the adaptor according to an example of the present technology.

Fig. 11 is a cross-sectional view of the patient interface and the adaptor according to an example of the present technology.

Fig. 12 is a cross-sectional view showing a pressure sensor within the RPT device according to an example of the present technology.

Fig. 13 is a cross-sectional view showing a flow sensor within the RPT device according to an example of the present technology.

Fig. 14 is a perspective view of a CPAP system including an RPT device, a heated air delivery tube, and a patient interface with an HME according to an example of the present technology.

Fig. 15 is a cross-sectional view of the patient interface with an HME according to an example of the present technology.

Fig. 16 is a perspective view of a CPAP system including an RPT device, a humidifier, a heated air delivery tube, a patient interface, and an adaptor with an HME according to an example of the present technology.

Fig. 17 is a chart showing HME temperature vs. Dew Point for heated tube and non-heated tube arrangements according to an example of the present technology.

Figs. 18 to 33 are flowcharts showing control algorithms according to examples of the present technology.

## 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising the step of applying positive pressure to the entrance of the airways of a patient 1000.
In certain examples of the present technology, a supply of air at positive pressure is provided to the nasal passages of the patient via one or both nares.
In certain examples of the present technology, mouth breathing is limited, restricted or prevented.

### 5.2 TREATMENT SYSTEMS

In one form, the present technology comprises an apparatus or device for treating a respiratory disorder. The apparatus or device may comprise an RPT device 4000 for supplying pressurised air to the patient 1000 via an air circuit 4170 to a patient interface 3000, e.g., see Figs. 1A to 1C.

### 5.3 PATIENT INTERFACE

Fig. 3A shows a non-invasive patient interface 3000 in accordance with one aspect of the present technology comprising the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.
If a patient interface is unable to comfortably deliver a minimum level of positive pressure to the airways, the patient interface may be unsuitable for respiratory pressure therapy.
The patient interface 3000 in accordance with one form of the present technology is constructed and arranged to be able to provide a supply of air at a positive pressure of at least 6 $cmH_2O$ with respect to ambient.
The patient interface 3000 in accordance with one form of the present technology is constructed and arranged to be able to provide a supply of air at a positive pressure of at least 10 $cmH_2O$ with respect to ambient.
The patient interface 3000 in accordance with one form of the present technology is constructed and arranged to be able to provide a supply of air at a positive pressure of at least 20 $cmH_2O$ with respect to ambient.

5.4 RPT DEVICE

An exploded view of an RPT device 4000 in accordance with one aspect of the present technology is shown in Fig. 5A. An RPT device 4000 may comprise mechanical, pneumatic, and/or electrical components and be configured to execute one or more algorithms. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to + 150 L/min while maintaining a positive pressure of at least 6 $cmH_2O$, or at least 10$cmH_2O$, or at least 20 $cmH_2O$.

The RPT device 4000 may include an external housing having one or more panel(s) such as a main panel 4010, a front panel 4012 and a side panel 4014. The RPT device 4000 may also comprise an outlet muffler 4124 as shown in Figs. 5A and 5B. The outlet muffler 4124 may be removable and replaced with a water reservoir 5110 (see Fig. 5C). In such forms, the RPT device 4000 may be considered to include an integrated humidifier 5000. Thus, the RPT device 4000 may be used with or without humidification depending upon whether the water reservoir 5110 or the outlet muffler 4124 respectively is attached. Preferably the RPT device 4000 comprises a chassis 4016 that supports one or more internal components of the RPT device 4000. In one form the RPT device 4000 comprises a pressure generator 4140, which may be housed in a pneumatic block 4020 coupled to the chassis 4016.

Further examples and details of an exemplary RPT device are described in PCT Publication No. WO2015/089582.

The pneumatic path of the RPT device 4000 (e.g. shown in Fig. 5D) may comprise an inlet air filter 4112, an inlet muffler 4122, a pressure generator 4140 capable of supplying air at positive pressure (preferably a blower 4142) and an outlet muffler 4124 (or a water reservoir 5110 if humidification is required). One or more transducers 4270, such as pressure sensors and flow sensors may be included in the pneumatic path. The pneumatic path may also include anti-spill back valve 4160 to prevent water from the humidifier 5000 spilling back to the electrical components of the RPT device 4000.

As shown in Fig. 5E, the RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202 (e.g., see Fig. 5A). In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

#### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

#### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

#### 5.4.1.3 Pressure generator

In one form of the present technology, a pressure generator 4140 for producing a flow, or a supply, of air at positive pressure is a controllable blower 4142. For example the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 $cmH_2O$ to about 20 $cmH_2O$, or in other forms up to about 30 $cmH_2O$. The blower may be as described in any one of the following patents or patent applications**:**
U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

#### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

##### *5.4.1.4.1 Flow rate sensor*

A flow rate sensor 4274 in accordance with the present technology may be based on a differential pressure transducer, for example, an SDP600 Series differential pressure transducer from SENSIRION.

In one form, a signal representing a flow rate from the flow rate sensor 4274 is received by the central controller 4230.

##### *5.4.1.4.2 Pressure sensor*

A pressure sensor 4272 in accordance with the present technology is located in fluid communication with the pneumatic path. An example of a suitable pressure sensor is a transducer from the HONEYWELL ASDX series. An alternative suitable pressure sensor is a transducer from the NPA Series from GENERAL ELECTRIC.

In one form, a signal from the pressure sensor 4272 is received by the central controller 4230.

##### *5.4.1.4.3 Motor speed transducer*

In one form of the present technology a motor speed transducer 4276 is used to determine a rotational velocity of the motor 4144 and/or the blower 4142. A motor speed signal from the motor speed transducer 4276 may be provided to the therapy device controller 4240. The motor speed transducer 4276 may, for example, be a speed sensor, such as a Hall effect sensor.

#### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

#### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

#### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller 4230.

In one form, the input device 4220 may be constructed and arranged to allow a person to select a value and/or a menu option.

#### 5.4.2.3 Central controller

In one form of the present technology, the central controller 4230 is one or a plurality of processors suitable to control an RPT device 4000.

Suitable processors may include an x86 INTEL processor, a processor based on ARM® Cortex®-M processor from ARM Holdings such as an STM32 series microcontroller from ST MICROELECTRONIC. In certain alternative forms of the present technology, a 32-bit RISC CPU, such as an STR9 series microcontroller from ST MICROELECTRONICS or a 16-bit RISC CPU such as a processor from the MSP430 family of microcontrollers, manufactured by TEXAS INSTRUMENTS may also be suitable.

In one form of the present technology, the central controller 4230 is a dedicated electronic circuit.

In one form, the central controller 4230 is an application-specific integrated circuit. In another form, the central controller 4230 comprises discrete electronic components.

The central controller 4230 may be configured to receive input signal(s) from one or more transducers 4270, one or more input devices 4220, and the humidifier 5000.

The central controller 4230 may be configured to provide output signal(s) to one or more of an output device 4290, a therapy device controller 4240, a data communication interface 4280, and the humidifier 5000.

In some forms of the present technology, the central controller 4230 is configured to implement the one or more methodologies described herein, such as the one or more algorithms 4300 expressed as computer programs stored in a non-transitory computer readable storage medium, such as memory 4260. In some forms of the present technology, the central controller 4230 may be integrated with an RPT device 4000. However, in some forms of the present technology, some methodologies may be performed by a remotely located device. For example, the remotely located device may determine control settings for a ventilator or detect respiratory related events by analysis of stored data such as from any of the sensors described herein.

#### 5.4.2.4 Clock

The RPT device 4000 may include a clock 4232 that is connected to the central controller 4230.

#### 5.4.2.5 Therapy device controller

In one form of the present technology, therapy device controller 4240 is a therapy control module 4330 that forms part of the algorithms 4300 executed by the central controller 4230.

In one form of the present technology, therapy device controller 4240 is a dedicated motor control integrated circuit. For example, in one form a MC33035 brushless DC motor controller, manufactured by ONSEMI is used.

#### 5.4.2.6 Protection circuits

The one or more protection circuits 4250 in accordance with the present technology may comprise an electrical protection circuit, a temperature and/or pressure safety circuit.

#### 5.4.2.7 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions expressing the one or more methodologies described herein, such as the one or more algorithms 4300.

#### 5.4.2.8 Data communication systems

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile phone, tablet or remote control.

#### 5.4.2.9 Output devices including optional display, alarms

An output device 4290 in accordance with the present technology may take the form of one or more of a visual, audio and haptic unit. A visual display may be a Liquid Crystal Display (LCD) or Light Emitting Diode (LED) display.

##### *5.4.2.9.1 Display driver*

A display driver 4292 receives as an input the characters, symbols, or images intended for display on the display 4294, and converts them to commands that cause the display 4294 to display those characters, symbols, or images.

##### *5.4.2.9.2 Display*

A display 4294 is configured to visually display characters, symbols, or images in response to commands received from the display driver 4292. For example, the display 4294 may be an eight-segment display, in which case the display driver 4292 converts each character or symbol, such as the figure "0", to eight logical signals indicating whether the eight respective segments are to be activated to display a particular character or symbol.

### 5.4.3 RPT device algorithms

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules, e.g., see Fig. 5F.

#### 5.4.3.1 Pre-processing module

A pre-processing module 4310 in accordance with one form of the present technology receives as an input a signal from a transducer 4270, for example a flow rate sensor 4274 or pressure sensor 4272, and performs one or more process steps to calculate one or more output values that will be used as an input to another module, for example a therapy engine module 4320.

In one form of the present technology, the output values include the interface or mask pressure *Pm,* the respiratory flow rate *Qr,* and the leak flow rate *Ql.*

In various forms of the present technology, the pre-processing module 4310 comprises one or more of the following algorithms: pressure compensation 4312, vent flow rate estimation 4314, leak flow rate estimation 4316, and respiratory flow rate estimation 4318.

#### *5.4.3.1.1 Pressure compensation*

In one form of the present technology, a pressure compensation algorithm 4312 receives as an input a signal indicative of the pressure in the pneumatic path proximal to an outlet of the pneumatic block. The pressure compensation algorithm 4312 estimates the pressure drop through the air circuit 4170 and provides as an output an estimated pressure, *Pm,* in the patient interface 3000.

#### *5.4.3.1.2 Vent flow rate estimation*

In one form of the present technology, a vent flow rate estimation algorithm 4314 receives as an input an estimated pressure, *Pm,* in the patient interface 3000 and estimates a vent flow rate of air, *Qv,* from a vent 3400 in a patient interface 3000.

#### *5.4.3.1.3 Leak flow rate estimation*

In one form of the present technology, a leak flow rate estimation algorithm 4316 receives as an input a total flow rate, *Qt,* and a vent flow rate *Qv,* and provides as an output an estimate of the leak flow rate *Ql.* In one form, the leak flow rate estimation algorithm estimates the leak flow rate *Ql* by calculating an average of the difference between total flow rate *Qt* and vent flow rate *Qv* over a period sufficiently long to include several breathing cycles, e.g. about 10 seconds.

In one form, the leak flow rate estimation algorithm 4316 receives as an input a total flow rate *Qt,* a vent flow rate *Qv,* and an estimated pressure, *Pm,* in the patient interface 3000, and provides as an output a leak flow rate *Ql,* by calculating a leak conductance, and determining a leak flow rate *Ql* to be a function of leak conductance and pressure, *Pm.* Leak conductance is calculated as the quotient of low pass filtered non-vent flow rate equal to the difference between total flow rate *Qt* and vent flow rate *Qv,* and low pass filtered square root of pressure *Pm,* where the low pass filter time constant has a value sufficiently long to include several breathing cycles, e.g. about 10 seconds. The leak flow rate *Ql* may be estimated as the product of leak conductance and a function of pressure, *Pm.*

#### *5.4.3.1.4 Respiratory flow rate estimation*

In one form of the present technology, a respiratory flow rate estimation algorithm 4318 receives as an input a total flow rate, *Qt,* a vent flow rate, *Qv,* and a leak flow rate, *Ql,* and estimates a respiratory flow rate of *air*, *Qr*, to the patient, by subtracting the vent flow rate *Qv* and the leak flow rate *Ql* from the total flow rate *Qt.*

### 5.4.3.2 Therapy Engine Module

In one form of the present technology, a therapy engine module 4320 receives as inputs one or more of a pressure, *Pm,* in a patient interface 3000, and a respiratory flow rate of air to a patient, *Qr,* and provides as an output one or more therapy parameters.

In one form of the present technology, a therapy parameter is a treatment pressure *Pt.*

In one form of the present technology, therapy parameters are one or more of an amplitude of a pressure variation, a base pressure, and a target ventilation.

In various forms, the therapy engine module 4320 comprises one or more of the following algorithms: phase determination 4321, waveform determination 4322, ventilation determination 4323, inspiratory flow limitation determination 4324, apnea / hypopnea determination 4325, snore determination 4326, airway patency determination 4327, target ventilation determination 4328, and therapy parameter determination 4329.

#### *5.4.3.2.1 Phase determination*

In one form of the present technology, the RPT device 4000 does not determine phase.

In one form of the present technology, a phase determination algorithm 4321 receives as an input a signal indicative of respiratory flow rate, *Qr,* and provides as an output a phase $\Phi$ of a current breathing cycle of a patient 1000.

In some forms, known as discrete phase determination, the phase output $\Phi$ is a discrete variable. One implementation of discrete phase determination provides a bi-valued phase output $\Phi$ with values of either inhalation or exhalation, for example represented as values of 0 and 0.5 revolutions respectively, upon detecting the start of spontaneous inhalation and exhalation respectively. RPT devices 4000 that "trigger" and "cycle" effectively perform discrete phase determination, since the trigger and cycle points are the instants at which the phase changes from exhalation to inhalation and from inhalation to exhalation, respectively. In one implementation of bi-valued phase determination, the phase output $\Phi$ is determined to have a discrete value of 0 (thereby "triggering" the RPT device 4000) when the respiratory flow rate *Qr* has a value that exceeds a positive threshold, and a discrete value of 0.5 revolutions (thereby "cycling" the RPT device 4000) when a respiratory flow rate *Qr* has a value that is more negative than a negative threshold. The inhalation time *Ti* and the exhalation time *Te* may be estimated as typical values over many respiratory cycles of the time spent with phase $\Phi$ equal to 0 (indicating inspiration) and 0.5 (indicating expiration) respectively.

Another implementation of discrete phase determination provides a tri-valued phase output $\Phi$ with a value of one of inhalation, mid-inspiratory pause, and exhalation.

In other forms, known as continuous phase determination, the phase output $\Phi$ is a continuous variable, for example varying from 0 to 1 revolutions, or 0 to $2\pi$ radians. RPT devices 4000 that perform continuous phase determination may trigger and cycle when the continuous phase reaches 0 and 0.5 revolutions, respectively. In one implementation of continuous phase determination, a continuous value of phase $\Phi$ is determined using a fuzzy logic analysis of the respiratory flow rate *Qr.* A continuous value of phase determined in this implementation is often referred to as "fuzzy phase". In one implementation of a fuzzy phase determination algorithm 4321, the following rules are applied to the respiratory flow rate *Qr*:

1. If the respiratory flow rate is zero and increasing fast then the phase is 0 revolutions.

2. If the respiratory flow rate is large positive and steady then the phase is 0.25 revolutions.

3. If the respiratory flow rate is zero and falling fast, then the phase is 0.5 revolutions.

4. If the respiratory flow rate is large negative and steady then the phase is 0.75 revolutions.

5. If the respiratory flow rate is zero and steady and the 5-second low-pass filtered absolute value of the respiratory flow rate is large then the phase is 0.9 revolutions.

6. If the respiratory flow rate is positive and the phase is expiratory, then the phase is 0 revolutions.

7. If the respiratory flow rate is negative and the phase is inspiratory, then the phase is 0.5 revolutions.

8. If the 5-second low-pass filtered absolute value of the respiratory flow rate is large, the phase is increasing at a steady rate equal to the patient's breathing rate, low-pass filtered with a time constant of 20 seconds.

The output of each rule may be represented as a vector whose phase is the result of the rule and whose magnitude is the fuzzy extent to which the rule is true. The fuzzy extent to which the respiratory flow rate is "large", "steady", etc. is determined with suitable membership functions. The results of the rules, represented as vectors, are then combined by some function such as taking the centroid. In such a combination, the rules may be equally weighted, or differently weighted.

In another implementation of continuous phase determination, the phase $\Phi$ is first discretely estimated from the respiratory flow rate *Qr* as described above, as are the inhalation time *Ti* and the exhalation time *Te.* The continuous phase $\Phi$ at any instant may be determined as the half the proportion of the inhalation time *Ti* that has elapsed since the previous trigger instant, or 0.5 revolutions plus half the proportion of the exhalation time *Te* that has elapsed since the previous cycle instant (whichever instant was more recent).

#### *5.4.3.2.2 Waveform determination*

In one form of the present technology, the therapy parameter determination algorithm 4329 provides an approximately constant treatment pressure throughout a respiratory cycle of a patient.

In other forms of the present technology, the therapy control module 4330 controls the pressure generator 4140 to provide a treatment pressure *Pt* that varies as a function of phase $\Phi$ of a respiratory cycle of a patient according to a waveform template $\Pi(\Phi)$.

In one form of the present technology, a waveform determination algorithm 4322 provides a waveform template $\Pi(\Phi)$ with values in the range [0, 1] on the domain of phase values $\Phi$ provided by the phase determination algorithm 4321 to be used by the therapy parameter determination algorithm 4329.

In one form, suitable for either discrete or continuously-valued phase, the waveform template $\Pi(\Phi)$ is a square-wave template, having a value of 1 for values of phase up to and including 0.5 revolutions, and a value of 0 for values of phase above 0.5 revolutions. In one form, suitable for continuously-valued phase, the waveform template $\Pi(\Phi)$ comprises two smoothly curved portions, namely a smoothly curved (e.g. raised cosine) rise from 0 to 1 for values of phase up to 0.5 revolutions, and a smoothly curved (e.g. exponential) decay from 1 to 0 for values of phase above 0.5 revolutions. In one form, suitable for continuously-valued phase, the waveform template $\Pi(\Phi)$ is based on a square wave, but with a smooth rise from 0 to 1 for values of phase up to a "rise time" that is less than 0.5 revolutions, and a smooth fall from 1 to 0 for values of phase within a "fall time" after 0.5 revolutions, with a "fall time" that is less than 0.5 revolutions.

In some forms of the present technology, the waveform determination algorithm 4322 selects a waveform template $\Pi(\Phi)$ from a library of waveform templates, dependent on a setting of the RPT device. Each waveform template $\Pi(\Phi)$ in the library may be provided as a lookup table of values $\Pi$ against phase values $\Phi$. In other forms, the waveform determination algorithm 4322 computes a waveform template $\Pi(\Phi)$ "on the fly" using a predetermined functional form, possibly parametrised by one or more parameters (e.g. time constant of an exponentially curved portion). The parameters of the functional form may be predetermined or dependent on a current state of the patient 1000.

In some forms of the present technology, suitable for discrete bi-valued phase of either inhalation ($\Phi$ = 0 revolutions) or exhalation ($\Phi$ = 0.5 revolutions), the waveform determination algorithm 4322 computes a waveform template $\Pi$ "on the fly" as a function of both discrete phase $\Phi$ and time t measured since the most recent trigger instant. In one such form, the waveform determination algorithm 4322 computes the waveform template $\Pi(\Phi, t)$ in two portions (inspiratory and expiratory) as follows:

$$\Pi(\Phi,t)=\begin{cases}\Pi_i(t), & \Phi=0\\ \Pi_e(t-T_i), & \Phi=0.5\end{cases}$$

where $\Pi_i(t)$ and $\Pi_e(t)$ are inspiratory and expiratory portions of the waveform template $\Pi(\Phi, t)$. In one such form, the inspiratory portion $\Pi_i(t)$ of the waveform template is a smooth rise from 0 to 1 parametrised by a rise time, and the expiratory portion $\Pi_e(t)$ of the waveform template is a smooth fall from 1 to 0 parametrised by a fall time.

#### *5.4.3.2.3 Ventilation determination*

In one form of the present technology, a ventilation determination algorithm 4323 receives an input a respiratory flow rate *Qr,* and determines a measure indicative of current patient ventilation, *Vent.*

In some implementations, the ventilation determination algorithm 4323 determines a measure of ventilation *Vent* that is an estimate of actual patient ventilation. One such implementation is to take half the absolute value of respiratory flow rate, *Qr,* optionally filtered by low-pass filter such as a second order Bessel low-pass filter with a corner frequency of 0.11 Hz.

In other implementations, the ventilation determination algorithm 4323 determines a measure of ventilation *Vent* that is broadly proportional to actual patient ventilation. One such implementation estimates peak respiratory flow rate *Qpeak* over the inspiratory portion of the cycle. This and many other procedures involving sampling the respiratory flow rate *Qr* produce measures which are broadly proportional to ventilation, provided the flow rate waveform shape does not vary very much (here, the shape of two breaths is taken to be similar when the flow rate waveforms of the breaths normalised in time and amplitude are similar). Some simple examples include the median positive respiratory flow rate, the median of the absolute value of respiratory flow rate, and the standard deviation of flow rate. Arbitrary linear combinations of arbitrary order statistics of the absolute value of respiratory flow rate using positive coefficients, and even some using both positive and negative coefficients, are approximately proportional to ventilation. Another example is the mean of the respiratory flow rate in the middle K proportion (by time) of the inspiratory portion, where $0 < K < 1$. There is an arbitrarily large number of measures that are exactly proportional to ventilation if the flow rate shape is constant.

#### *5.4.3.2.4 Determination of Inspiratory Flow limitation*

In one form of the present technology, the central controller 4230 executes an inspiratory flow limitation determination algorithm 4324 for the determination of the extent of inspiratory flow limitation.

In one form, the inspiratory flow limitation determination algorithm 4324 receives as an input a respiratory flow rate signal *Qr* and provides as an output a metric of the extent to which the inspiratory portion of the breath exhibits inspiratory flow limitation.

In one form of the present technology, the inspiratory portion of each breath is identified by a zero-crossing detector. A number of evenly spaced points (for example, sixty-five), representing points in time, are interpolated by an interpolator along the inspiratory flow rate-time curve for each breath. The curve described by the points is then scaled by a scalar to have unity length (duration/period) and unity area to remove the effects of changing breathing rate and depth. The scaled breaths are then compared in a comparator with a pre-stored template representing a normal unobstructed breath, similar to the inspiratory portion of the breath shown in Fig. 6A. Breaths deviating by more than a specified threshold (typically 1 scaled unit) at any time during the inspiration from this template, such as those due to coughs, sighs, swallows and hiccups, as determined by a test element, are rejected. For non-rejected data, a moving average of the first such scaled point is calculated by the central controller 4230 for the preceding several inspiratory events. This is repeated over the same inspiratory events for the second such point, and so on. Thus, for example, sixty five scaled data points are generated by the central controller 4230, and represent a moving average of the preceding several inspiratory events, e.g., three events. The moving average of continuously updated values of the (e.g., sixty five) points are hereinafter called the "scaled flow rate ", designated as *Qs(t)*. Alternatively, a single inspiratory event can be utilised rather than a moving average.

From the scaled flow rate, two shape factors relating to the determination of partial obstruction may be calculated.

Shape factor 1 is the ratio of the mean of the middle (e.g. thirty-two) scaled flow rate points to the mean overall (e.g. sixty-five) scaled flow rate points. Where this ratio is in excess of unity, the breath will be taken to be normal. Where the ratio is unity or less, the breath will be taken to be obstructed. A ratio of about 1.17 is taken as a threshold between partially obstructed and unobstructed breathing, and equates to a degree of obstruction that would permit maintenance of adequate oxygenation in a typical patient.

Shape factor 2 is calculated as the RMS deviation from unit scaled flow rate, taken over the middle (e.g. thirty two) points. An RMS deviation of about 0.2 units is taken to be normal. An RMS deviation of zero is taken to be a totally flow-limited breath. The closer the RMS deviation to zero, the breath will be taken to be more flow limited.

Shape factors 1 and 2 may be used as alternatives, or in combination. In other forms of the present technology, the number of sampled points, breaths and middle points may differ from those described above. Furthermore, the threshold values can be other than those described.

### *5.4.3.2.5 Determination of apneas and hypopneas*

In one form of the present technology, the central controller 4230 executes an apnea / hypopnea determination algorithm 4325 for the determination of the presence of apneas and/or hypopneas.

In one form, the apnea / hypopnea determination algorithm 4325 receives as an input a respiratory flow rate signal *Qr* and provides as an output a flag that indicates that an apnea or a hypopnea has been detected.

In one form, an apnea will be said to have been detected when a function of respiratory flow rate *Qr* falls below a flow rate threshold for a predetermined period of time. The function may determine a peak flow rate, a relatively short-term mean flow rate, or a flow rate intermediate of relatively short-term mean and peak flow rate, for example an RMS flow rate. The flow rate threshold may be a relatively long-term measure of flow rate.

In one form, a hypopnea will be said to have been detected when a function of respiratory flow rate *Qr* falls below a second flow rate threshold for a predetermined period of time. The function may determine a peak flow, a relatively short-term mean flow rate, or a flow rate intermediate of relatively short-term mean and peak flow rate, for example an RMS flow rate. The second flow rate threshold may be a relatively long-term measure of flow rate. The second flow rate threshold is greater than the flow rate threshold used to detect apneas.

### *5.4.3.2.6 Determination of snore*

In one form of the present technology, the central controller 4230 executes one or more snore determination algorithms 4326 for the determination of the extent of snore.

In one form, the snore determination algorithm 4326 receives as an input a respiratory flow rate signal *Qr* and provides as an output a metric of the extent to which snoring is present.

The snore determination algorithm 4326 may comprise the step of determining the intensity of the flow rate signal in the range of 30-300 Hz. Further, the snore determination algorithm 4326 may comprise a step of filtering the respiratory flow rate signal *Qr* to reduce background noise, e.g., the sound of airflow in the system from the blower.

### *5.4.3.2.7 Determination of airway patency*

In one form of the present technology, the central controller 4230 executes one or more airway patency determination algorithms 4327 for the determination of the extent of airway patency.

In one form, the airway patency determination algorithm 4327 receives as an input a respiratory flow rate signal *Qr,* and determines the power of the signal in the frequency range of about 0.75 Hz and about 3 Hz. The presence of a peak in this frequency range is taken to indicate an open airway. The absence of a peak is taken to be an indication of a closed airway.

In one form, the frequency range within which the peak is sought is the frequency of a small forced oscillation in the treatment pressure *Pt*. In one implementation, the forced oscillation is of frequency 2 Hz with amplitude about 1 $cmH_2O$.

In one form, airway patency determination algorithm 4327 receives as an input a respiratory flow rate signal *Qr,* and determines the presence or absence of a cardiogenic signal. The absence of a cardiogenic signal is taken to be an indication of a closed airway.

### *5.4.3.2.8 Determination of target ventilation*

In one form of the present technology, the central controller 4230 takes as input the measure of current ventilation, *Vent,* and executes one or more target ventilation determination algorithms 4328 for the determination of a target value *Vtgt* for the measure of ventilation.

In some forms of the present technology, there is no target ventilation determination algorithm 4328, and the target value *Vtgt* is predetermined, for example by hard-coding during configuration of the RPT device 4000 or by manual entry through the input device 4220.

In other forms of the present technology, such as adaptive servo-ventilation (ASV), the target ventilation determination algorithm 4328 computes a target value *Vtgt* from a value *Vtyp* indicative of the typical recent ventilation of the patient.

In some forms of adaptive servo-ventilation, the target ventilation *Vtgt* is computed as a high proportion of, but less than, the typical recent ventilation *Vtyp.* The high proportion in such forms may be in the range (80%, 100%), or (85%, 95%), or (87%, 92%).

In other forms of adaptive servo-ventilation, the target ventilation *Vtgt* is computed as a slightly greater than unity multiple of the typical recent ventilation *Vtyp.*

The typical recent ventilation *Vtyp* is the value around which the distribution of the measure of current ventilation *Vent* over multiple time instants over some predetermined timescale tends to cluster, that is, a measure of the central tendency of the measure of current ventilation over recent history. In one implementation of the target ventilation determination algorithm 4328, the recent history is of the order of several minutes, but in any case should be longer than the timescale of Cheyne-Stokes waxing and waning cycles. The target ventilation determination algorithm 4328 may use any of the variety of well-known measures of central tendency to determine the typical recent ventilation *Vtyp* from the measure of current ventilation, *Vent.* One such measure is the output of a low-pass filter on the measure of current ventilation *Vent,* with time constant equal to one hundred seconds.

### *5.4.3.2.9 Determination of therapy parameters*

In some forms of the present technology, the central controller 4230 executes one or more therapy parameter determination algorithms 4329 for the determination of one or more therapy parameters using the values returned by one or more of the other algorithms in the therapy engine module 4320.

In one form of the present technology, the therapy parameter is an instantaneous treatment pressure *Pt.* In one implementation of this form, the therapy parameter determination algorithm 4329 determines the treatment pressure *Pt* using the equation

$$Pt = A\Pi(\Phi, t) + P_0 \qquad (1)$$

where:

- *A* is the amplitude,
- $\Pi(\Phi, t)$ is the waveform template value (in the range 0 to 1) at the current value $\Phi$ of phase and *t* of time, and
- $P_0$ is a base pressure.

If the waveform determination algorithm 4322 provides the waveform template $\Pi(\Phi, t)$ as a lookup table of values $\Pi$ indexed by phase $\Phi$, the therapy parameter determination algorithm 4329 applies equation (1) by locating the nearest lookup table entry to the current value $\Phi$ of phase returned by the phase determination algorithm 4321, or by interpolation between the two entries straddling the current value $\Phi$ of phase.

The values of the amplitude *A* and the base pressure $P_0$ may be set by the therapy parameter determination algorithm 4329 depending on the chosen respiratory pressure therapy mode in the manner described below.

### 5.4.3.3 Therapy Control module

The therapy control module 4330 in accordance with one aspect of the present technology receives as inputs the therapy parameters from the therapy parameter determination algorithm 4329 of the therapy engine module 4320, and controls the pressure generator 4140 to deliver a flow of air in accordance with the therapy parameters.

In one form of the present technology, the therapy parameter is a treatment pressure *Pt,* and the therapy control module 4330 controls the pressure generator 4140 to deliver a flow of air whose mask pressure *Pm* at the patient interface 3000 is equal to the treatment pressure *Pt.*

#### 5.4.3.4 Detection of fault conditions

In one form of the present technology, the central controller 4230 executes one or more methods 4340 for the detection of fault conditions. The fault conditions detected by the one or more methods 4340 may include at least one of the following:

- Power failure (no power, or insufficient power)
- Transducer fault detection
- Failure to detect the presence of a component
- Operating parameters outside recommended ranges (e.g. pressure, flow rate, temperature, $PaO_2$)
- Failure of a test alarm to generate a detectable alarm signal.

Upon detection of the fault condition, the corresponding algorithm 4340 signals the presence of the fault by one or more of the following:

- Initiation of an audible, visual &/or kinetic (e.g. vibrating) alarm
- Sending a message to an external device
- Logging of the incident

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

#### 5.5.1 Oxygen delivery

In one form of the present technology, supplemental oxygen 4180 is delivered to one or more points in the pneumatic path, such as upstream of the pneumatic block 4020, to the air circuit 4170 and/or to the patient interface 3000.

### 5.6 HUMIDIFICATION WITH HME AND HEATED AIR DELIVERY TUBE

#### 5.6.1 HME overview

Delivery of a flow of air without humidification may cause drying of airways. A heat and moisture exchanger (HME) may be utilized in PAP therapy to partially recover heat and moisture present in exhaled gas from a patient's airways. This heat and moisture can be retained and recycled to the patient in a passive manner as a flow of breathable gas passes through the HME prior to inspiration. Thus, the use of a HME can provide the needed moisture and humidity (generally recognized as >10mg/l) to most patients during PAP therapy to minimize any detrimental effects associated with PAP therapy with non-humidified ambient air.

HMEs are generally made up of foam, paper, or a substance capable of acting as a condensation and absorption surface. The material may carry hygroscopic salts to improve the water-retaining capacity. Suitable salts include calcium chloride.

A problem common with the use of HMEs in PAP therapy relates to condensation in the patient interface in a cold environment. When a patient is sleeping during PAP therapy in a cold room, the room air chills the outside surfaces of the patient interface and the air delivery tube, and the incoming cold air on each patient inhalation chills the inside surfaces of the patient interface. The colder the patient interface, the more of the patient's warm humid breath that will condense on the inside surfaces of the patient interface with each patient exhalation. This effect is exacerbated by adding a HME to the air delivery circuit, as its retention of moisture in the patient interface causes more condensation.

Also, HMEs may include different capacities for moisture retention, from which it is necessary to select the appropriate HME capacity to suit the ambient humidity. For example, one HME model may be configured to suit a moderate environment and another HME model may be configured to suit a dry environment. When the ambient humidity rises, the moisture inhaled through the HME also rises and this can exacerbate condensation in the patient interface.

Thus, there is a need to provide HME use in PAP therapy to achieve desired patient humidification while reducing the effects of a cold environment on the HME system.

5.6.2 HME and Heated Air Delivery Tube

An aspect of the present technology is directed to humidification with an HME and a heated air delivery tube. As described below, the heated air delivery tube warms the incoming air, which warms the inside surfaces of the patient interface to reduce the effect of chilling the inside surfaces of the patient interface on each patient inhalation. Further, when the incoming air to the HME is warmed, the moisture returned from the HME is reduced. As a result, the increased air temperature provided by the heated air delivery tube may be controlled to reduce condensation on the inside surfaces of the patient interface and balance the moisture level inhaled by the patient.

Figs. 6 to 13 show a CPAP system 7000 according to an example of the present technology. As illustrated, the CPAP system 7000 includes an RPT device 4000, a patient interface 3000, an adaptor 6000 including an HME 6500, and a heated air delivery tube 4170. The heated air delivery tube 4170 and the adaptor 6000 cooperate to deliver the flow of air from the RPT device 4000 to the patient interface 3000.

As described above, the RPT device 4000 comprises a pneumatic block 4020 supported within an external housing. The blower 4142 of the pneumatic block 4020 is structured and arranged for producing a flow, or a supply, of air at positive pressure, e.g., in the range of 2-50 $cmH_2O$. In an example, the blower may include a single stage design or a multi-stage design, e.g., two or more stage designs. The blower is operable to draw a supply of air into the external housing, e.g., through one or more intake openings in the external housing, and into an inlet thereof (blower inlet), and provide a pressurized supply of air at an outlet (blower outlet). Examples and details of an exemplary RPT device are described in PCT Publication No. WO 2015/089582. The blower outlet is communicated with the heated air delivery tube 4170.

The air delivery tube 4170 includes a tube portion 4172, an RPT device connector/cuff (outlet connector) 4174 adapted to connect the air delivery tube 4170 to the outlet of the RPT device 4000, and a patient interface connector/cuff (inlet connector) 4176 adapted to connect the air delivery tube 4170 to the inlet of the patient interface 3000, e.g., via the adaptor 6000 as shown in Figs. 6 to 8.

In an example, the RPT device connector 4174 is structured and arranged to form a pneumatic, mechanical and electrical connection with the RPT device 4000. These connections allow pressurized gas to flow from the RPT device 4000 to the patient interface 3000, locate and secure the air delivery tube 4170 to the RPT device 4000, and provide electrical power and signalling to the heating element and transducers of the air delivery tube 4170. These connections may be formed simultaneously or in series, e.g., one of the mechanical, pneumatic or electrical connections may be completed before others.

Examples and details of an exemplary heated air delivery tube are described in PCT Publication No. WO 2015/089582.

In an example, the air delivery tube 4170 may include a plurality of wires helically wound around the axis of the air delivery tube 4170, e.g., configured to heat air in the air delivery tube 4170 and/or transmit signal from one or more transducers (e.g., temperature sensor, flow sensor).

For example, as shown in Fig. 9, a gas temperature sensor, e.g., thermistor 4178, may be provided within the patient interface connector 4176 to measure the temperature of the air flow. The temperature sensed by the thermistor 4178 may be provided as a signal through the wires of the air delivery tube 4170 to a controller.

Examples and details of exemplary sensors and heated air delivery tube are described in US Patent No. 9,903,371.

The patient interface 3000 includes a seal-forming structure 3100 (e.g., nasal pillows), a plenum chamber 3200, and a connection port 3600. The adaptor 6000 including an HME 6500 may be connected to the connection port 3600, e.g., via a short tube assembly and/or an elbow assembly. A vent may be provided to the plenum chamber 3200 or adaptor 6000 for gas washout.

In the illustrated example, as shown in Figs. 6-8 and 10-11, the adaptor 6000 includes multiple components configured and arranged to support HME 6500 and provide a diffuse vent arrangement for gas washout. For example, the adaptor 6000 includes vent and HME assembly 6050, short tube assembly 6010 to provide connection of the vent and HME assembly 6050 with the patient interface 3000, and short tube assembly 6020 to provide connection of the vent and HME assembly 6050 with the heated air delivery tube 4170.

In the illustrated example, the vent and HME assembly 6050 includes connector 6060 to join the vent and HME assembly 6050 with the short tube assembly 6010, housing 6070, core structure 6080 providing orifices for vent flow, and diffuser cover 6090 supporting diffuser 6095 for diffusing vent flow from core structure 6080. The HME 6500 may be retained within the vent and HME assembly 6050, e.g., by housing 6070 and connector 6060.

The short tube assembly 6010 includes tube 6012, connector 6014 to provide connection between the tube 6012 and the connection portion 3600 of the patient interface 3000, and connector 6016 to provide connection between the tube 6012 and the vent and HME assembly 6050, e.g., via the connector 6060.

The short tube assembly 6020 includes tube 6022, connector 6024 to provide connection between the tube 6022 and the vent and HME assembly 6050, e.g., via core structure 6080, and connector 6026 to provide connection between the tube 6022 and the heated air delivery tube 4170, e.g., via patient interface connector 4176.

Examples and details of an exemplary patient interface and adaptor including an HME are described in PCT Publication No. WO 2018/126295.

In an alternative example, as shown in Figs. 14 and 15, the HME 6500 may be positioned within a plenum chamber 3200 of the patient interface 3000. As illustrated, patient interface 3000 includes a seal-forming structure 3100 (e.g., nasal cushion), a plenum chamber 3200, and a connection port 3600. A vent 3400 is provided for gas washout. In this example, HME 6500 is positioned in a flow path of breathable gas within a plenum chamber 3200 of patient interface 3000 and in close proximity to the entrance of the patient's airways.

As shown in Fig. 14, a short tube assembly 6025 may provide connection of the patient interface 3000 with the heated air delivery tube 4170. For example, short tube assembly 6025 may include connector 6027 to provide connection with heated air delivery tube 4170, e.g., via patient interface connector 4176.

Examples and details of an exemplary patient interface including an HME within the plenum chamber are described in PCT Publication No. WO 2015/013761.

It should be appreciated that the patient interface and HME may include other suitable configurations and arrangements. For example, it should be appreciated that aspects of the present technology may be adapted for use with other suitable interface arrangements and types, e.g., full-face/oro-nasal interface, nasal interface, nasal prongs. Also, it should be appreciated that aspects of the present technology may be adapted for use with other suitable HME arrangements and types, e.g., HME positioned upstream of the plenum chamber of the patient interface (e.g., within an elbow assembly, short tube assembly, etc.), HME positioned directly within the plenum chamber of the patient interface.

The RPT device 4000, the heated air delivery tube 4170, and/or the patient interface 3000 may each comprise one or more transducers (sensors), e.g., temperature sensor, humidity sensor, flow sensor, breath rate sensor. The sensors may produce one or more output signals which may be communicated to a controller to control the level of condensation within the patient interface and the temperature and moisture of air inhaled by the patient. For example, the controller may receive as inputs measures of temperature and humidity in the HME system. The controller may be configured to execute or implement algorithms and/or deliver one or more output signals, e.g., control the temperature of the heated air delivery tube.

In an example, as shown in Figs. 12 and 13, the pneumatic block 4020 of the RPT device 4000 may include a pressure sensor 4090 and a flow sensor 4095. As shown in Fig. 12, the pressure sensor 4090 may be communicated with a pressure port 4092 adjacent the outlet 4093 of the pneumatic block 4020, e.g., measures static pressure perpendicular to the air flow direction. As shown in Fig. 13, the flow sensor 4095 may be communicated with flow sensor ports 4096, 4097, e.g., measures the drop in pressure between first and second chambers in the pneumatic block 4020. Further examples and details of sensors are described in PCT Publication No. WO 2015/089582. The RPT device may also include a temperature sensor and/or a humidity sensor. The heated air delivery tube 4170 may include a temperature sensor, e.g., thermistor 4178 provided within the patient interface connector 4176 of the heated air delivery tube 4170 as described above. In alternative example, a temperature sensor and/or a humidity sensor may be provided to a downstream side of the HME 6500, i.e., the side of the HME 6500 closer to the patient. The controller may be configured to control power to the heated air delivery tube 4170 based on feedback from one or more of these sensors, e.g., ambient temperature, ambient humidity, patient settings, and/or tube temperature, for example, to regulate condensation and temperature/moisture of inhaled air. It should be appreciated that one or more alternative sensors may be provided to the RPT device 4000, the heated air delivery tube 4170, and/or the patient interface 3000 to facilitate control.

In environments in which the temperature in the patient interface 3000 (e.g., temperature measured on the patient side of the HME) falls below the Dew Point, condensation will form in the patient interface 3000 (e.g., on interior surfaces of the plenum chamber 3200 and/or along interior surfaces of the elbow/short tube connecting the HME to the plenum chamber 3200 (e.g., along short tube assembly 6010)). According to an aspect of the present technology, condensation is reduced or completely eliminated by warming the incoming air with the heated air delivery tube 4170. That is, the warm incoming air will warm the interior surfaces of the patient interface 3000 so the temperature of such interior surfaces will stay above the Dew Point.

Fig. 17 is a chart showing an example of HME temperature vs. Dew Point for heated tube (e.g., heated tube at "30°C") and non-heated tube (e.g., heated tube at "off") arrangements when tested in an ambient temperature below 10°C. As illustrated, the temperature in the patient interface (e.g., HME temperature measured on the patient side of the HME) is kept above or close to the Dew Point when HME is utilized with the heated air delivery tube. When a heated air delivery tube is not used with the HME, the temperature in the patient interface falls well below the Dew Point, and hence condensation will occur in the patient interface.

Also, when the incoming air to the HME is warmed, the moisture returned from the HME is reduced, e.g., moisture absorption by HME material dependent on temperature. Therefore, the moisture returned to the air by the HME can be reduced by raising the air temperature in the heated air delivery tubing 4170, i.e., controller can compensate for a rising ambient humidity by raising the air temperature in the heated air delivery tubing 4170 and thereby reduce the HME moisture return and balance the moisture level inhaled by the patient. That is, the output of the HME may be controlled by the temperature of the incoming air.

One or more aspects of such arrangement may improve patient comfort (e.g., warmer air, moist air, and/or less condensation) and/or may allow HME humidification to be more suitable for use in cold ambient temperature. Also, one or more aspects of such arrangement may allow one variant of HME to be suitable for a wide range of ambient conditions, e.g., high capacity HME model, thereby avoiding need to select from more than one variant, reducing inventory, and/or improving patient comfort across the wider range.

It should be appreciated that the level of condensation within the patient interface and the temperature and moisture of air inhaled by the patient may be dependent on patient preference, e.g., at least some level of condensation may be preferable by the patient. That is, some patients may be discomforted by a damp feeling where other patients may tolerate quite a wet nose. Also, the internal geometry of the patient interface and the patient's sleeping position may affect where droplets from condensation may run to and collect, whether droplets spit from the vent, or whether droplets trickle back into the patient's nose and/or mouth, i.e., some droplets from condensation may not be disruptive. An aspect of the present technology is to allow the level of condensation and the temperature and moisture of air inhaled by the patient to be controlled based on patient feedback, i.e., control at least partially based on patient input or setting provided by the patient.

Figs. 18 to 33 are flowcharts showing control algorithms according to alternative examples of the present technology.

**In** Fig. 18, the patient sets a preferred or target gas temperature (e.g., in degrees), and feedback control is provided by the controller to adjust power provided to the heated air delivery tube 4170 as required to correct any differences between the preferred gas temperature setting and the gas temperature measured by the gas temperature sensor 4178 provided to the heated air delivery tube 4170. For example, feedback control may increase power to the heated air delivery tube 4170 to raise the gas temperature when the gas temperature measured by the gas temperature sensor 4178 is lower than the preferred gas temperature setting.

**In** Fig. 19, the target gas temperature is determined with reference to ambient humidity. For example, ambient humidity is measured by an ambient humidity sensor (e.g., provided to the RPT device), and the target gas temperature is set based on the measured ambient humidity, e.g., a higher gas temperature is set in higher humidity. The relationship between the measured ambient humidity and set target gas temperature may be pre-determined, e.g., via a lookup table. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 20, a preferred gas temperature setting from the patient is used along with measured ambient humidity as a control input. For example, the target gas temperature is set based on the preferred gas temperature setting from the patient and the measured ambient humidity. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 21, the target gas temperature is determined with reference to ambient temperature. For example, ambient temperature is measured by an ambient temperature sensor (e.g., provided to the RPT device), and the target gas temperature is set based on the measured ambient temperature, e.g., a higher gas temperature is set in colder ambient temperature. The relationship between the measured ambient temperature and set target gas temperature may be pre-determined, e.g., via a lookup table. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 22, a preferred gas temperature setting from the patient is used along with measured ambient temperature as a control input. For example, the target gas temperature is set based on the preferred gas temperature setting from the patient and the measured ambient temperature. **In** an example, the minimum gas temperature set by the patient may be raised in a very cold ambient based on the ambient temperature sensor. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 23, ambient humidity is used along with a preferred gas temperature setting from the patient and measured ambient temperature as a control input. For example, the target gas temperature is set based on the preferred gas temperature setting from the patient, the measured ambient temperature, and the measured ambient humidity. **In** an example, the gas temperature set by the patient may be adjusted based on the ambient temperature and/or the ambient humidity. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 24, the target gas temperature is determined with reference to gas flow. For example, an average gas flow rate is measured by a flow rate sensor (e.g., flow sensor 4095 provided to the **RPT** device), and the target gas temperature is set based on the measured gas flow rate. The relationship between the measured gas flow rate and set target gas temperature may be pre-determined, e.g., via a lookup table. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 25, the target gas temperature is determined with reference to breath rate and/or depth. For example, breath rate and/or depth is measured by a sensor, and the target gas temperature is set based on the measured breath rate and/or depth. The relationship between the measured breath rate and/or depth and set target gas temperature may be pre-determined, e.g., via a lookup table. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 26, a preferred gas temperature setting from the patient, ambient temperature, gas flow rate, breath rate and/or volume, and ambient humidity is used as a control input for the target gas temperature. For example, the target gas temperature is set based on the preferred gas temperature setting from the patient, the measured ambient temperature, the measured gas flow rate, the measured breath rate and/or volume, and the measured ambient humidity. **In** an example, the gas temperature set by the patient may be adjusted based on the ambient temperature, the gas flow rate, the breath rate and/or volume and/or the ambient humidity. Feedback control is provided by the controller to adjust power provided to the heated air delivery tube 4170 as required to correct any differences between the target gas temperature setting and the gas temperature measured by the gas temperature sensor 4178 provided to the heated air delivery tube 4170.

**In** Fig. 27, a target power to the heated air delivery tube 4170 is used as a control input. **In** this example, the heated air delivery tube 4170 is provided without a temperature sensor, and therefore no feedback control of the heated air delivery tube 4170 is provided. The patient sets a preferred warmth level (e.g., low warmth, medium warmth, high warmth) and the target power is set based on the warmth setting. The relationship between the warmth setting and the set target power may be pre-determined, e.g., via a lookup table.

**In** Fig. 28, the target power to the heated air delivery tube 4170 is determined with reference to ambient temperature. For example, ambient temperature is measured by an ambient temperature sensor (e.g., provided to the **RPT** device), and the target power is set based on the measured ambient temperature, e.g., higher power is set in colder ambient temperature. The relationship between the measured ambient temperature and set target power may be pre-determined, e.g., via a lookup table.

**In** Fig. 29, a preferred warmth setting and ambient temperature is used as a control input. For example, the target power is set based on the preferred warmth setting and the measured ambient temperature. The power associated with the set warmth setting may be updated if not sufficient in view of the measured ambient temperature, e.g., power adjusted higher for colder ambient temperature.

**In** Fig. 30, ambient humidity is used as a control input. For example, ambient humidity is measured by an ambient humidity sensor (e.g., provided to the **RPT** device), and the target power is set based on the measured ambient humidity, e.g., higher power is set in higher ambient humidity. The relationship between the measured ambient humidity and set target power may be pre-determined, e.g., via a lookup table.

**In** Fig. 31, gas flow is used as a control input. For example, an average gas flow rate is measured by a flow rate sensor (e.g., flow sensor 4095 provided to the **RPT** device), and the target power is set based on the measured gas flow rate. The relationship between the measured gas flow rate and set target power may be pre-determined, e.g., via a lookup table.

**In** Fig. 32, breath rate and/or depth is used as a control input. For example, breath rate and/or depth is measured by a sensor, and the target power is set based on the measured breath rate and/or depth. The relationship between the measured breath rate and/or depth and set target power may be pre-determined, e.g., via a lookup table.

In Fig. 33, a preferred warmth setting, ambient temperature, gas flow, breath rate and/or depth, and ambient humidity is used as a control input. For example, the target power is set based on the preferred warmth setting, the measured ambient temperature, the measured gas flow rate, the measured breath rate and/or depth, and the measured ambient humidity. The power associated with the set warmth setting may be updated if not sufficient in view of the measured ambient temperature, the measured gas flow rate, the measured breath rate and/or depth, and/or the measured ambient humidity e.g., power adjusted higher for colder ambient temperature and higher ambient humidity.

In an alternative example, fixed power may be provided to the heated air delivery tube 4170. While such arrangement would not require temperature sensing, such arrangement provides poorer adjustment, e.g., for patient comfort.

In an alternative example, the HME may be heated in alternative manners than via a heated air delivery tube. For example, a heater may be provided to the patient interface to heat the patient interface and the HME, e.g., heater along the plenum chamber of the patient interface. According to the invention, a heater is provided to the HME to directly heat the HME. One or more of the control algorithms described above for controlling power to a heated air delivery tube may be also be applicable for controlling power to a heater provided to the HME. In another example, a high thermal capacity material may be provided in the HME to increase passive heat storage.

In an alternative example, as shown in Fig. 16, the HME may be used in conjunction with a heated passover humidifier. As illustrated, the CPAP system in Fig. 16 includes an RPT device 4000 with integrated humidifier including water reservoir 5110, a patient interface 3000, an adaptor 6000 including an HME 6500, and a heated air delivery tube 4170. The humidifier raises the ambient temperature and humidity and the HME further captures moisture from patient. The humidifier output could be updated When the HME is detected.

## 5.7 HUMIDIFIER

### 5.7.1 Humidifier overview

In one form of the present technology there is provided a humidifier 5000 (e.g. as shown in Fig. 5C) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet to receive a flow of air, and a humidifier outlet to deliver a humidified flow of air. The humidifier 5000 may further comprise a humidifier base, which may be adapted to receive the humidifier reservoir 5110 and comprise a heating element.

### 5.7.2 Humidifier components

#### 5.7.2.1 Water reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

#### 5.7.2.2 Conductive portion

According to one arrangement, the reservoir 5110 comprises a conductive portion configured to allow efficient transfer of heat from the heating element to the volume of liquid in the reservoir 5110. In one form, the conductive portion may be arranged as a plate, although other shapes may also be suitable. All or a part of the conductive portion may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

#### 5.7.2.3 Humidifier reservoir dock

In one form, the humidifier 5000 may comprise a humidifier reservoir dock configured to receive the humidifier reservoir 5110. In some arrangements, the humidifier reservoir dock may comprise a locking feature such as a locking lever configured to retain the reservoir 5110 in the humidifier reservoir dock.

#### 5.7.2.4 Water level indicator

The humidifier reservoir 5110 may comprise a water level indicator. In some forms, the water level indicator may provide one or more indications to a user such as the patient 1000 or a care giver regarding a quantity of the volume of water in the humidifier reservoir 5110. The one or more indications provided by the water level indicator may include an indication of a maximum, predetermined volume of water, any portions thereof, such as 25%, 50% or 75% or volumes such as 200 ml, 300 ml or 400ml.

#### 5.7.2.5 Humidifier transducer(s)

The humidifier 5000 may comprise one or more humidifier transducers (sensors) 5210 instead of, or in addition to, transducers 4270 described above. Humidifier transducers 5210 may include one or more of an air pressure sensor 5212, an air flow rate transducer 5214, a temperature sensor 5216, or a humidity sensor 5218 as shown in Fig. 5G. A humidifier transducer 5210 may produce one or more output signals which may be communicated to a controller such as the central controller 4230 and/or the humidifier controller 5250. In some forms, a humidifier transducer may be located externally to the humidifier 5000 (such as in the air circuit 4170) while communicating the output signal to the controller.

##### *5.7.2.5.1 Pressure transducer*

One or more pressure transducers 5212 may be provided to the humidifier 5000 in addition to, or instead of, a pressure sensor 4272 provided in the RPT device 4000.

##### *5.7.2.5.2 Flow rate transducer*

One or more flow rate transducers 5214 may be provided to the humidifier 5000 in addition to, or instead of, a flow rate sensor 4274 provided in the RPT device 4000.

##### *5.7.2.5.3 Temperature transducer*

The humidifier 5000 may comprise one or more temperature transducers 5216. The one or more temperature transducers 5216 may be configured to measure one or more temperatures such as of the heating element 5240 and/or of the flow of air downstream of the humidifier outlet. In some forms, the humidifier 5000 may further comprise a temperature sensor 5216 to detect the temperature of the ambient air.

##### *5.7.2.5.4 Humidity transducer*

In one form, the humidifier 5000 may comprise one or more humidity sensors 5218 to detect a humidity of a gas, such as the ambient air. The humidity sensor 5218 may be placed towards the humidifier outlet in some forms to measure a humidity of the gas delivered from the humidifier 5000. The humidity sensor may be an absolute humidity sensor or a relative humidity sensor.

#### 5.7.2.6 Heating element

A heating element 5240 may be provided to the humidifier 5000 in some cases to provide a heat input to one or more of the volume of water in the humidifier reservoir 5110 and/or to the flow of air. The heating element 5240 may comprise a heat generating component such as an electrically resistive heating track. One suitable example of a heating element 5240 is a layered heating element such as one described in the PCT Patent Application Publication No. WO 2012/17072.

In some forms, the heating element 5240 may be provided in the humidifier base where heat may be provided to the humidifier reservoir 5110 primarily by conduction.

#### 5.7.2.7 Humidifier controller

According to one arrangement of the present technology, a humidifier 5000 may comprise a humidifier controller 5250 as shown in Fig. 5G. In one form, the humidifier controller 5250 may be a part of the central controller 4230. In another form, the humidifier controller 5250 may be a separate controller, which may be in communication with the central controller 4230.

In one form, the humidifier controller 5250 may receive as inputs measures of properties (such as temperature, humidity, pressure and/or flow rate), for example of the flow of air, the water in the reservoir 5110 and/or the humidifier 5000. The humidifier controller 5250 may also be configured to execute or implement humidifier algorithms and/or deliver one or more output signals.

As shown in Fig. 5G, the humidifier controller 5250 may comprise one or more controllers, such as a central humidifier controller 5251, a heated air circuit controller 5254 configured to control the temperature of a heated air circuit 4171 and/or a heating element controller 5252 configured to control the temperature of a heating element 5240.

## 5.8 BREATHING WAVEFORMS

Fig. 4 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

## 5.9 GLOSSARY

For the purposes of the present technology disclosure, in certain forms of the present technology, one or more of the following definitions may apply. In other forms of the present technology, alternative definitions may apply.

### 5.9.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Total flow rate, *Qt,* is the flow rate of air leaving the RPT device. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Humidifier:* The word humidifier will be taken to mean a humidifying apparatus constructed and arranged, or configured with a physical structure to be capable of providing a therapeutically beneficial amount of water ($H_2O$) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including $cmH_2O$, $g\text{-}f/cm^2$ and hectopascal. 1 $cmH_2O$ is equal to 1 $g\text{-}f/cm^2$ and is approximately 0.98 hectopascal. In this specification, unless otherwise stated, pressure is given in units of $cmH_2O$.

The pressure in the patient interface is given the symbol *Pm,* while the treatment pressure, which represents a target value to be achieved by the mask pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

#### 5.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

#### 5.9.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness:* The ability of a material *per se* to resist deformation (e.g. described by a Young's Modulus, or an indentation *hardness* scale measured on a standardised sample size).

- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions.

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 $cmH_2O$ pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.9.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Trot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:

*(i) Flattened:* Having a rise followed by a relatively flat portion, followed by a fall.

(ii) *M-shaped:* Having two local peaks, one at the leading edge, and one at the trailing edge, and a relatively flat portion between the two peaks.

(iii) *Chair-shaped:* Having a single local peak, the peak being at the leading edge, followed by a relatively flat portion.

(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:

(i) a 30% reduction in patient breathing for at least 10 seconds plus an associated 4% desaturation; or

(ii) a reduction in patient breathing (but less than 50%) for at least 10 seconds, with an associated desaturation of at least 3% or an arousal.

*Hyperpnea:* An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume Ve.

*(inhalation) Time (Ti):* The duration of the inspiratory portion of the respiratory flow rate waveform.
*(exhalation) Time (Te):* The duration of the expiratory portion of the respiratory flow rate waveform.
*(total) Time (Ttot):* The total duration between the start of one inspiratory portion of a respiratory flow rate waveform and the start of the following inspiratory portion of the respiratory flow rate waveform.
*Typical recent ventilation:* The value of ventilation around which recent values of ventilation *Vent* over some predetermined timescale tend to cluster, that is, a measure of the central tendency of the recent values of ventilation.
*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).
*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.9.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.
*Backup rate:* A parameter of a ventilator that establishes the minimum breathing rate (typically in number of breaths per minute) that the ventilator will deliver to the patient, if not triggered by spontaneous respiratory effort.
*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.
*Expiratory positive airway pressure (EPAP):* a base pressure, to which a pressure varying within the breath is added to produce the desired mask pressure which the ventilator will attempt to achieve at a given time.
*End expiratory pressure (EEP):* Desired mask pressure which the ventilator will attempt to achieve at the end of the expiratory portion of the breath. If the pressure waveform template $\Pi(\Phi)$ is zero-valued at the end of expiration, i.e. $\Pi(\Phi) = 0$ when $\Phi = 1$, the EEP is equal to the EPAP.
*Inspiratory positive airway pressure (IPAP):* Maximum desired mask pressure which the ventilator will attempt to achieve during the inspiratory portion of the breath.
*Pressure support:* A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.
*Servo-ventilator:* A ventilator that measures patient ventilation, has a target ventilation, and which adjusts the level of pressure support to bring the patient ventilation towards the target ventilation.
*Spontaneous/Timed (S/T):* A mode of a ventilator or other device that attempts to detect the initiation of a breath of a spontaneously breathing patient. If however, the device is unable to detect a breath within a predetermined period of time, the device will automatically initiate delivery of the breath.
*Swing:* Equivalent term to pressure support.
*Triggered:* When a ventilator delivers a breath of *air* to a spontaneously breathing patient, it is said to be triggered to do so at the initiation of the respiratory portion of the breathing cycle by the patient's efforts.

#### 5.9.3.1 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.
*Larynx:* The larynx, or voice box houses the vocal folds and connects the inferior part of the pharynx (hypopharynx) with the trachea.
*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.
*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.9.4 Patient interface

*Anti-asphyxia valve (AAV):* The component or sub-assembly of a mask system that, by opening to atmosphere in a failsafe manner, reduces the risk of excessive $CO_2$ rebreathing by a patient.
*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.
*Frame:* Frame will be taken to mean a mask structure that bears the load of tension between two or more points of connection with a headgear. A mask frame may be a non-airtight load bearing structure in the mask. However, some forms of mask frame may also be air-tight.
*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.
*Membrane:* Membrane will be taken to mean a typically thin element that has, preferably, substantially no resistance to bending, but has resistance to being stretched.
*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.
*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*
*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.
*Stiffener:* A stiffener will be taken to mean a structural component designed to increase the bending resistance of another component in at least one direction.
*Strut:* A strut will be taken to be a structural component designed to increase the compression resistance of another component in at least one direction.
*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.
*Tie* (noun): A structure designed to resist tension.
*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.9.5 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.
To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.9.5.1 Curvature in one dimension

The curvature of a plane curve at *p* may be described as having a sign (e.g. positive, negative) and a magnitude (e.g. 1/radius of a circle that just touches the curve at*p*).

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve *at p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.9.5.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, "path" will be taken to mean a path in the mathematical - topological sense, e.g. a continuous space curve from *f*(0) to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from f(0) to *f*(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

#### 5.9.5.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 30).

*Osculating plane:* The plane containing the unit tangent vector and the unit principal normal vector. See Figures 30 and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 30), a space curve turning towards the direction of the left-hand binormal may be considered as having a left-hand positive torsion (e.g. a left-hand helix). Hence left-hand positive is equivalent to right-hand negative. See Fig. 3T.

#### 5.9.5.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.10 OTHER REMARKS

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

The invention is as defined in the appended claims.

The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

5.11 REFERENCE SIGNS LIST

| Feature Item | Number |
|---|---|
| patient | 1000 |
| bed partner | 1100 |
| patient interface | 3000 |
| seal-forming structure | 3100 |
| plenum chamber | 3200 |
| positioning and stabilising structure | 3300 |
| vent | 3400 |
| connection port | 3600 |
| forehead support | 3700 |
| RPT device | 4000 |
| main panel | 4010 |
| front panel | 4012 |
| side panel | 4014 |
| chassis | 4016 |

(continued)

| Feature Item | Number |
|---|---|
| pneumatic block | 4020 |
| pressure sensor | 4090 |
| pressure port | 4092 |
| outlet | 4093 |
| flow sensor | 4095 |
| flow sensor port | 4096 |
| flow sensor port | 4097 |
| air filter | 4110 |
| inlet air filter | 4112 |
| outlet air filter | 4114 |
| muffler | 4120 |
| mufflers | 4120 |
| inlet muffler | 4122 |
| outlet muffler | 4124 |
| pressure generator | 4140 |
| blower | 4142 |
| motor | 4144 |
| anti - spill back valve | 4160 |
| air delivery tube | 4170 |
| air circuit | 4171 |
| tube portion | 4172 |
| RPT device connector | 4174 |
| patient interface connector | 4176 |
| gas temperature sensor | 4178 |
| supplemental oxygen | 4180 |
| electrical components | 4200 |
| PCBA | 4202 |
| power supply | 4210 |
| input device | 4220 |
| central controller | 4230 |
| clock | 4232 |
| therapy device controller | 4240 |
| protection circuit | 4250 |
| memory | 4260 |
| transducer | 4270 |
| pressure sensor | 4272 |
| flow rate sensor | 4274 |
| motor speed transducer | 4276 |
| data communication interface | 4280 |

(continued)

| Feature Item | Number |
|---|---|
| remote external communication network | 4282 |
| local external communication network | 4284 |
| remote external device | 4286 |
| local external device | 4288 |
| output device | 4290 |
| display driver | 4292 |
| display | 4294 |
| algorithms | 4300 |
| pre - processing module | 4310 |
| pressure compensation algorithm | 4312 |
| vent flow rate estimation algorithm | 4314 |
| leak flow rate estimation algorithm | 4316 |
| respiratory flow rate estimation algorithm | 4318 |
| therapy engine module | 4320 |
| phase determination algorithm | 4321 |
| waveform determination algorithm | 4322 |
| ventilation determination algorithm | 4323 |
| inspiratory flow limitation determination algorithm | 4324 |
| apnea / hypopnea determination algorithm | 4325 |
| snore determination algorithm | 4326 |
| airway patency determination algorithm | 4327 |
| target ventilation determination algorithm | 4328 |
| therapy parameter determination algorithm | 4329 |
| therapy control module | 4330 |
| methods | 4340 |
| humidifier | 5000 |
| water reservoir | 5110 |
| humidifier transducer | 5210 |
| pressure transducer | 5212 |
| flow rate transducer | 5214 |
| temperature transducer | 5216 |
| humidity sensor | 5218 |
| heating element | 5240 |
| humidifier controller | 5250 |
| central humidifier controller | 5251 |
| heating element controller | 5252 |
| air circuit controller | 5254 |
| adaptor | 6000 |
| short tube assembly | 6010 |

(continued)

| Feature Item | Number |
|---|---|
| tube | 6012 |
| connector | 6014 |
| connector | 6016 |
| short tube assembly | 6020 |
| tube | 6022 |
| connector | 6024 |
| short tube assembly | 6025 |
| connector | 6026 |
| connector | 6027 |
| vent and HME assembly | 6050 |
| connector | 6060 |
| housing | 6070 |
| core structure | 6080 |
| diffuser cover | 6090 |
| diffuser | 6095 |
| HME | 6500 |
| CPAP system | 7000 |

## Claims

**1.** A CPAP system for providing air at positive pressure for respiratory therapy to a patient, the CPAP system comprising:

an RPT device (4000) configured to supply a flow of air at a therapeutic pressure:
a patient interface (3000) forming a plenum chamber (3200) pressurizable to the therapeutic pressure, the patient interface including a seal-forming structure constructed and arranged to form a seal with a region of a patient's face surrounding an entrance to a patient's airways;
an air delivery tube (4170) configured to pass the flow of air at the therapeutic pressure from the RPT device to the patient interface;
a heat and moisture exchanger (HME) (6500) constructed and arranged to retain moisture from a flow of expiratory air from the patient, wherein retained moisture from the HME is returned to the flow of air for humidification; and being **characterized in that** it further comprises:
a controllable heater provided to the HME and configured and arranged to directly heat the HME; and
a controller configured to adjust heating of the controllable heater to adjust the moisture returned from the HME to the flow of air for humidification.

**2.** The CPAP system according to claim 1, wherein the HME is provided within the plenum chamber of the patient interface.

**3.** The CPAP system according to claim 1, wherein the HME is provided upstream of the plenum chamber of the patient interface.

**4.** The CPAP system according to claim 3, wherein the HME is provided to an adaptor arranged between the air delivery tube and the patient interface.

**5.** The CPAP system according to any one of claims 1 to 4, wherein the seal-forming structure of the patient interface comprises nasal prongs or a nasal cushion.

6. The CPAP system according to any one of claims 1 to 5, wherein the RPT device, the patient interface and/or the air delivery tube comprises one or more sensors, each of the sensors configured to produce one or more output signals communicated to the controller, and wherein adjustment of the controllable heater is at least partially based on said one or more output signals from said one or more sensors.

7. The CPAP system according to claim 6, wherein the air delivery tube comprises a gas temperature sensor.

8. The CPAP system according to any one of claims 6 to 7, wherein the RPT device comprises a pressure sensor, a flow sensor, a humidity sensor, and/or a temperature sensor.

9. The CPAP system according to any one of claims 6 to 8, wherein the RPT device comprises a patient input, and adjustment of the controllable heater is at least partially based on said patient input.

10. The CPAP system according to claim 9, wherein the patient input comprises a preferred gas temperature setting.

11. The CPAP system according to any one of claims 1 to 10, wherein the controllable heater is configured and arranged to heat the patient interface, and wherein the controller is configured to adjust heating of the controllable heater to adjust condensation in the patient interface.

12. The CPAP system according to any one of claims 1 to 11, further comprising a heated passover humidifier provided to the RPT device.

**Patentansprüche**

1. CPAP-System zum Bereitstellen von Luft unter Überdruck zur Atemtherapie eines Patienten, wobei das CPAP-System aufweist:

   eine RPT-Vorrichtung (4000), die zum Zuführen eines Luftstroms mit einem therapeutischen Druck konfiguriert ist;
   eine Patientenschnittstelle (3000), die eine Luftkammer (3200) bildet, die mit dem therapeutischen Druck beaufschlagbar ist, wobei die Patientenschnittstelle eine dichtungsbildende Struktur aufweist, die zum Bilden einer Dichtung mit einem Bereich des Gesichts eines Patienten aufgebaut und angeordnet ist, die einen Eingang zu den Atemwegen eines Patienten umgibt;
   einen Luftzuführschlauch (4170), der zum Leiten des unter dem therapeutischen Druck stehenden Luftstroms von der RPT-Vorrichtung zur Patientenschnittstelle konfiguriert ist;
   einen Wärme- und Feuchtigkeitstauscher (HME) (6500), der zum Zurückhalten von Feuchtigkeit aus einem Strom von Ausatemluft des Patienten aufgebaut und angeordnet ist, wobei zurückgehaltene Feuchtigkeit aus dem HME zur Befeuchtung in den Luftstrom zurückgeführt wird; und das **dadurch gekennzeichnet ist, dass** es ferner aufweist:

      ein steuerbares Heizgerät, das am HME vorgesehen und zum direkten Beheizen des HME aufgebaut und angeordnet ist; und
      eine Steuerung, die zum Einstellen des Heizens des steuerbaren Heizgeräts konfiguriert ist, um die Feuchtigkeit einzustellen, die vom HME zur Befeuchtung an den Luftstrom zurückgeführt wird.

2. CPAP-System nach Anspruch 1, wobei der HME in der Luftkammer der Patientenschnittstelle vorgesehen ist.

3. CPAP-System nach Anspruch 1, wobei der HME stromaufwärts der Luftkammer der Patientenschnittstelle vorgesehen ist.

4. CPAP-System nach Anspruch 3, wobei der HME an einem Adapter vorgesehen ist, der zwischen dem Luftzuführschlauch und der Patientenschnittstelle angeordnet ist.

5. CPAP-System nach einem der Ansprüche 1 bis 4, wobei die dichtungsbildende Struktur der Patientenschnittstelle Nasenprongs oder ein Nasenpolster aufweist.

6. CPAP-System nach einem der Ansprüche 1 bis 5, wobei die RPT-Vorrichtung, die Patientenschnittstelle und/oder der Luftzuführschlauch einen oder mehrere Sensoren aufweist, wobei jeder der Sensoren zum Erzeugen eines oder mehrerer Ausgangssignale konfiguriert ist, die an die Steuerung übermittelt werden, und wobei die Einstellung des steuerbaren Heizgeräts mindestens teilweise auf dem einen oder den mehreren Ausgangssignalen von dem einen oder den mehreren Sensoren basiert.

**7.** CPAP-System nach Anspruch 6, wobei der Luftzuführschlauch einen Gastemperatursensor aufweist.

**8.** CPAP-System nach einem der Ansprüche 6 bis 7, wobei die RPT-Vorrichtung einen Drucksensor, einen Durchflusssensor, einen Feuchtigkeitssensor und/oder einen Temperatursensor aufweist.

**9.** CPAP-System nach einem der Ansprüche 6 bis 8, wobei die RPT-Vorrichtung eine Patienteneingabe aufweist und die Einstellung des steuerbaren Heizgeräts mindestens teilweise auf der Patienteneingabe basiert.

**10.** CPAP-System nach Anspruch 9, wobei die Patienteneingabe eine bevorzugte Gastemperatureinstellung aufweist.

**11.** CPAP-System nach einem der Ansprüche 1 bis 10, wobei das steuerbare Heizgerät zum Beheizen der Patientenschnittstelle konfiguriert und angeordnet ist, und wobei die Steuerung zum Einstellen des Heizens des steuerbaren Heizgeräts konfiguriert ist, um die Kondensation in der Patientenschnittstelle einzustellen.

**12.** CPAP-System nach einem der Ansprüche 1 bis 11, ferner aufweisend einen beheizten Durchgangsbefeuchter, der an der RPT-Vorrichtung vorgesehen ist.

**Revendications**

**1.** Système CPAP pour fournir de l’air à pression positive pour la thérapie respiratoire d’un patient, le système CPAP comprenant:

un dispositif RPT (4000) configuré pour fournir un flux d’air à une pression thérapeutique;
une interface patient (3000) formant une chambre de répartition (3200) pouvant être mise sous pression à la pression thérapeutique, l’interface patient incluant une structure formant joint conçue et agencée pour former un joint avec une région du visage d’un patient entourant une entrée des voies respiratoires du patient;
un tube d’alimentation en air (4170) configuré pour faire passer le flux d’air à la pression thérapeutique du dispositif RPT à l’interface patient;
un échangeur de chaleur et d’humidité (ECH) (6500) conçu et agencé pour retenir l’humidité provenant d’un flux d’air expiré par le patient, dans lequel l’humidité retenue provenant de l’ECH est renvoyée au flux d’air pour l’humidification; et étant **caractérisé en ce qu’**il comprend en outre:

un dispositif de chauffage régulable prévu au niveau de l’ECH et configuré et agencé pour chauffer directement l’ECH; et
un régulateur configuré pour ajuster le chauffage du dispositif de chauffage régulable pour ajuster l’humidité renvoyée de l’ECH au flux d’air pour l’humidification.

**2.** Système CPAP selon la revendication 1, dans lequel l’ECH est prévu dans la chambre de répartition de l’interface patient.

**3.** Système CPAP selon la revendication 1, dans lequel l’ECH est prévu en amont de la chambre de répartition de l’interface patient.

**4.** Système CPAP selon la revendication 3, dans lequel l’ECH est prévu au niveau d’un adaptateur agencé entre le tube d’alimentation en air et l’interface patient.

**5.** Système CPAP selon l’une quelconque des revendications 1 à 4, dans lequel la structure formant joint de l’interface patient comprend des pinces nasales ou un coussinet nasal.

**6.** Système CPAP selon l’une quelconque des revendications 1 à 5, dans lequel le dispositif RPT, l’interface patient et/ou le tube d’alimentation en air comprend un ou plusieurs capteurs, chacun des capteurs configuré pour produire un ou plusieurs signaux de sortie communiqués au régulateur, et dans lequel l’ajustement du dispositif de chauffage régulable est au moins partiellement basé sur lesdits un ou plusieurs signaux de sortie provenant desdits un ou plusieurs capteurs.

**7.** Système CPAP selon la revendication 6, dans lequel le tube d'alimentation en air comprend un capteur de température de gaz.

**8.** Système CPAP selon l'une quelconque des revendications 6 à 7, dans lequel le dispositif RPT comprend un capteur de pression, un capteur de flux, un capteur d'humidité et/ou un capteur de température.

**9.** Système CPAP selon l'une quelconque des revendications 6 à 8, dans lequel le dispositif RPT comprend une entrée patient, et l'ajustement du dispositif de chauffage régulable est au moins partiellement basé sur ladite entrée patient.

**10.** Système CPAP selon la revendication 9, dans lequel l'entrée patient comprend un réglage de température de gaz préféré.

**11.** Système CPAP selon l'une quelconque des revendications 1 à 10, dans lequel le dispositif de chauffage régulable est configuré et agencé pour chauffer l'interface patient, et dans lequel le régulateur est configuré pour ajuster le chauffage du dispositif de chauffage régulable pour ajuster la condensation dans l'interface patient.

**12.** Système CPAP selon l'une quelconque des revendications 1 à 11, comprenant en outre un humidificateur chauffant par passage prévu au niveau du dispositif RPT.

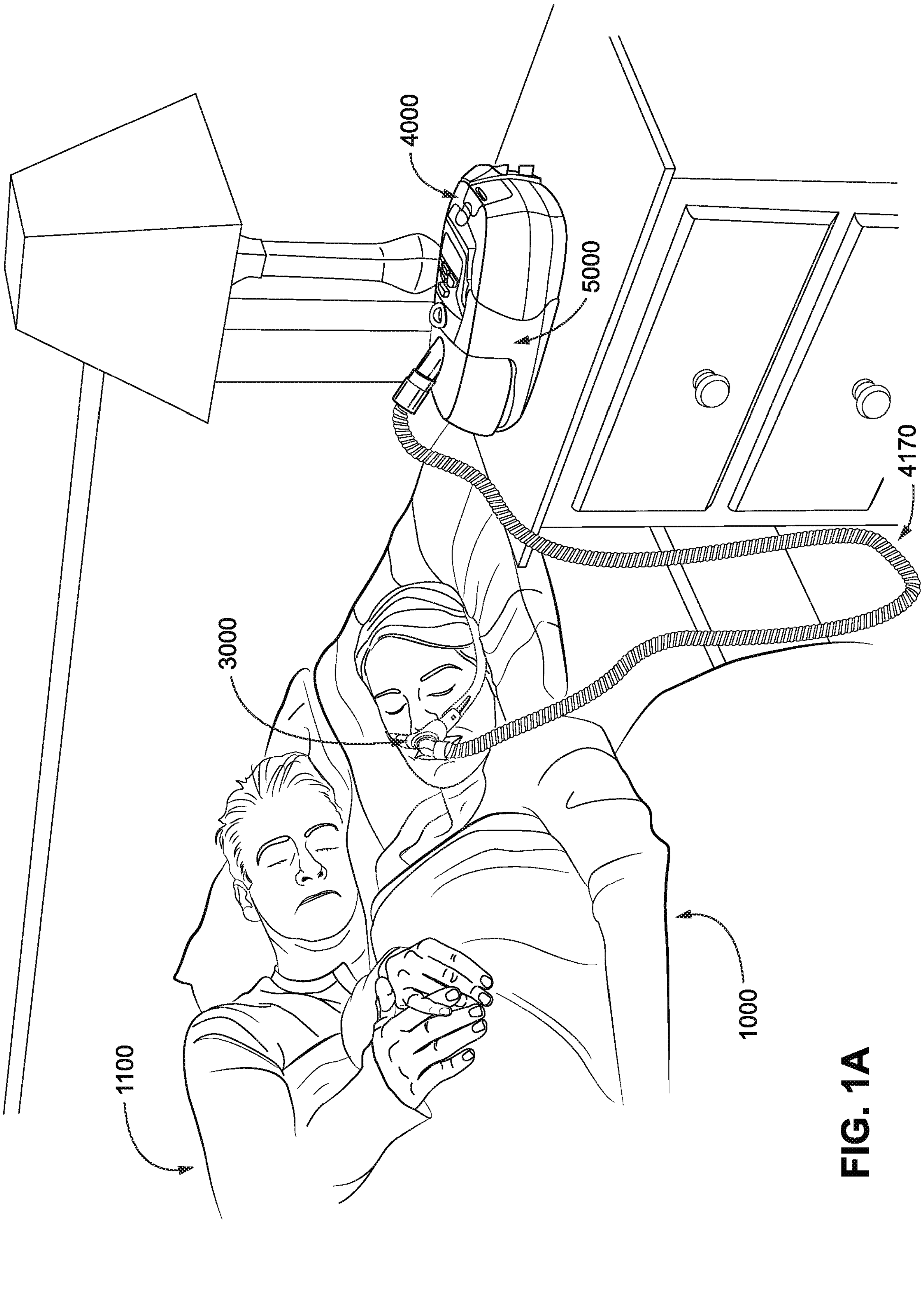


FIG. 1A

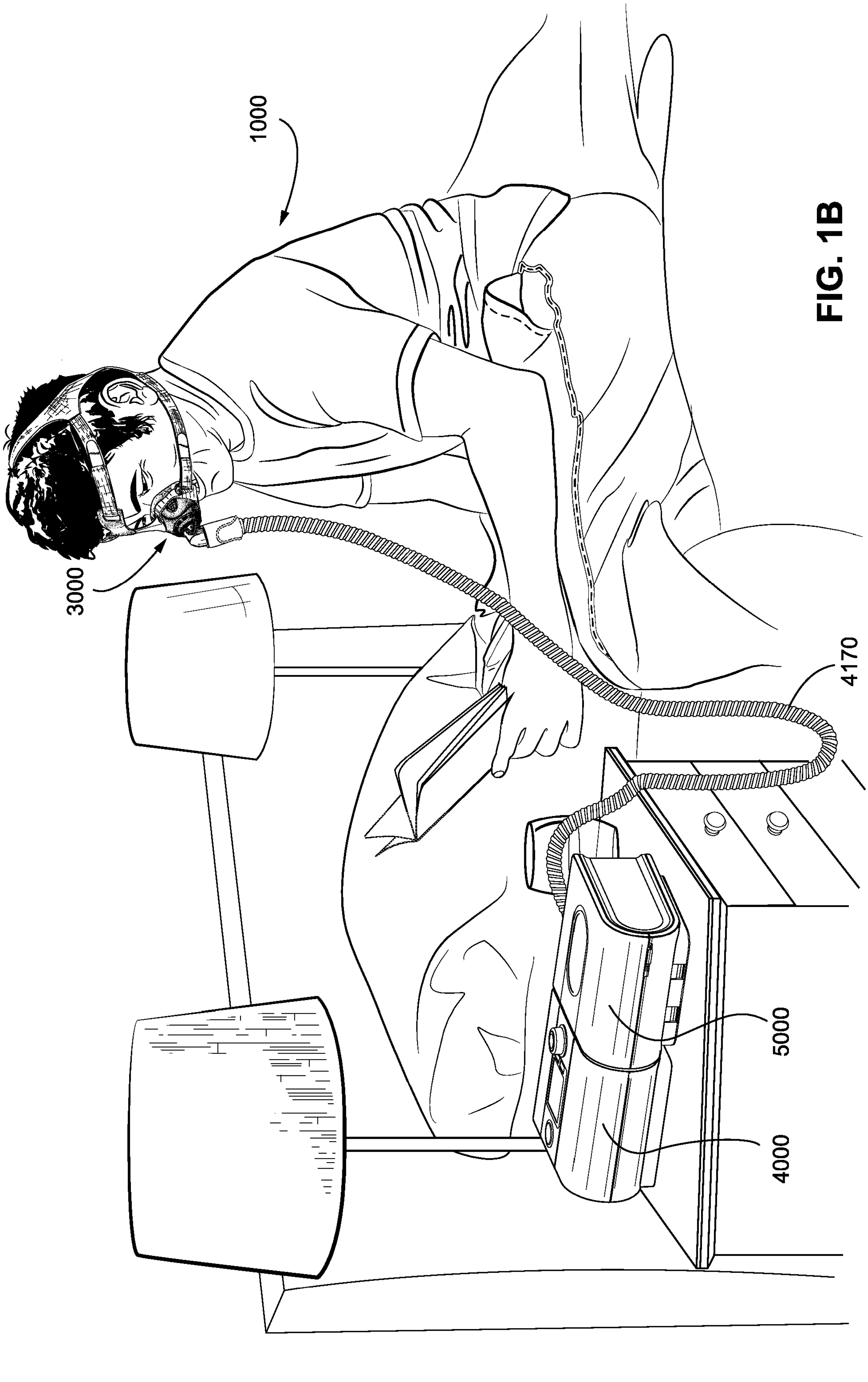


FIG. 1B

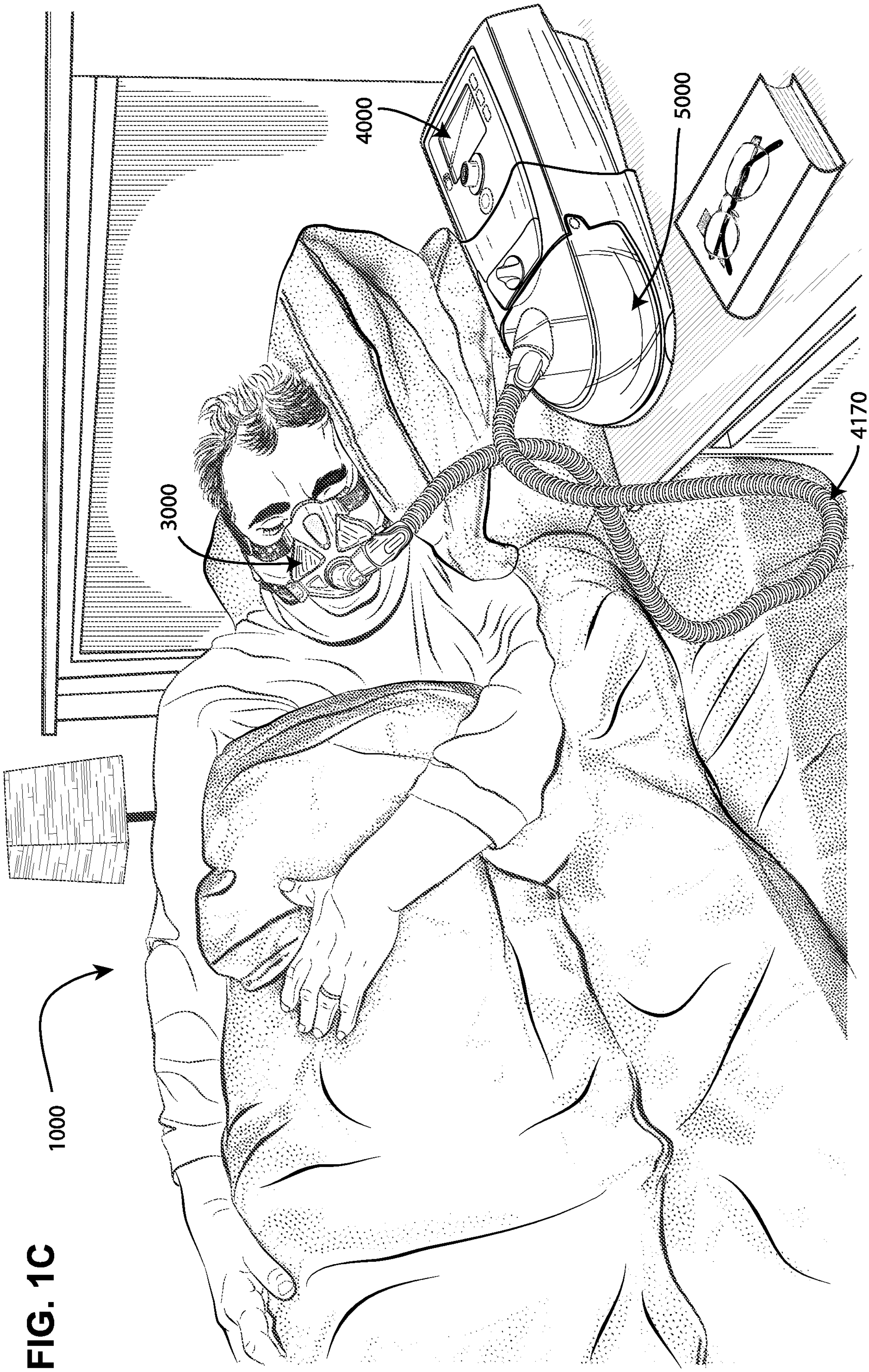


FIG. 1C

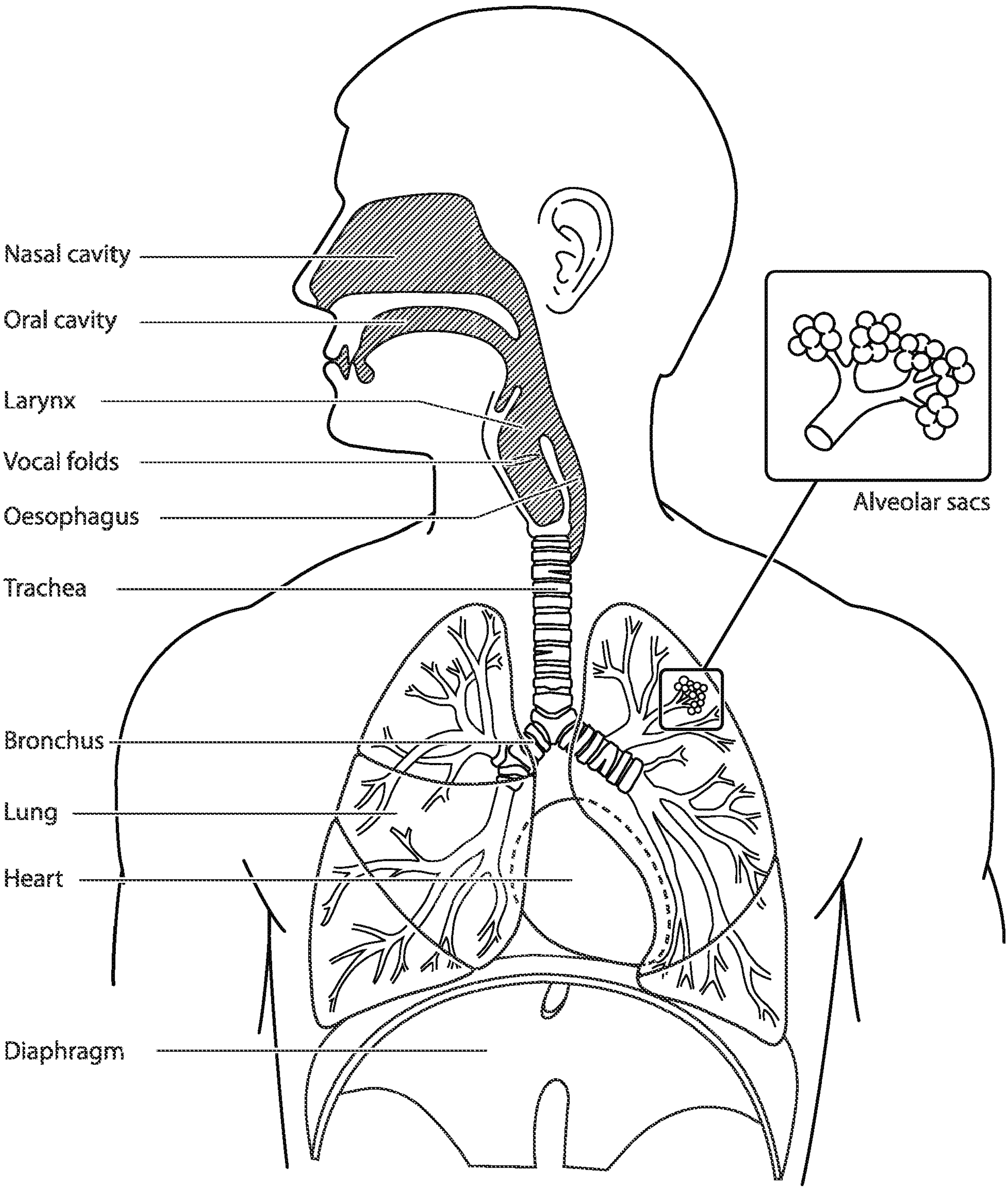
Nasal cavity
Oral cavity
Larynx
Vocal folds
Oesophagus
Trachea
Bronchus
Lung
Heart
Diaphragm
Alveolar sacs

FIG. 2A

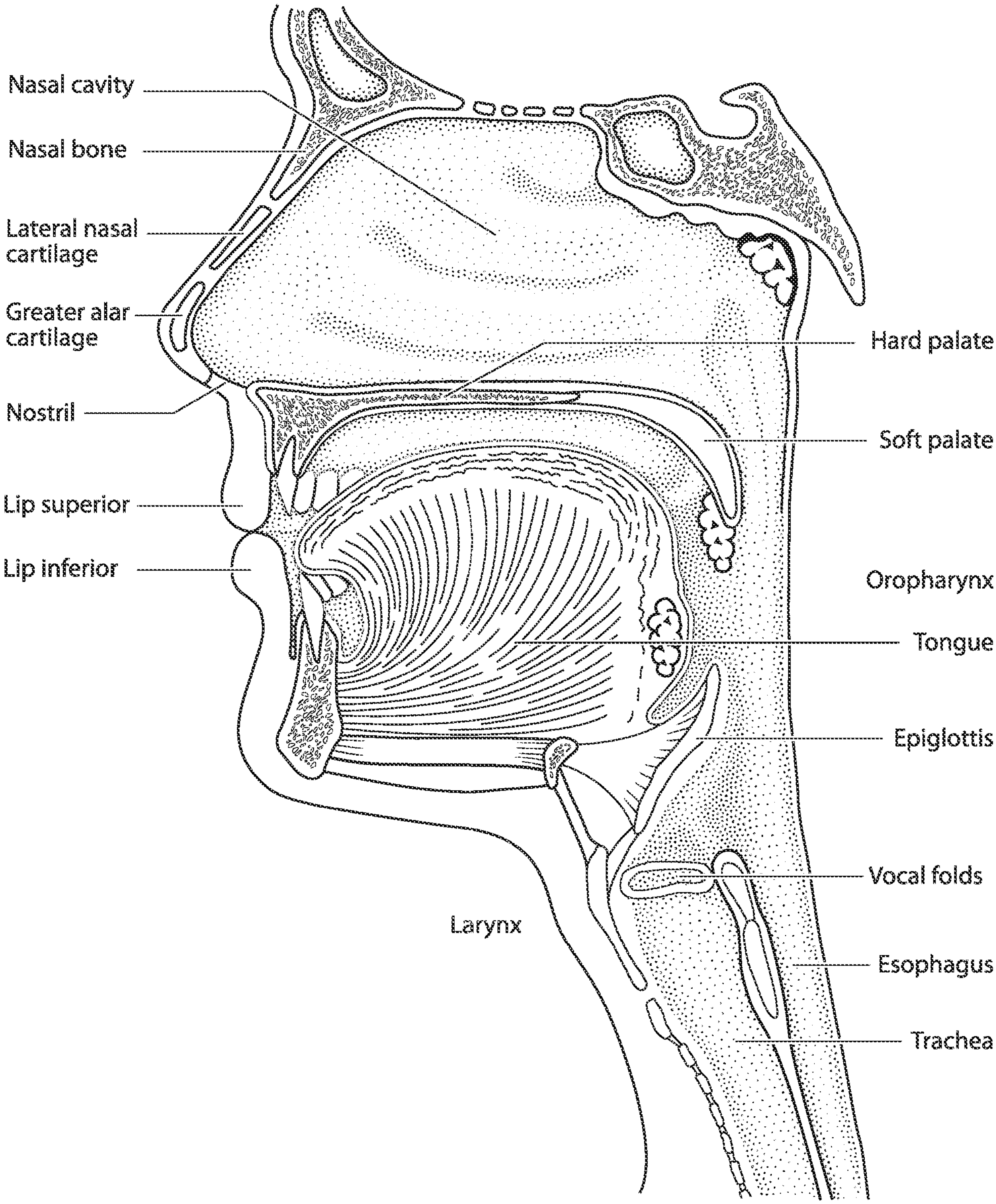
Nasal cavity
Nasal bone
Lateral nasal cartilage
Greater alar cartilage
Nostril
Lip superior
Lip inferior
Hard palate
Soft palate
Oropharynx
Tongue
Epiglottis
Vocal folds
Larynx
Esophagus
Trachea

FIG. 2B

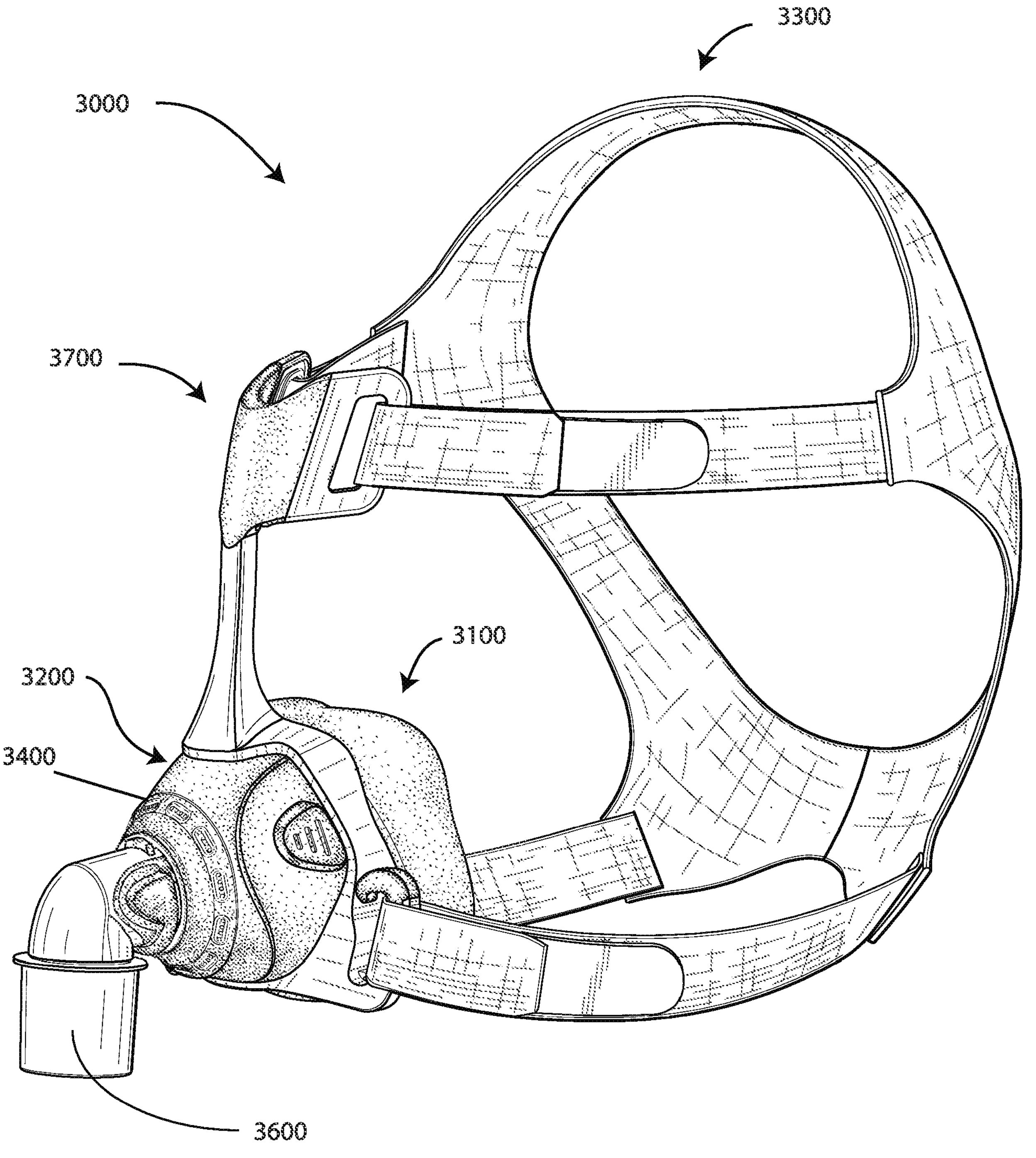


FIG. 3A

FIG. 3B

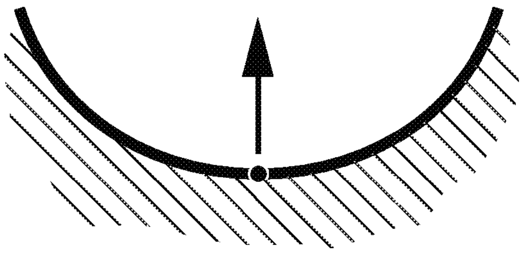

Relatively Large
Positive Curvature

FIG. 3C

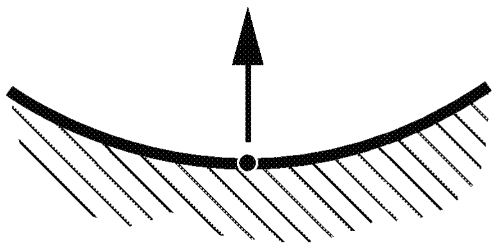

Relatively Small
Positive Curvature

FIG. 3D

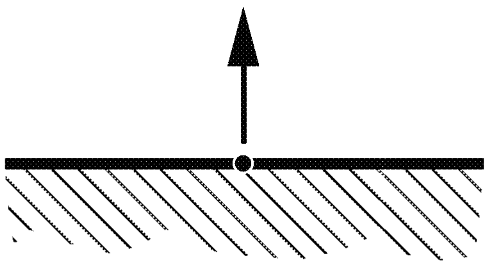

Zero Curvature

FIG. 3E

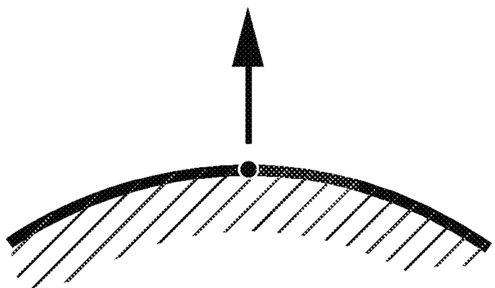

Relatively Small
Negative Curvature

FIG. 3F

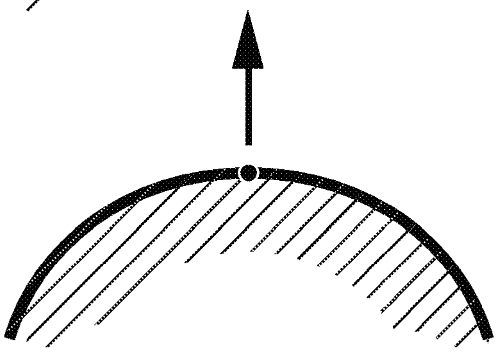

Relatively Large
Negative Curvature

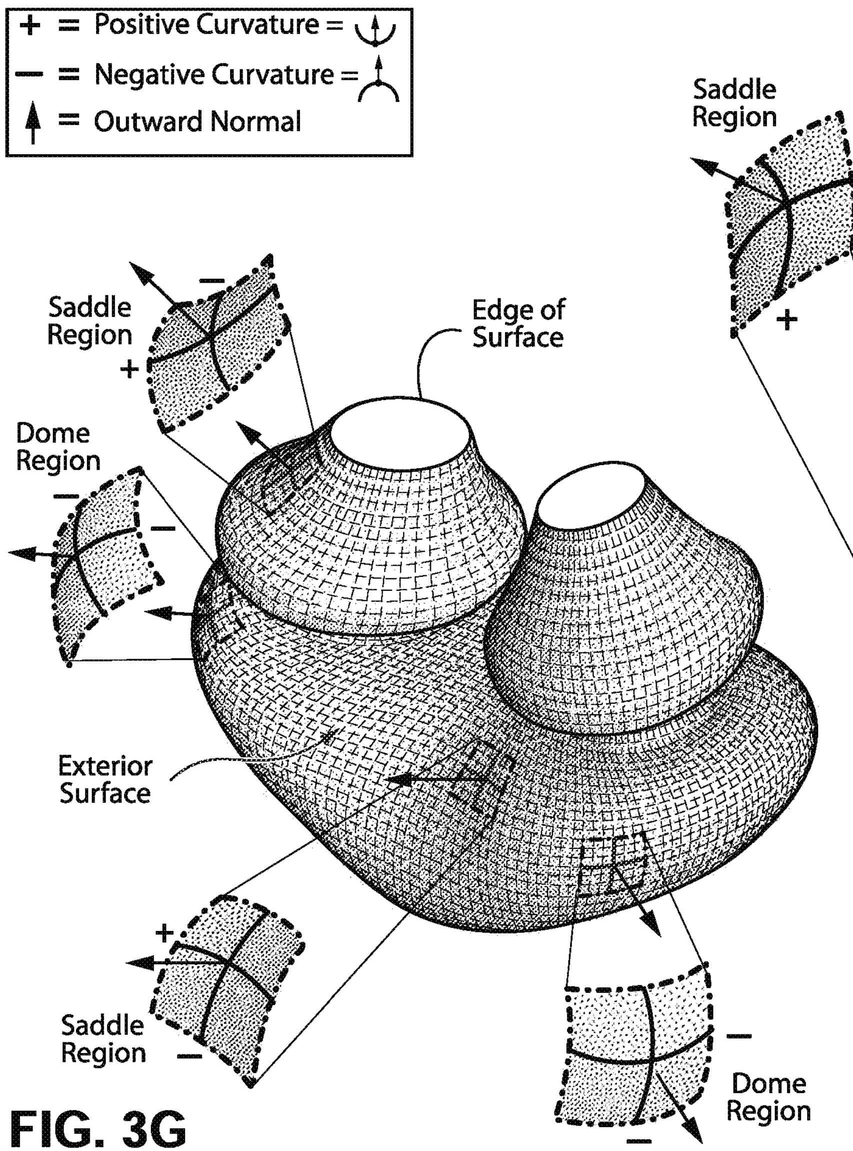

+ = Positive Curvature =
— = Negative Curvature =
= Outward Normal
Saddle Region
+
—
Edge of Surface
Dome Region
—
—
Exterior Surface
+
Saddle Region
—
—
Dome Region
—


FIG. 3G

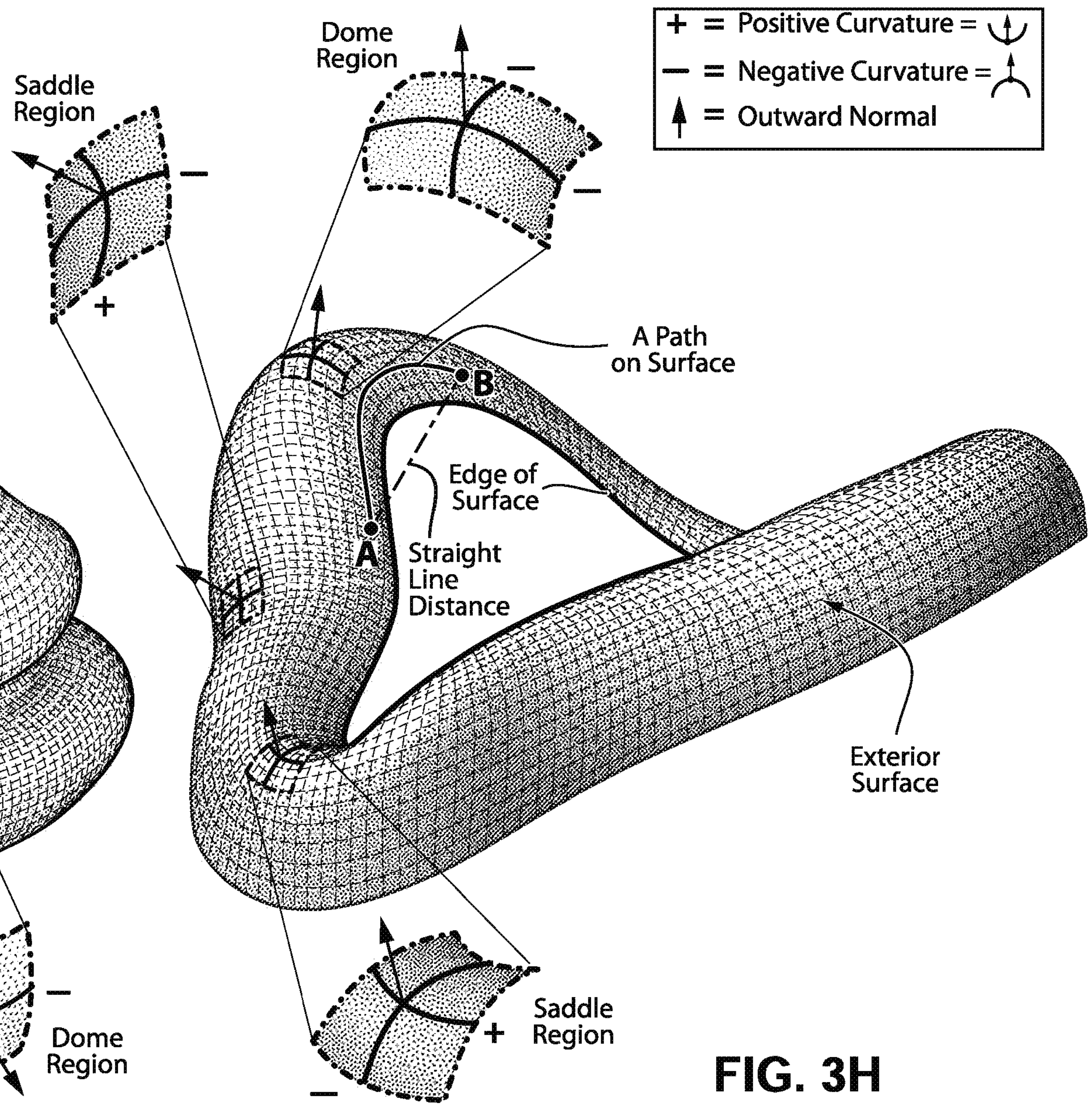

+ = Positive Curvature =
— = Negative Curvature =
= Outward Normal
Saddle Region
—
+
Dome Region
—
—
A Path on Surface
B
A
Edge of Surface
Straight Line Distance
Exterior Surface
Saddle Region
+
—


FIG. 3H

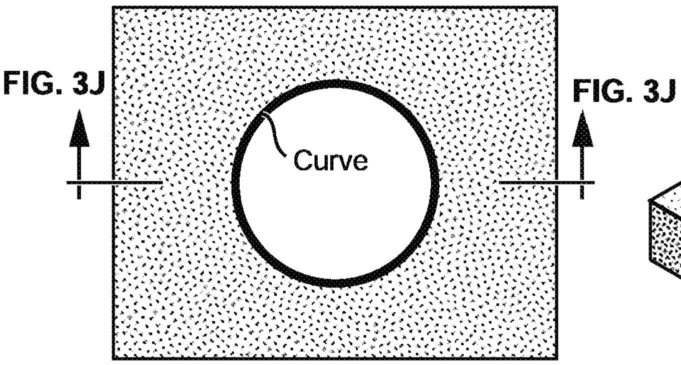


FIG. 3I

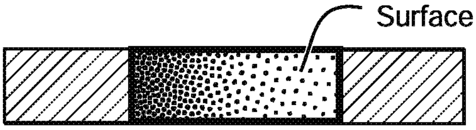


FIG. 3J

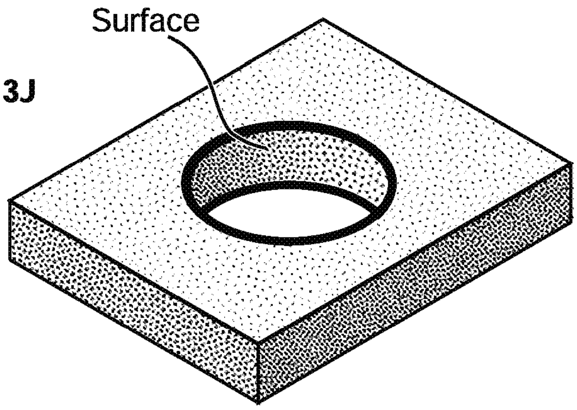


FIG. 3K

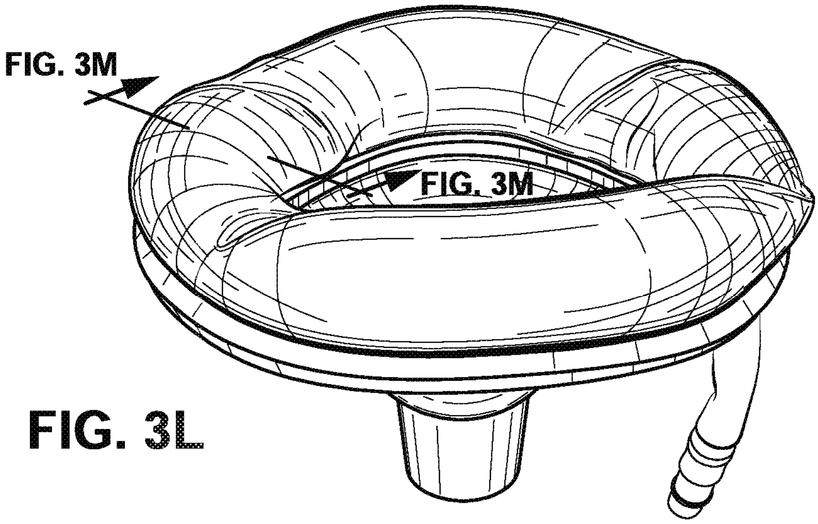


FIG. 3L

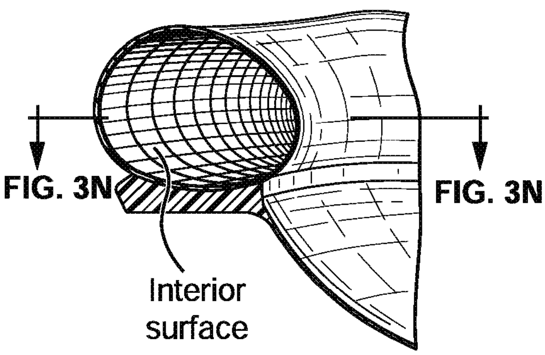


FIG. 3M

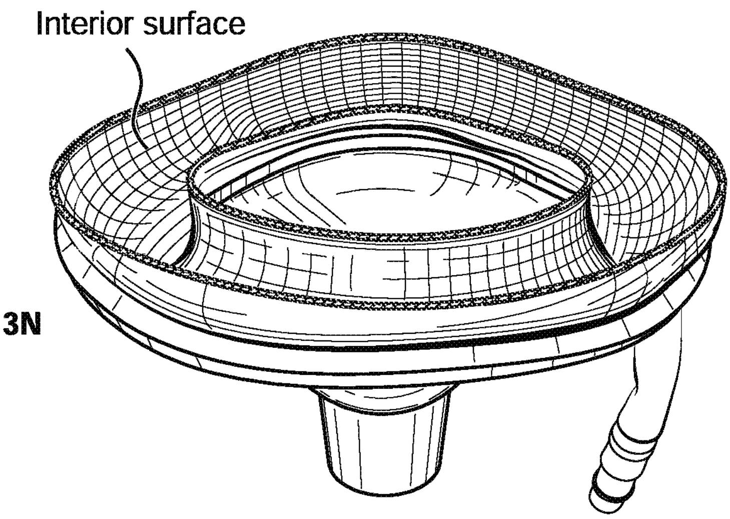


FIG. 3N

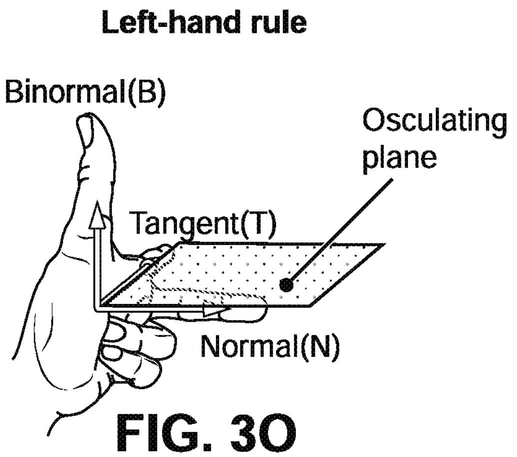


FIG. 3O

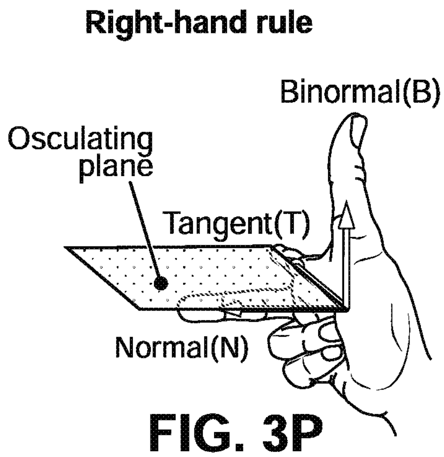


FIG. 3P

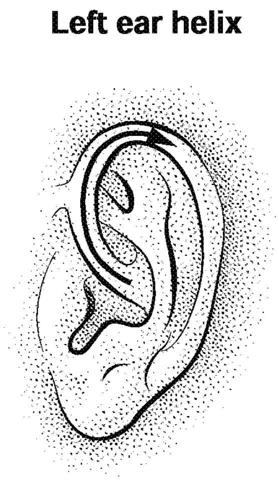


FIG. 3Q

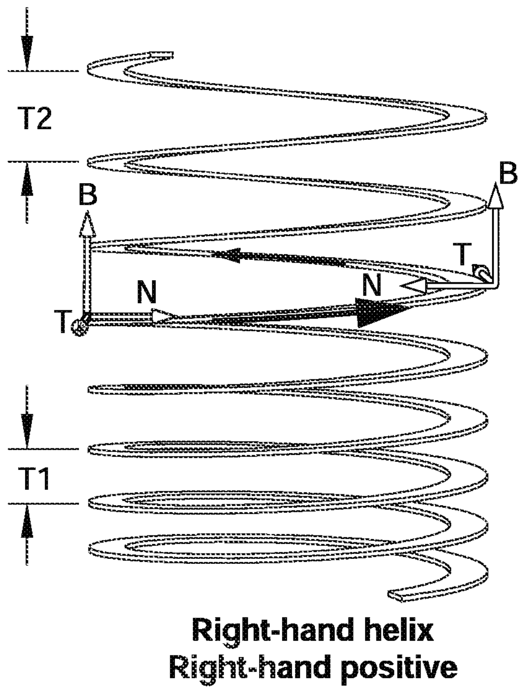


FIG. 3S

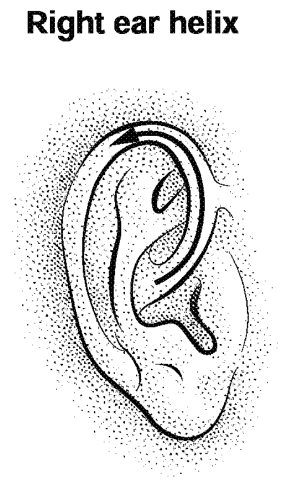


FIG. 3R

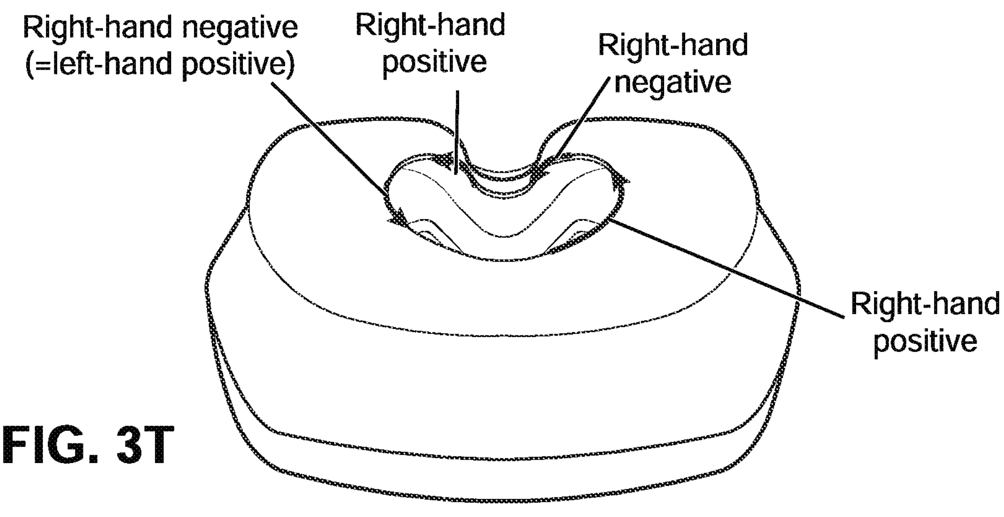


FIG. 3T

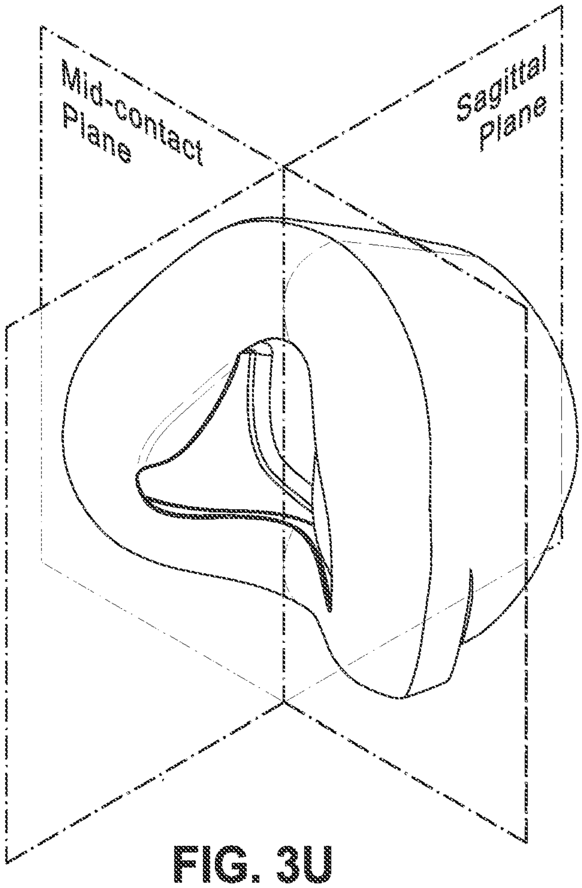


FIG. 3U

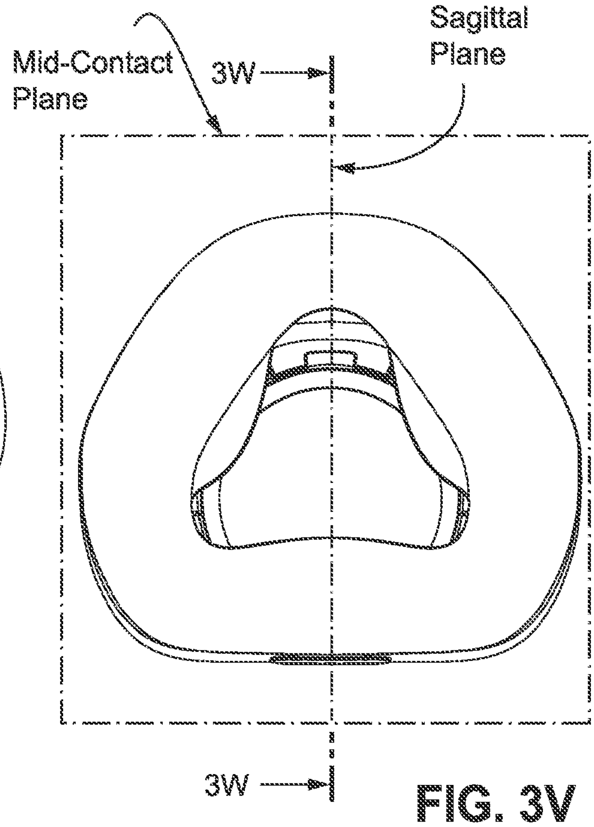


FIG. 3V

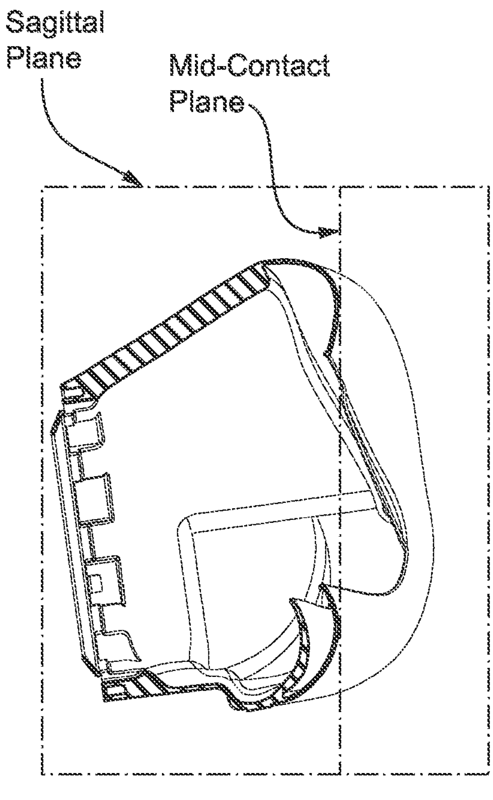


FIG. 3W

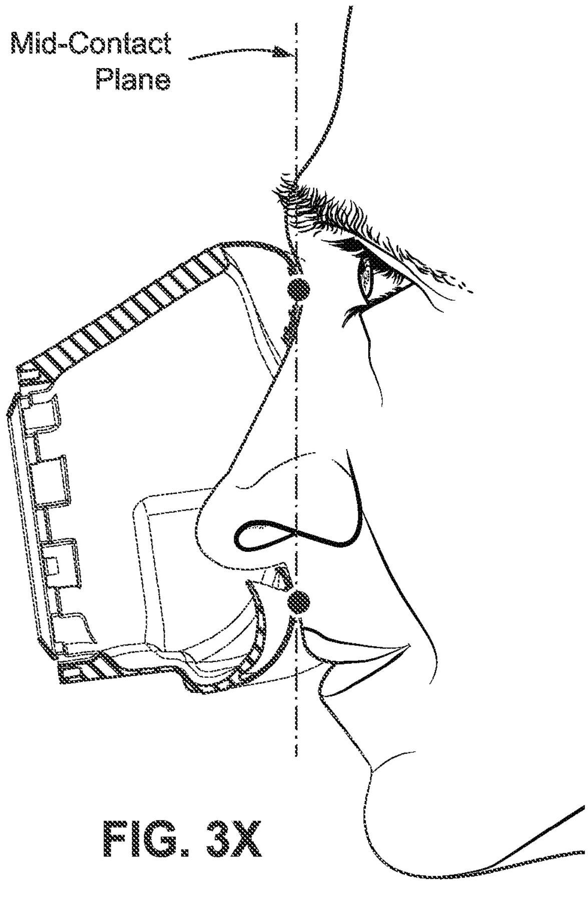


FIG. 3X

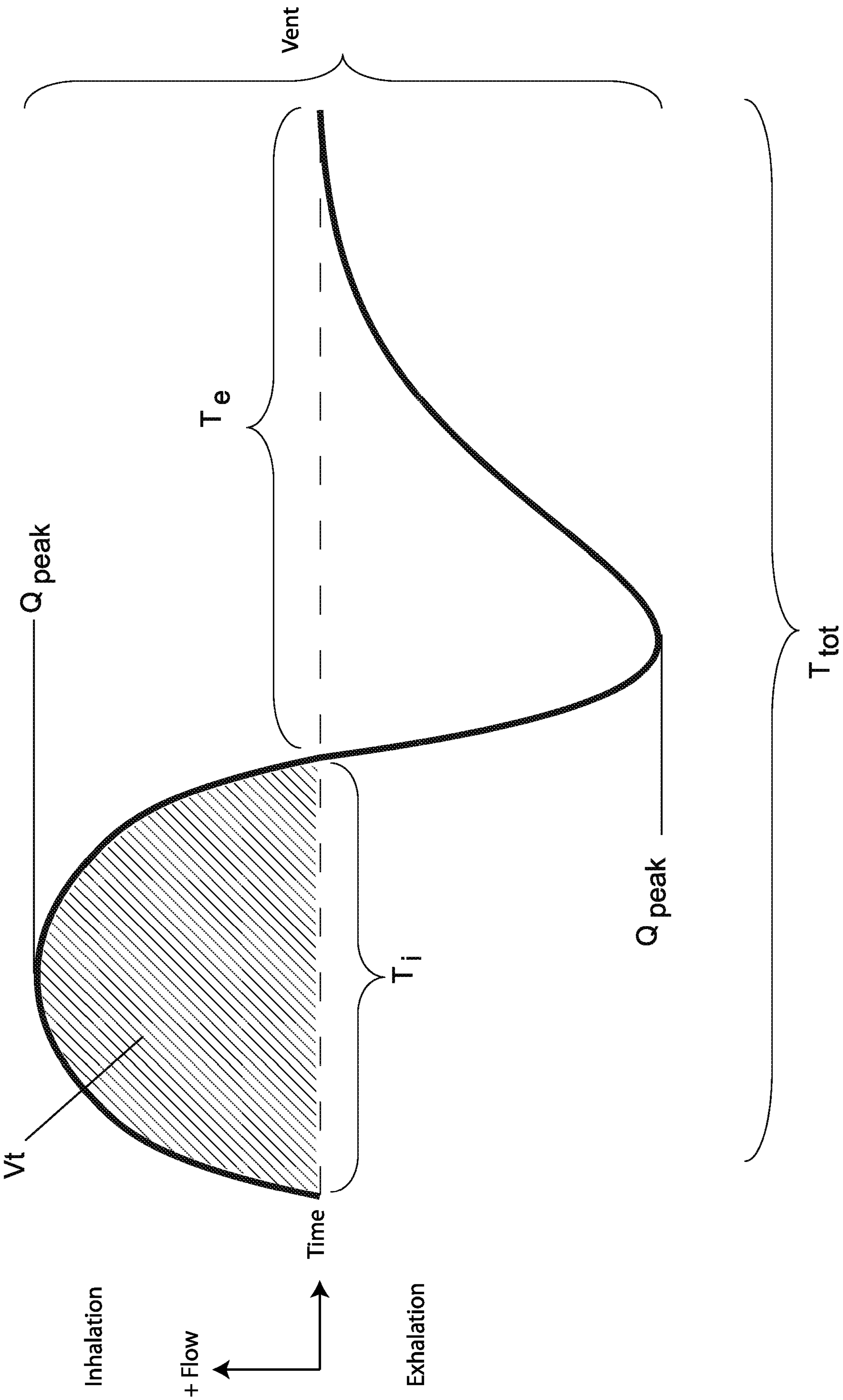
Vent
$Q_{peak}$
$T_e$
$T_{tot}$
Vt
$T_i$
$Q_{peak}$
Time
Inhalation
+ Flow
Exhalation

FIG. 4

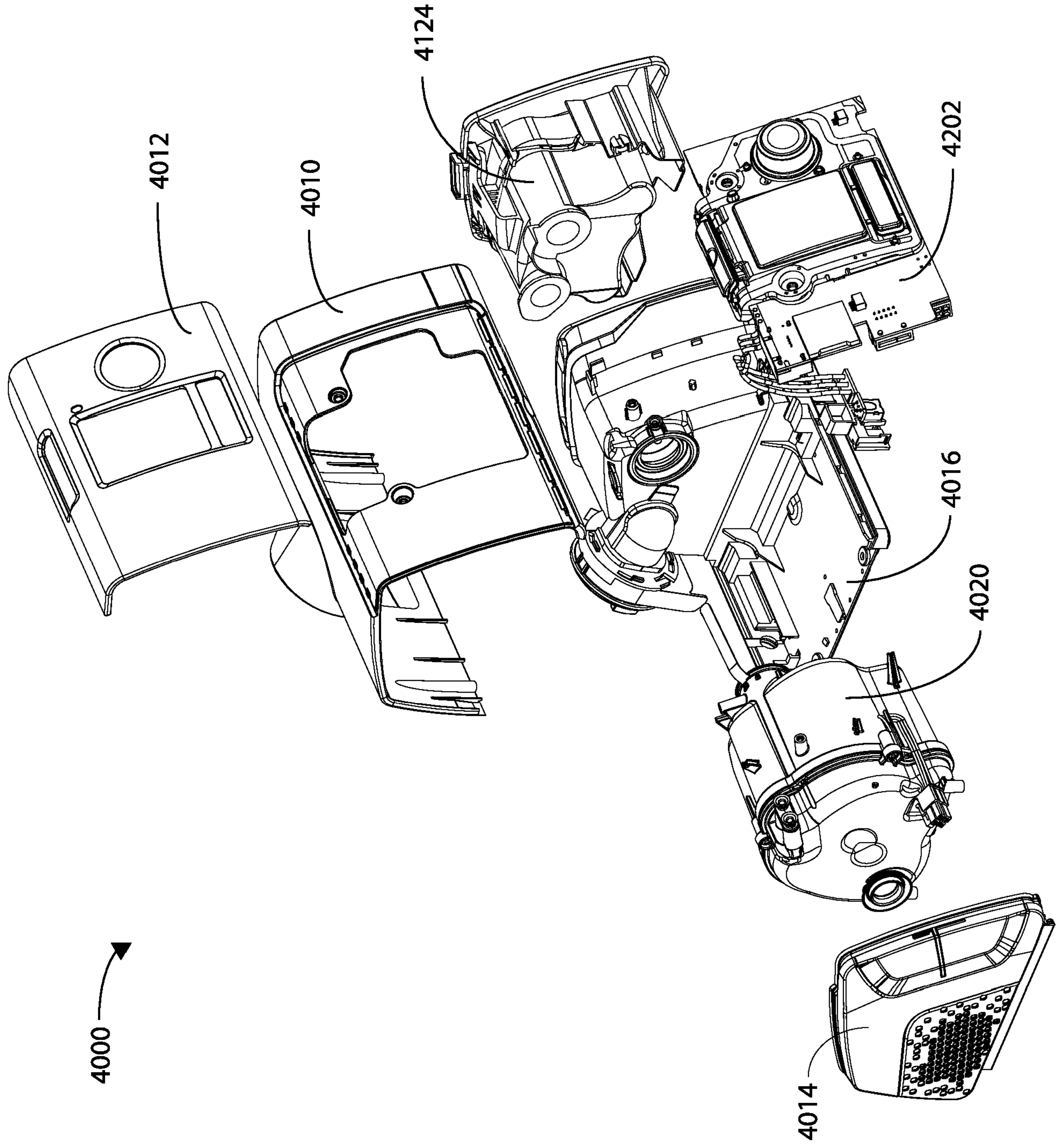


FIG. 5A

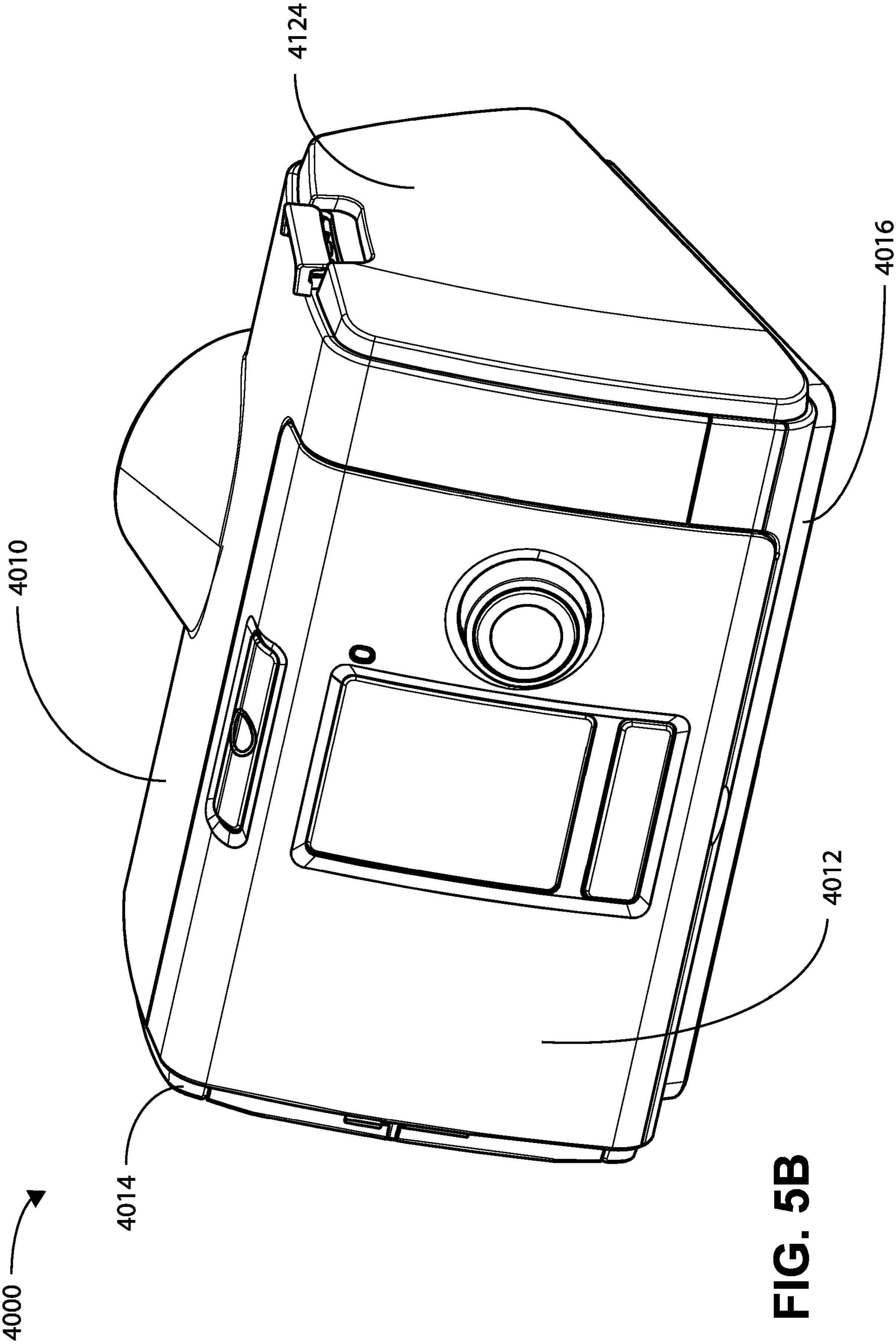


FIG. 5B

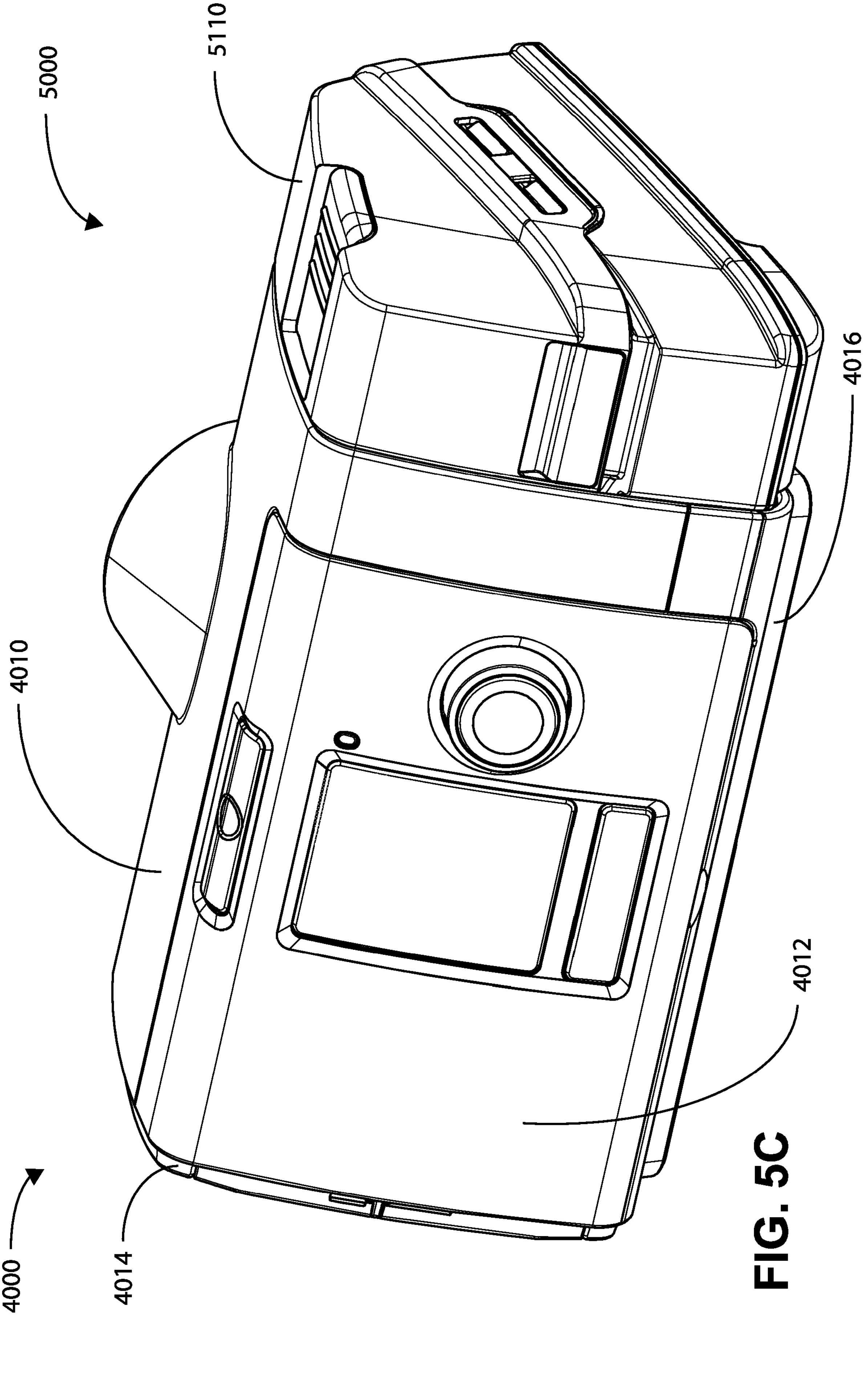


FIG. 5C

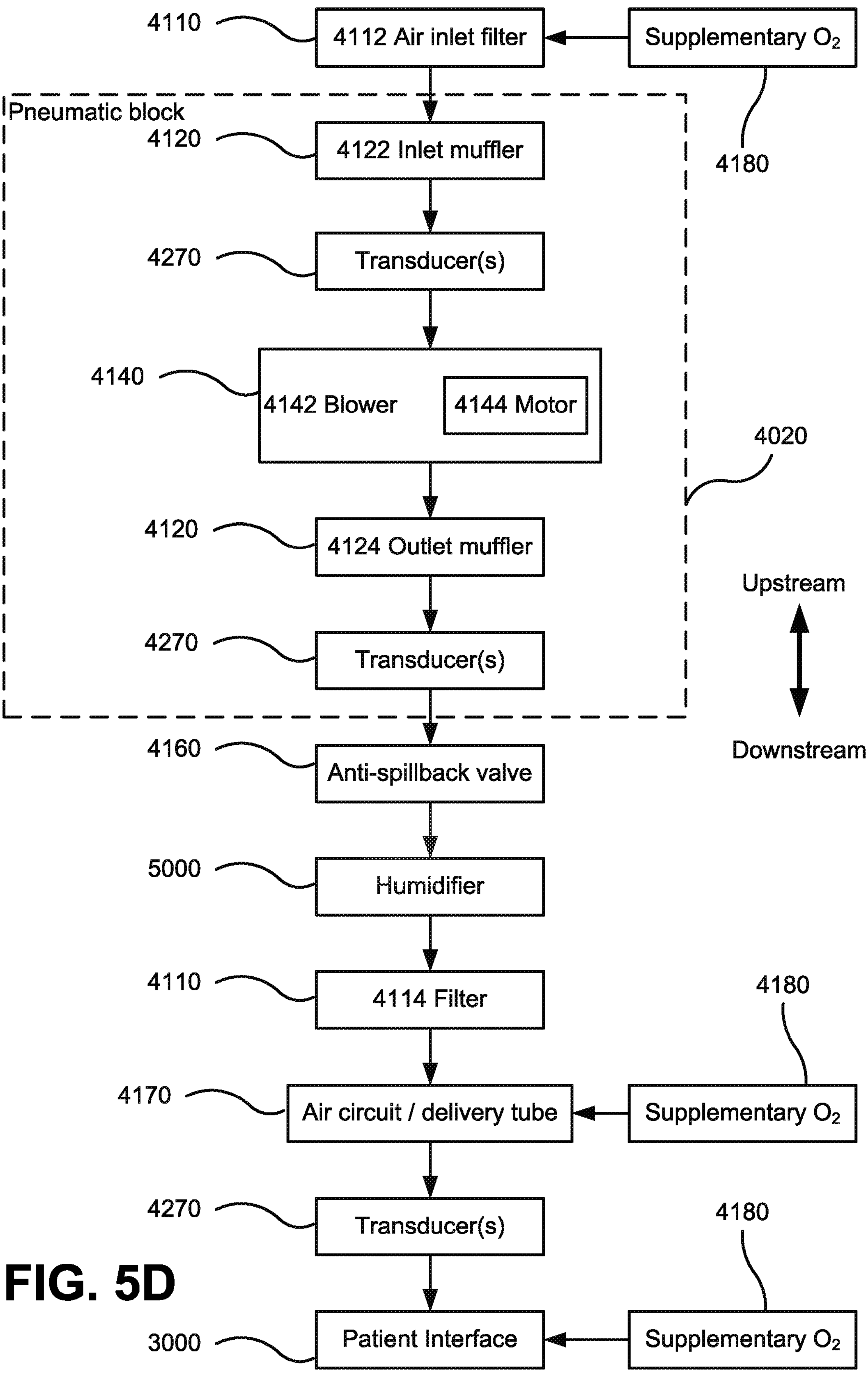
4110
4112 Air inlet filter
Supplementary $O_2$
4180
Pneumatic block
4120
4122 Inlet muffler
4270
Transducer(s)
4140
4142 Blower
4144 Motor
4020
4120
4124 Outlet muffler
Upstream
4270
Transducer(s)
Downstream
4160
Anti-spillback valve
5000
Humidifier
4110
4114 Filter
4180
4170
Air circuit / delivery tube
Supplementary $O_2$
4270
Transducer(s)
4180
3000
Patient Interface
Supplementary $O_2$

FIG. 5D

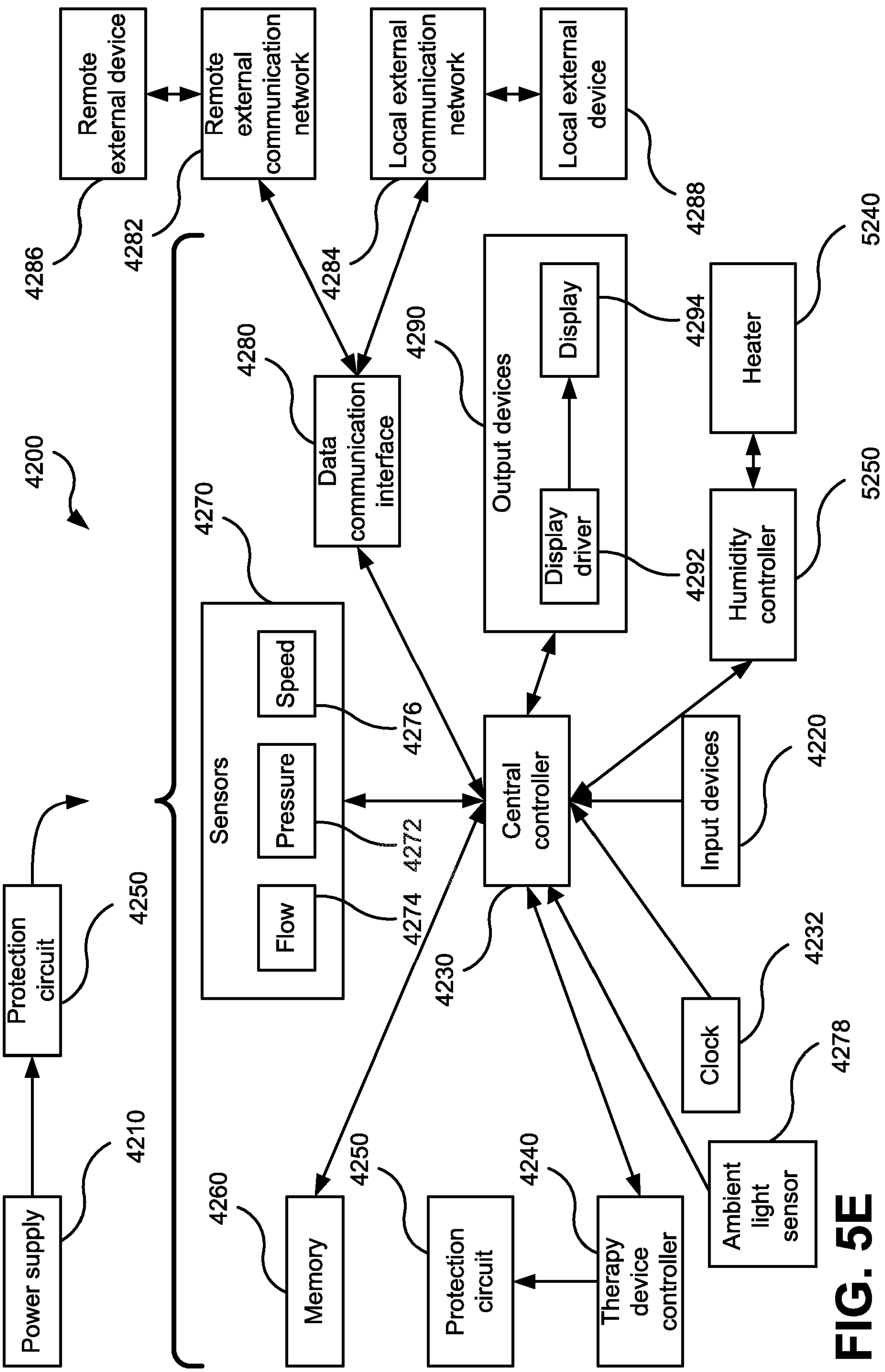
4200
Power supply
4210
Protection circuit
4250
Remote external device
4286
Remote external communication network
4282
Local external communication network
4284
Local external device
4288
Sensors
4270
Flow
4274
Pressure
4272
Speed
4276
Data communication interface
4280
Output devices
4290
Display driver
4292
Display
4294
Heater
5240
Humidity controller
5250
Central controller
4230
Input devices
4220
Clock
4232
Ambient light sensor
4278
Memory
4260
Protection circuit
4250
Therapy device controller
4240

FIG. 5E

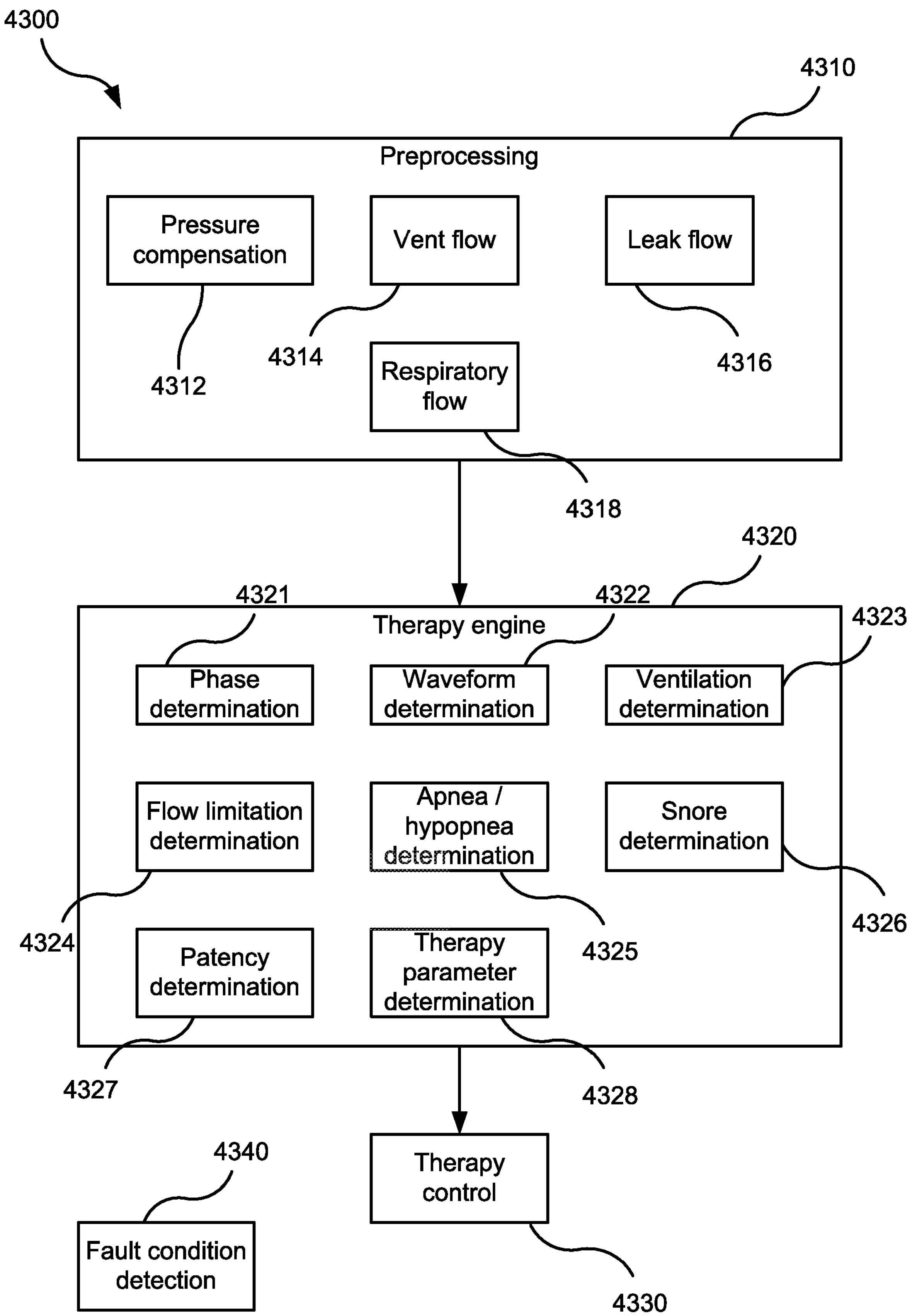
4300
4310
Preprocessing
Pressure compensation
Vent flow
Leak flow
4312
4314
4316
Respiratory flow
4318
4320
4321
4322
4323
Therapy engine
Phase determination
Waveform determination
Ventilation determination
Flow limitation determination
Apnea / hypopnea determination
Snore determination
4324
4325
4326
Patency determination
Therapy parameter determination
4327
4328
4340
Therapy control
Fault condition detection
4330

FIG. 5F

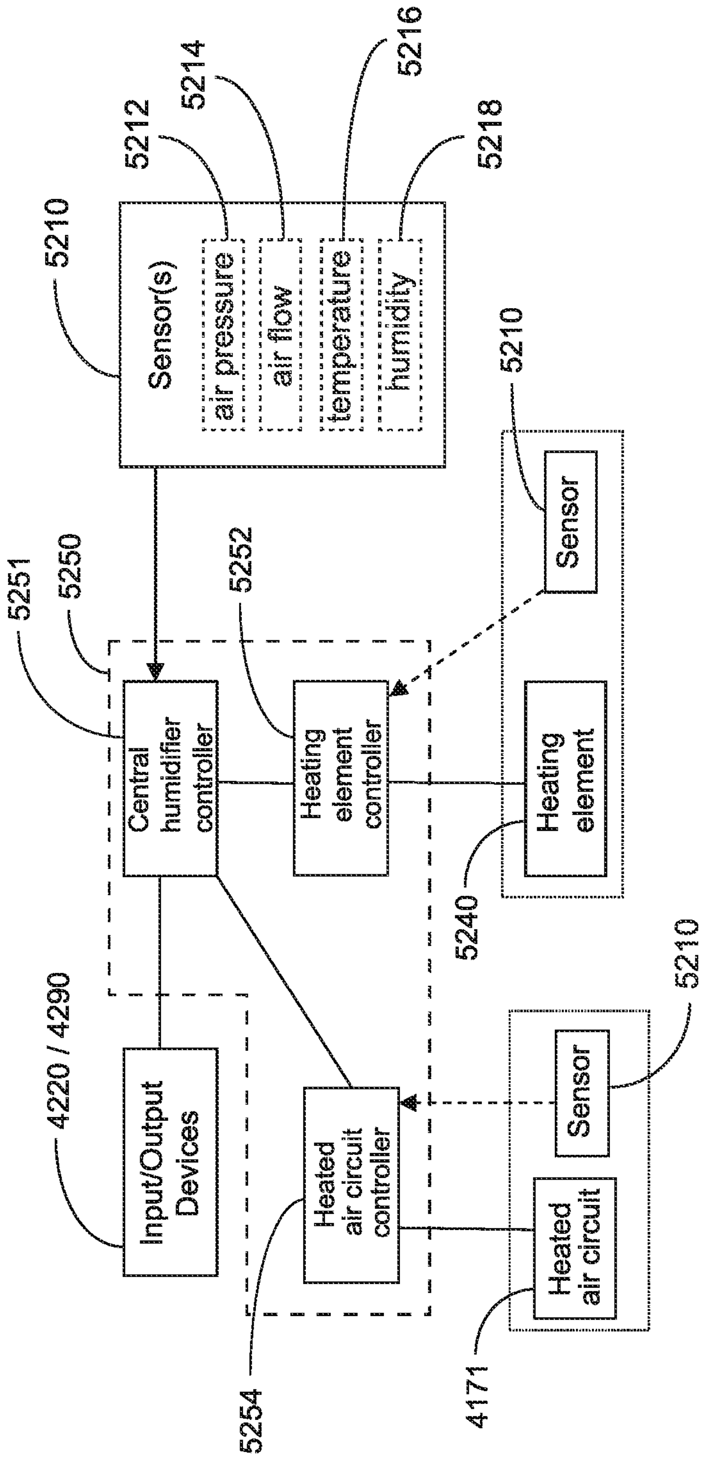
5210
Sensor(s)
air pressure
air flow
temperature
humidity
5212
5214
5216
5218
5251
5250
Central humidifier controller
5252
Heating element controller
5210
Sensor
Heating element
5240
4220 / 4290
Input/Output Devices
Heated air circuit controller
5254
5210
Sensor
Heated air circuit
4171

FIG. 5G

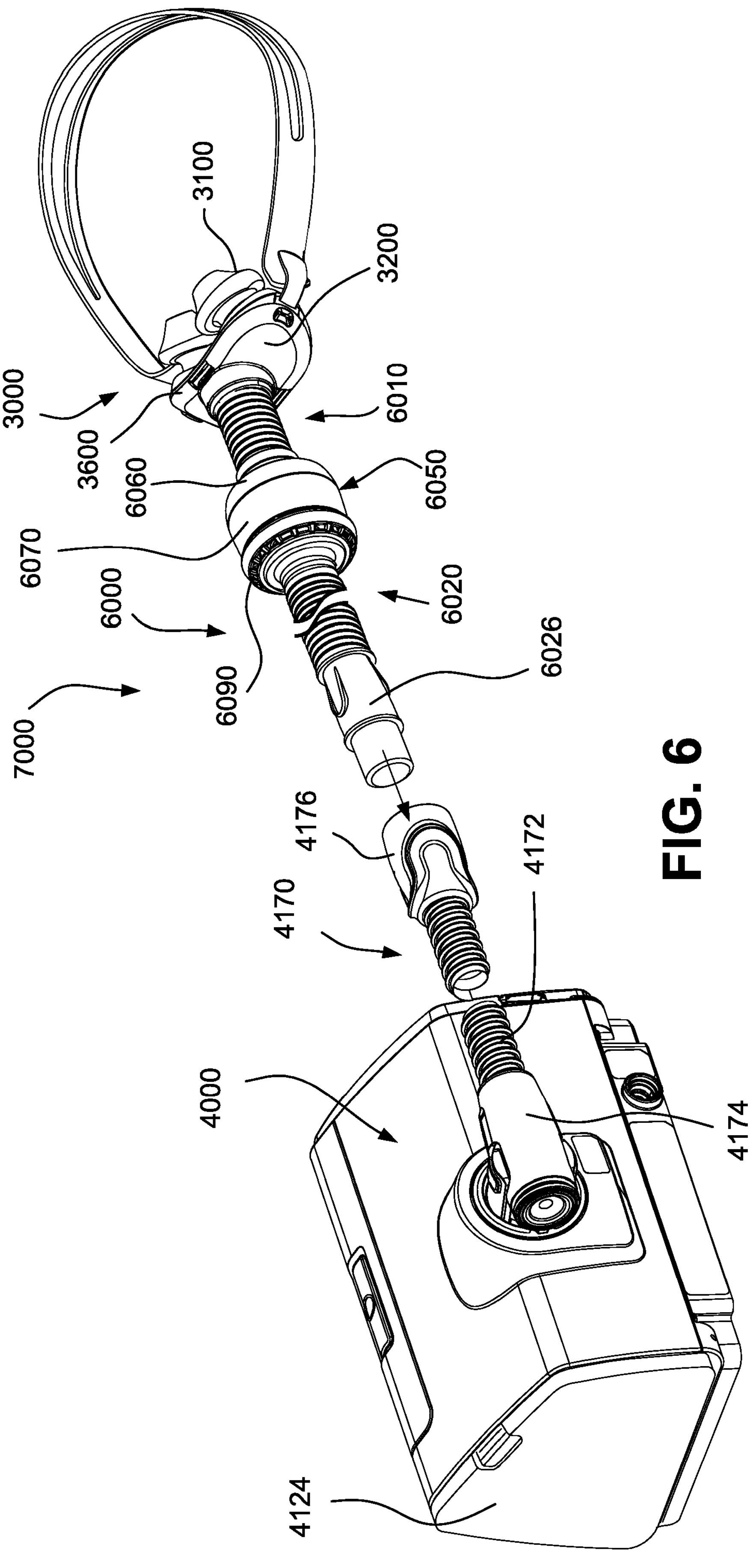
3100
3200
3000
3600
6010
6060
6050
6070
6020
6000
6090
6026
7000
4176
4172
4170
4000
4174
4124

FIG. 6

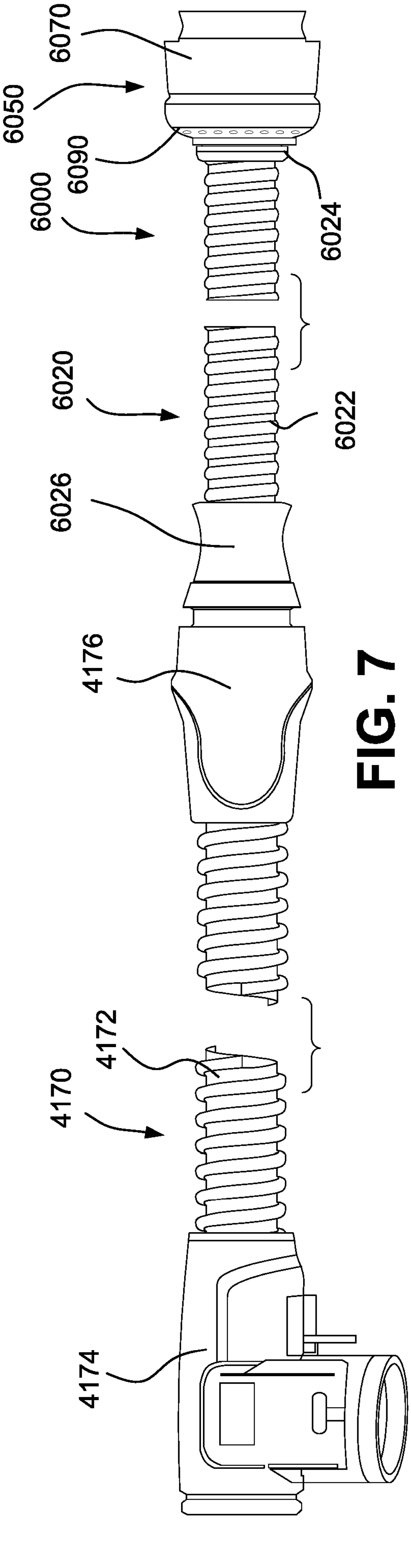
6070
6050
6090
6000
6024
6020
6022
6026
4176
4172
4170
4174

FIG. 7

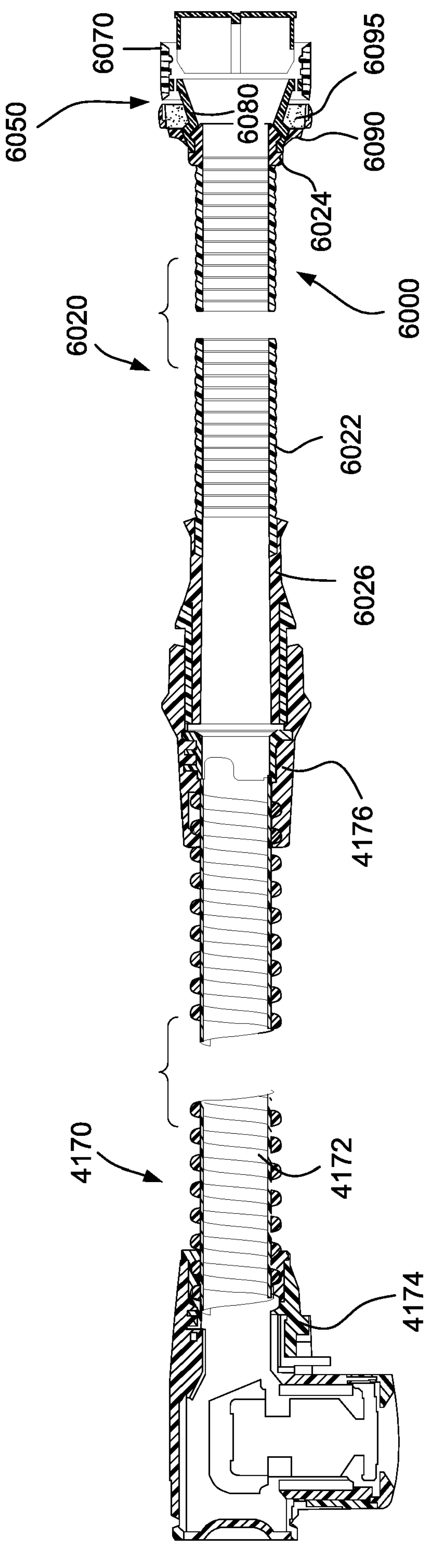
6070
6095
6050
6080
6090
6024
6020
6000
6022
6026
4176
4170
4172
4174

FIG. 8

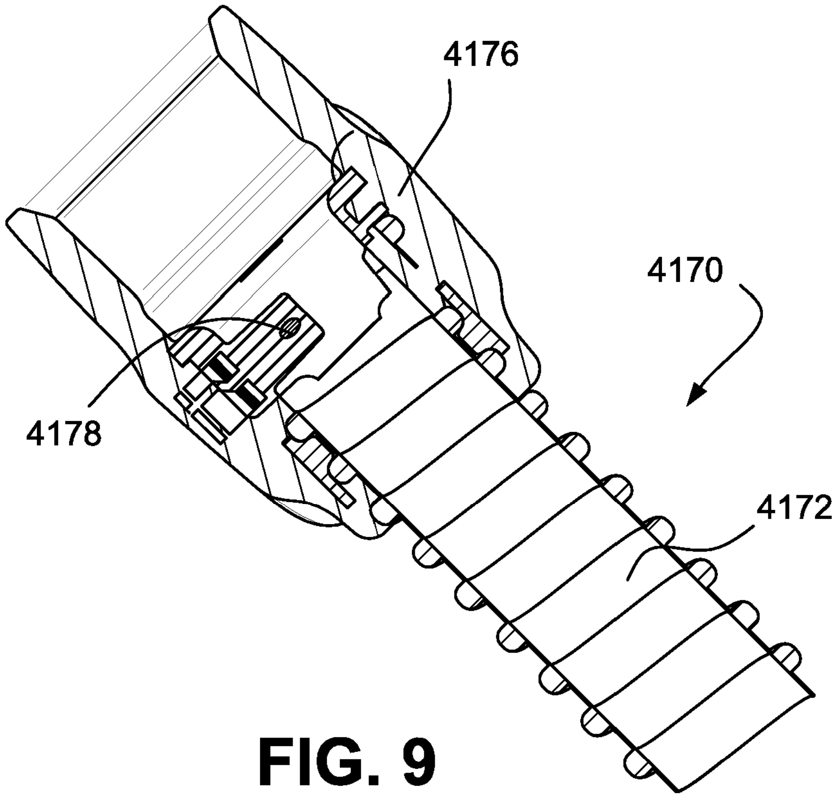


FIG. 9

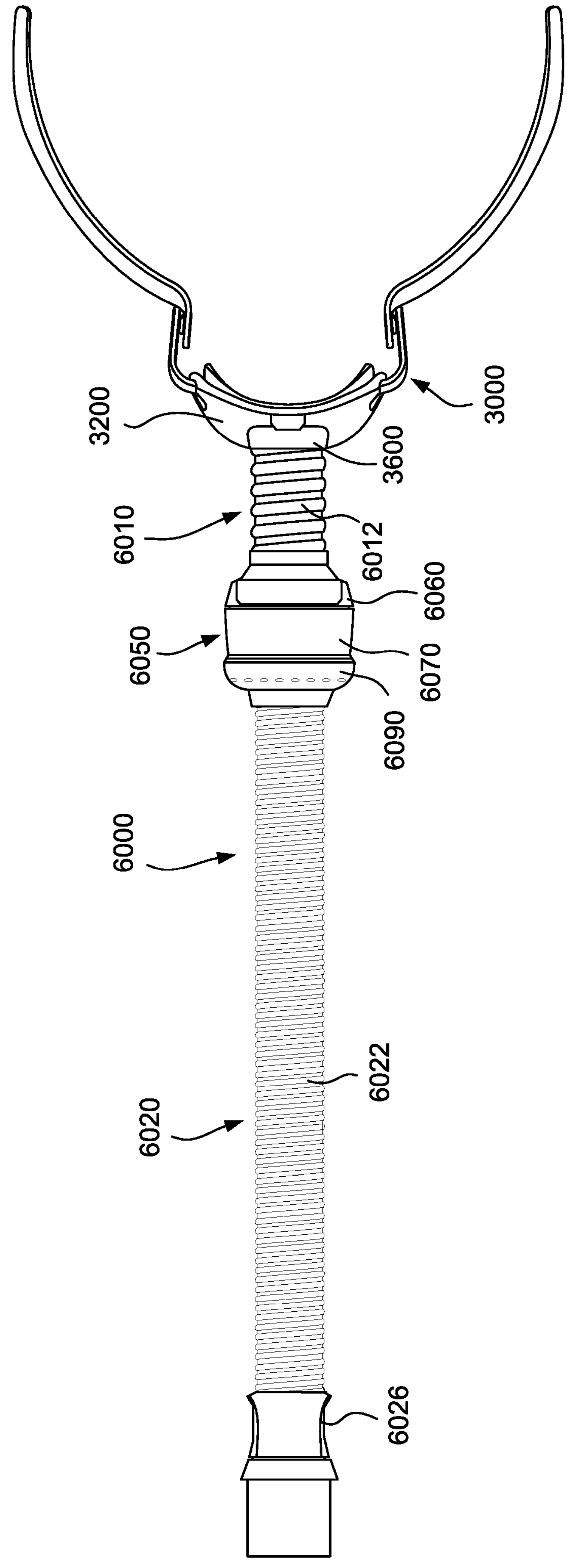

3000
3200
3600
6010
6012
6060
6050
6070
6090
6000
6022
6020
6026


FIG. 10

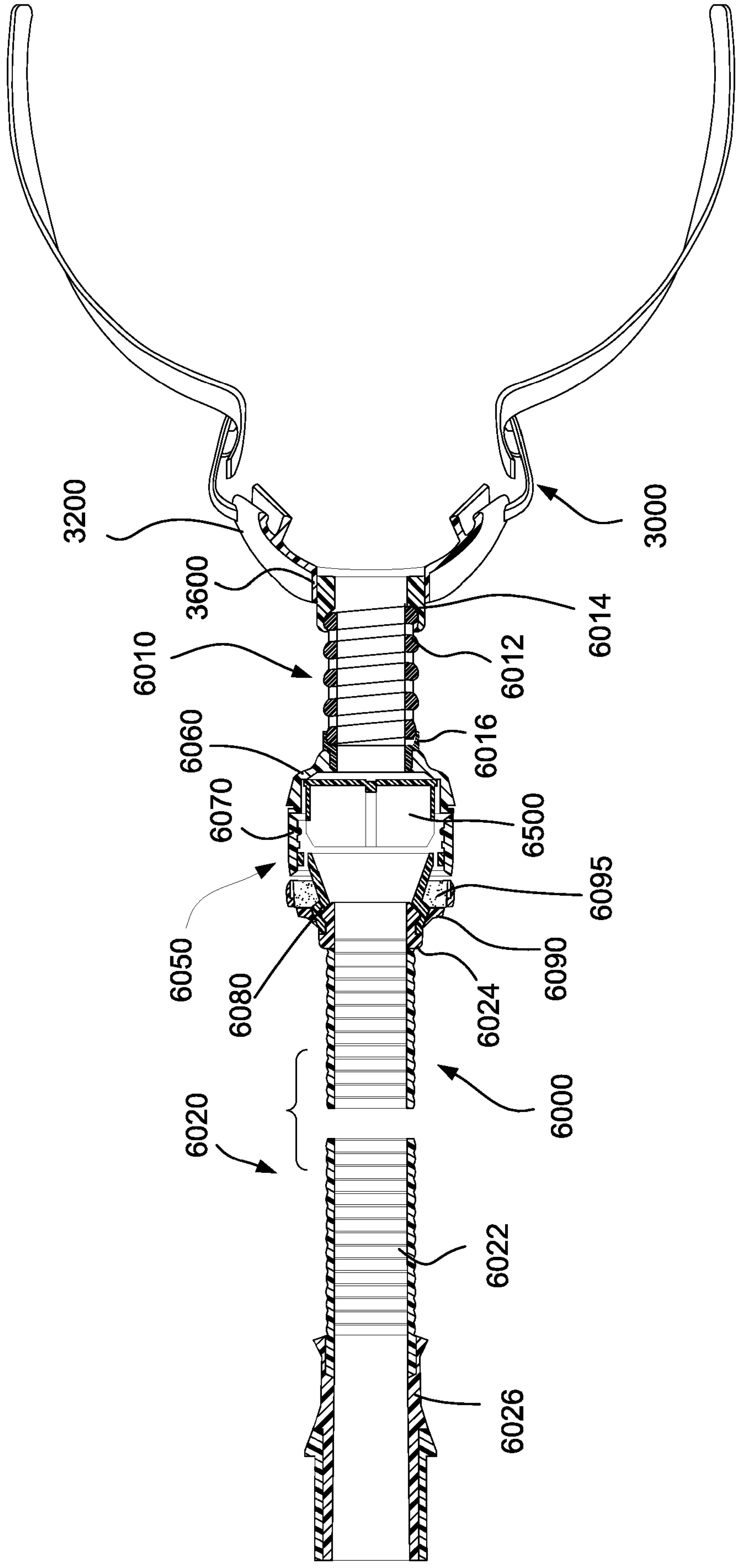
3200
3000
3600
6014
6010
6012
6060
6016
6070
6500
6050
6095
6080
6090
6024
6000
6020
6022
6026

FIG. 11

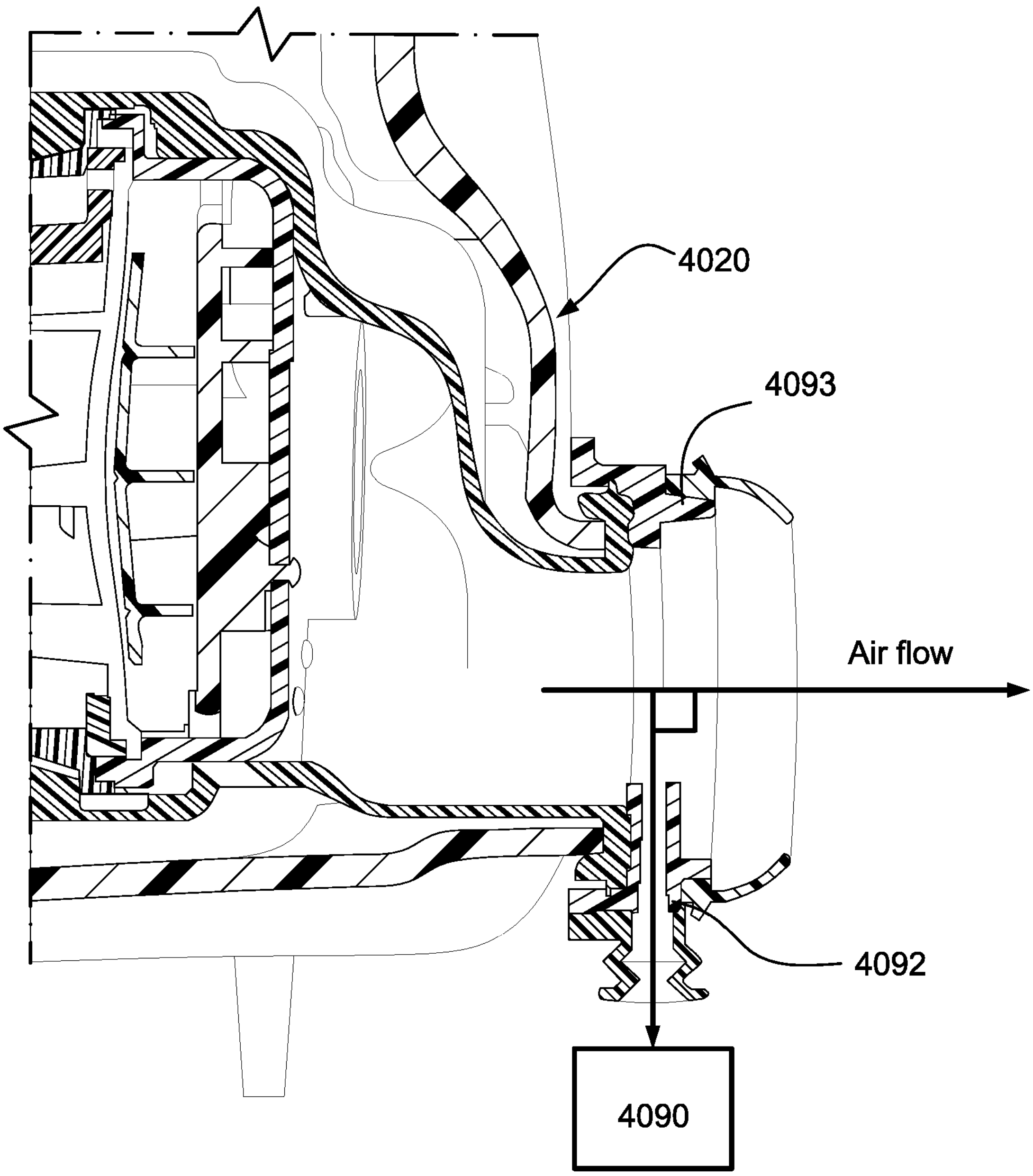


FIG. 12

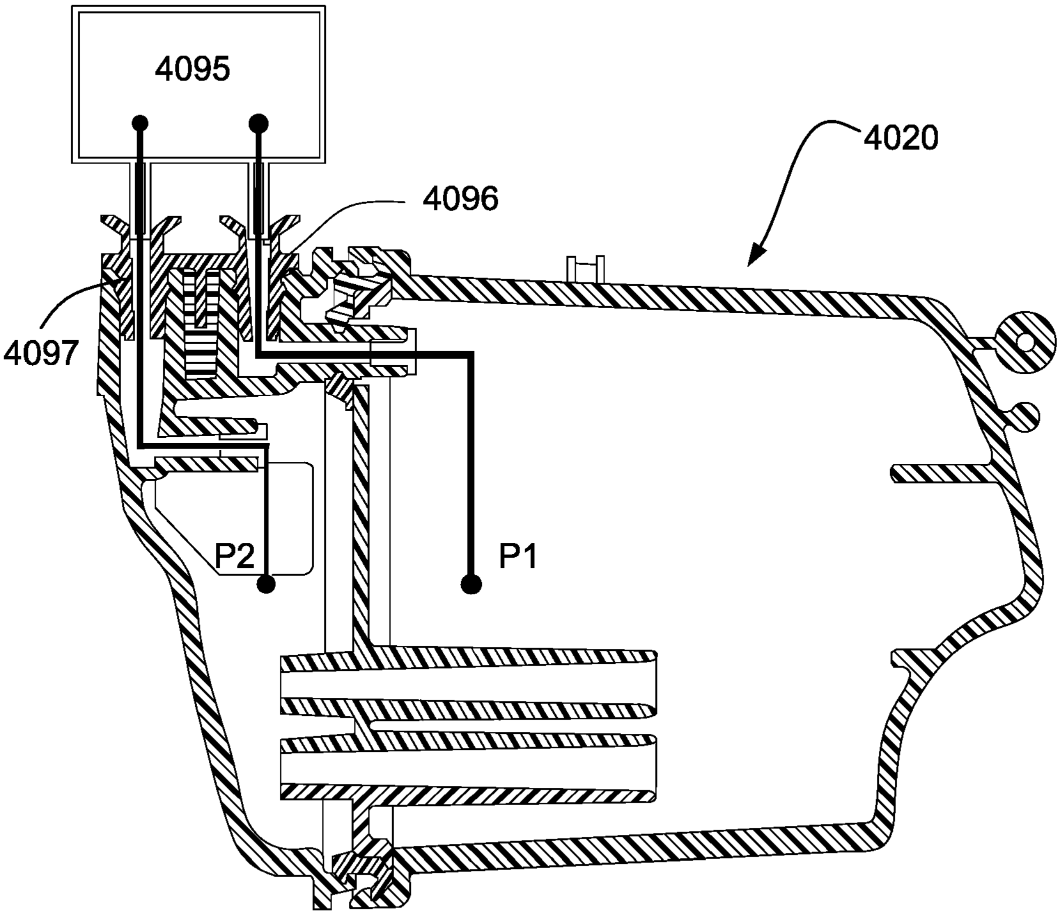


FIG. 13

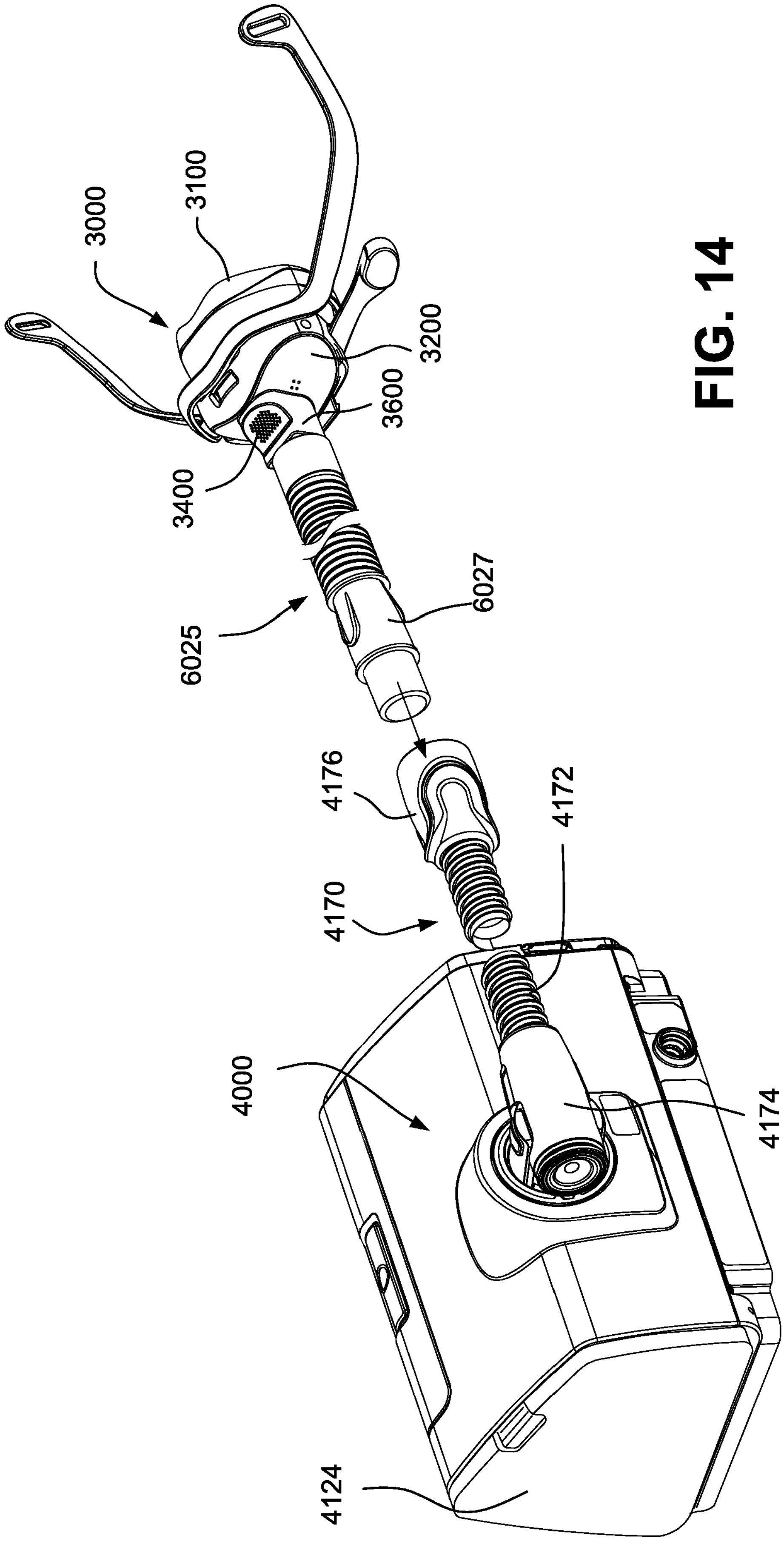
3000
3100
3200
3600
3400
6025
6027
4176
4172
4170
4000
4174
4124

FIG. 14

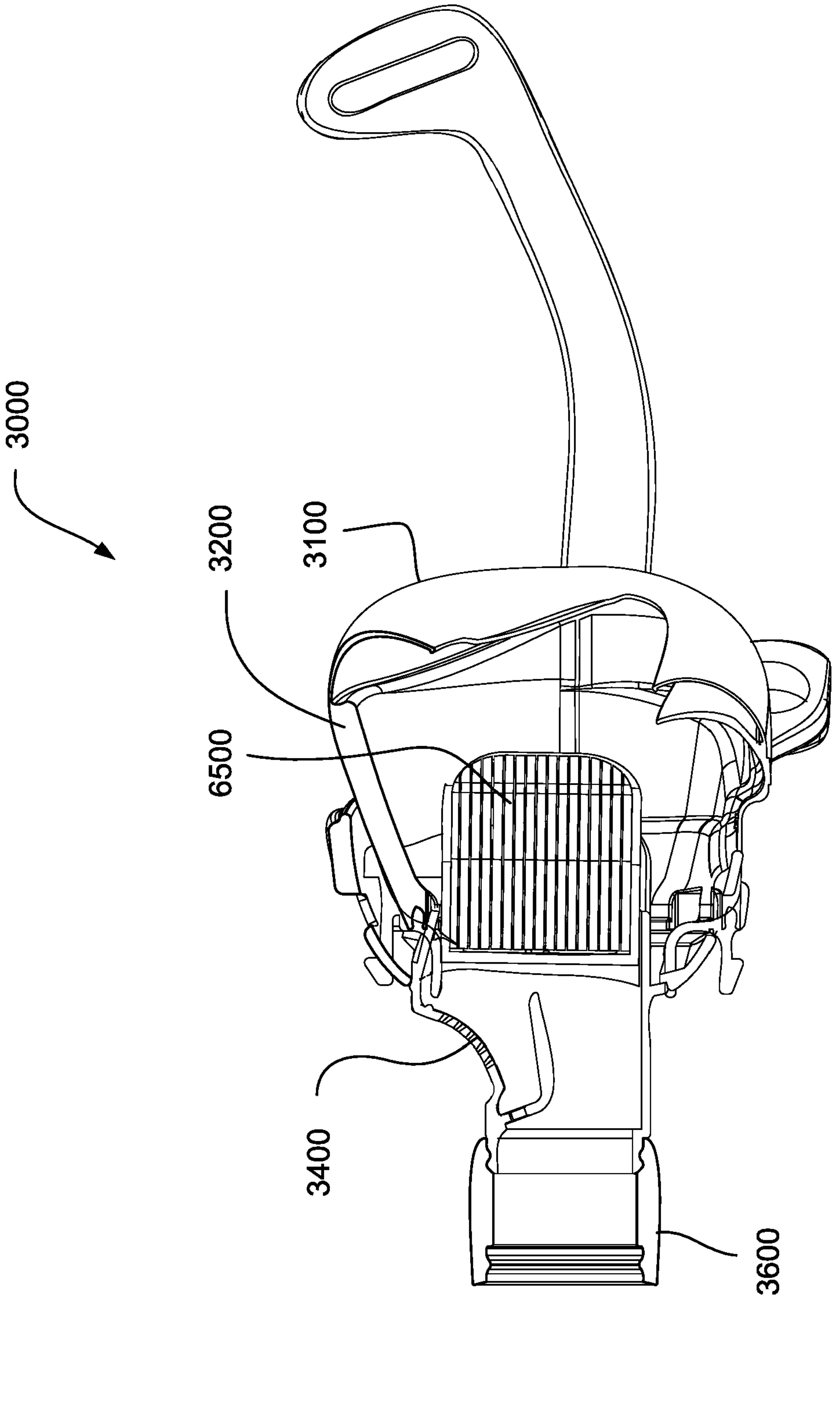


FIG. 15

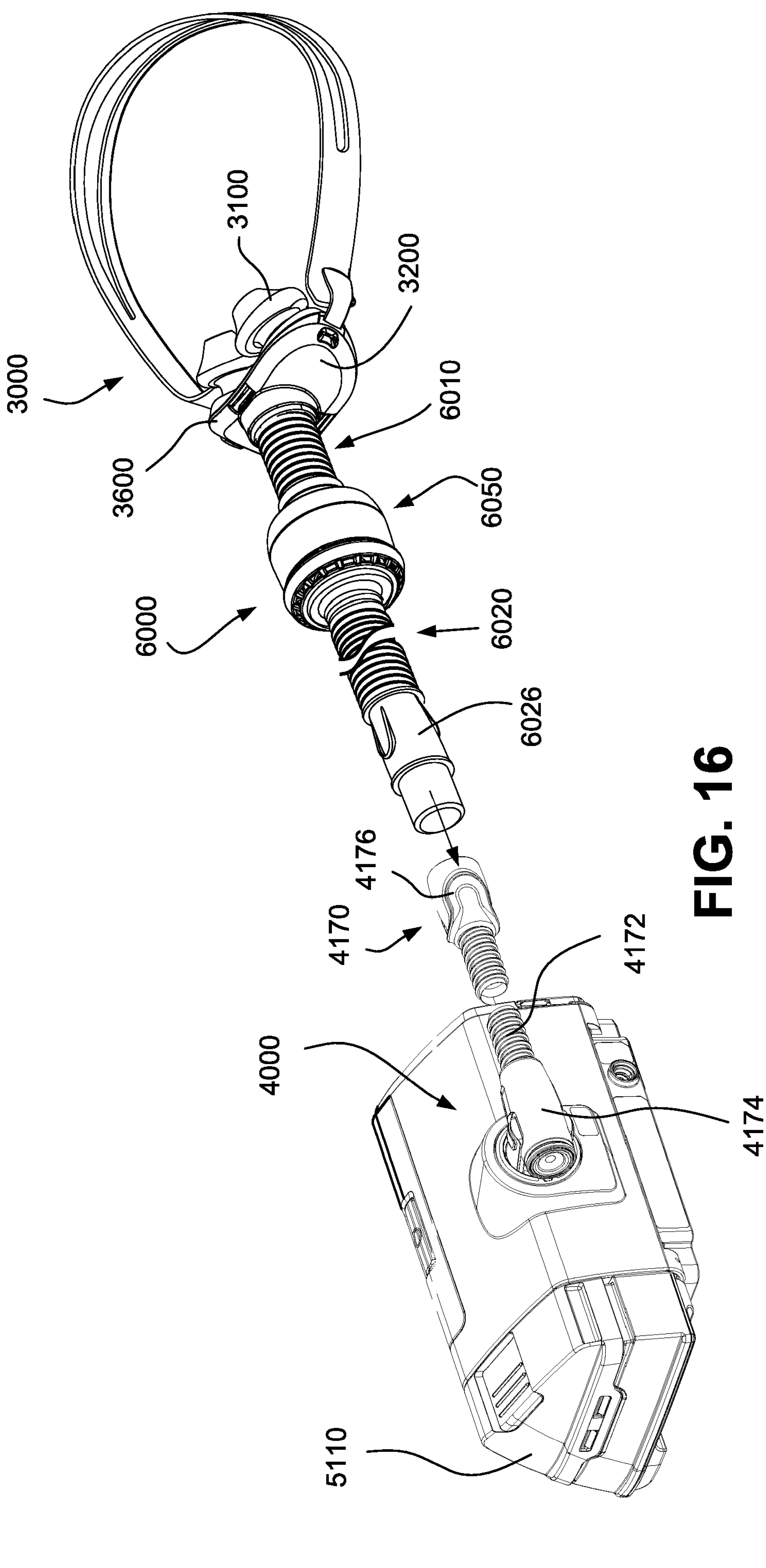
3000
3100
3200
6010
3600
6050
6000
6020
6026
4176
4170
4172
4000
4174
5110

FIG. 16

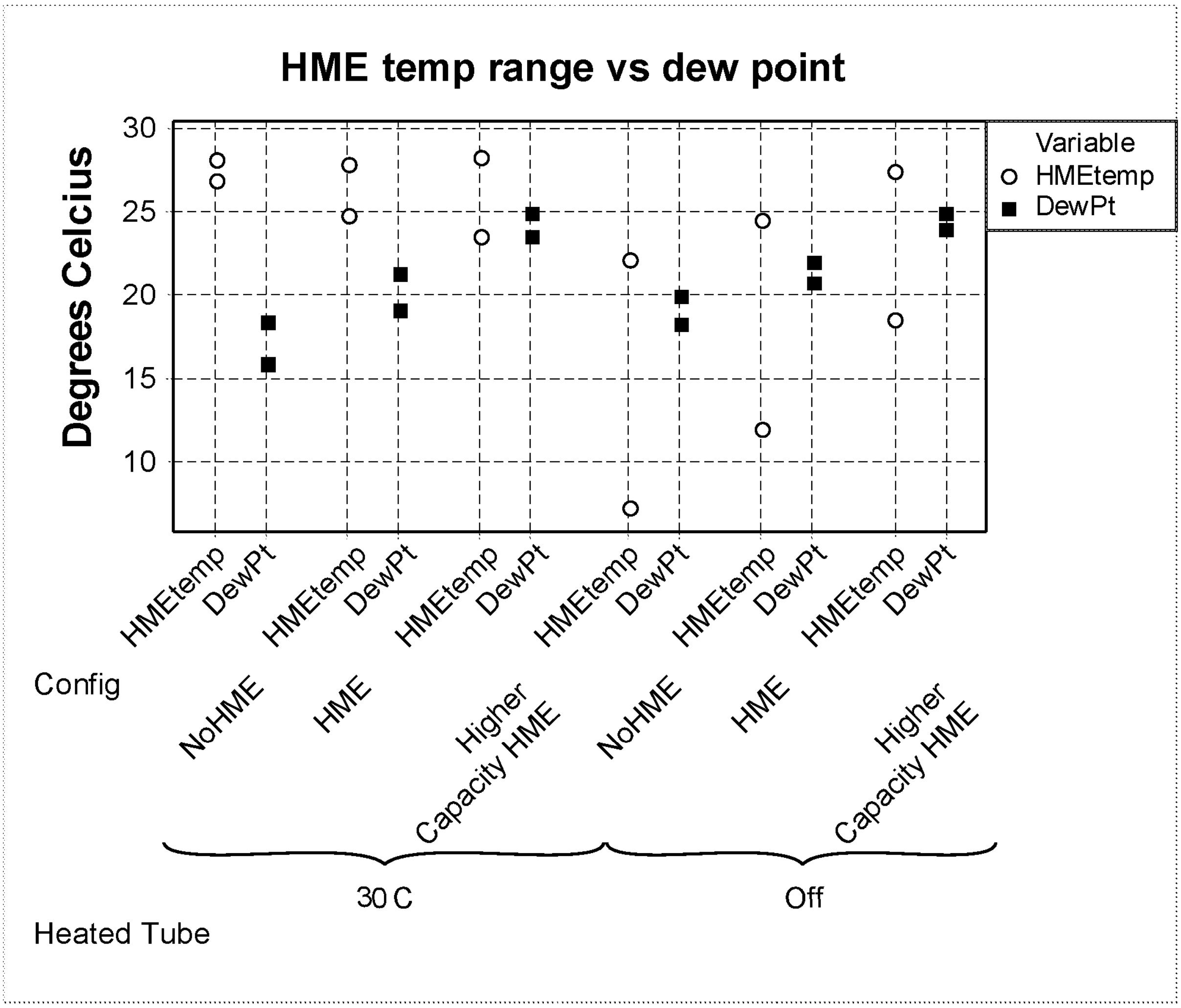
HME temp range vs dew point
Degrees Celcius
30
25
20
15
10
Variable
HMEtemp
DewPt
HMEtemp
DewPt
HMEtemp
DewPt
HMEtemp
DewPt
HMEtemp
DewPt
HMEtemp
DewPt
HMEtemp
DewPt
Config
NoHME
HME
Higher Capacity HME
NoHME
HME
Higher Capacity HME
30 C
Off
Heated Tube

FIG. 17

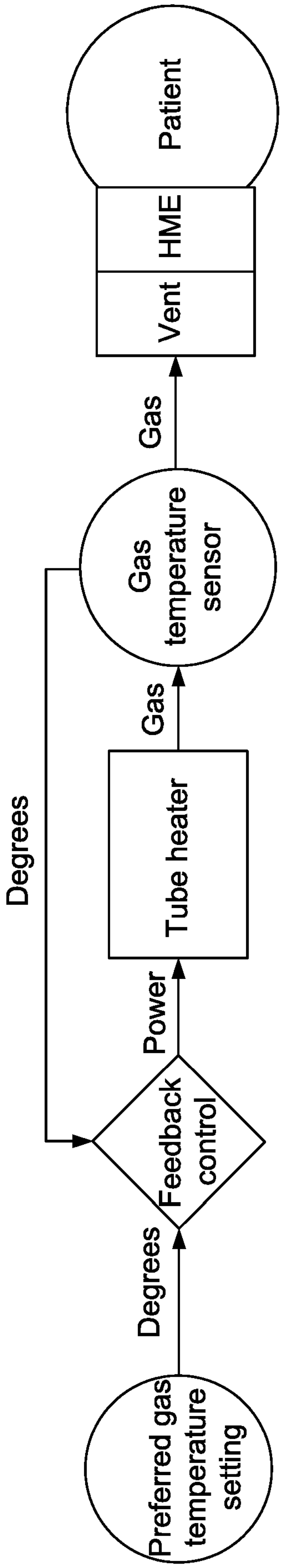

Preferred gas temperature setting
Degrees
Feedback control
Degrees
Power
Tube heater
Gas
Gas temperature sensor
Gas
Vent
HME
Patient


FIG. 18

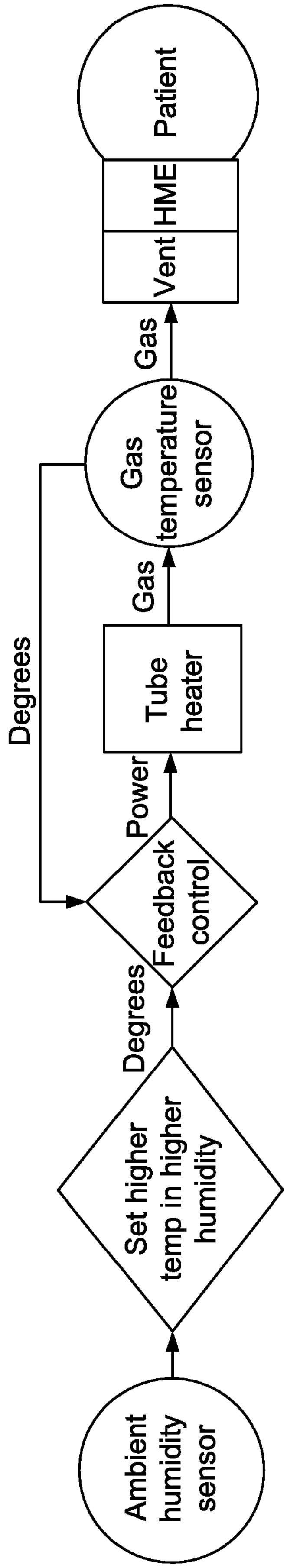

Ambient humidity sensor
Set higher temp in higher humidity
Degrees
Feedback control
Degrees
Power
Tube heater
Gas
Gas temperature sensor
Gas
Vent
HME
Patient


FIG. 19

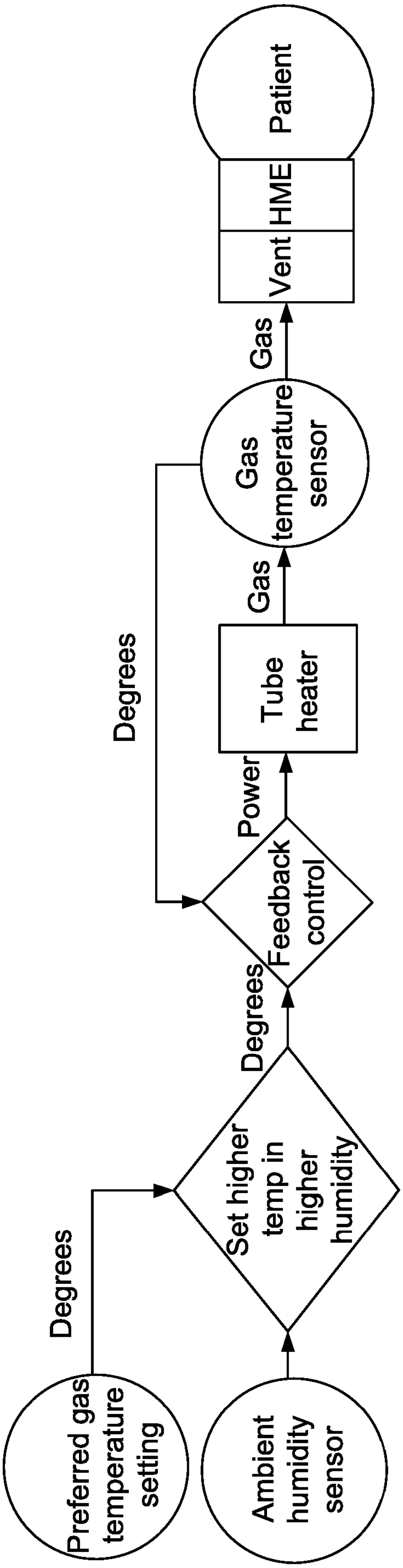
Preferred gas temperature setting
Ambient humidity sensor
Degrees
Set higher temp in higher humidity
Degrees
Feedback control
Power
Tube heater
Gas
Gas temperature sensor
Gas
Vent
HME
Patient
Degrees

FIG. 20

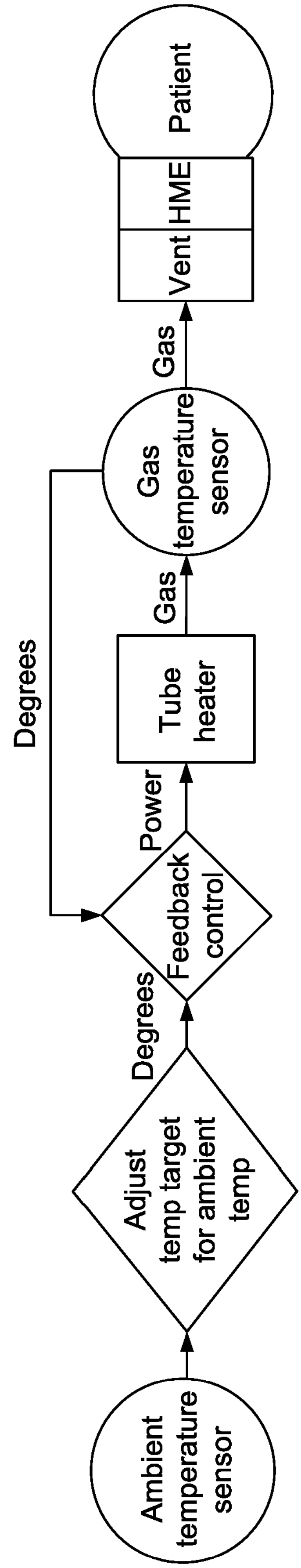
Ambient temperature sensor
Adjust temp target for ambient temp
Degrees
Feedback control
Power
Tube heater
Gas
Gas temperature sensor
Gas
Vent
HME
Patient
Degrees

FIG. 21

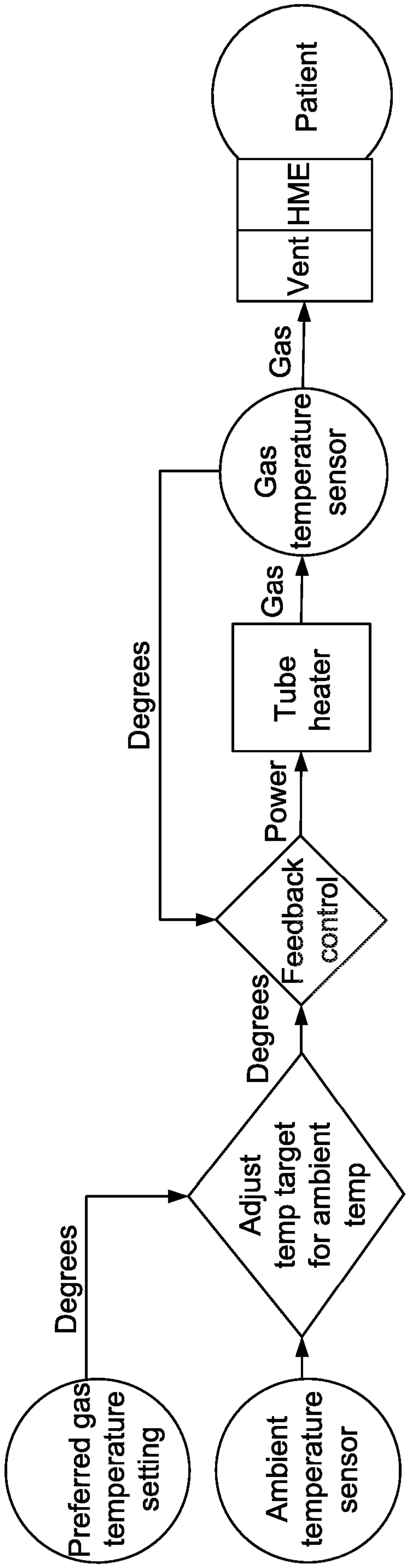
Preferred gas temperature setting
Ambient temperature sensor
Degrees
Adjust temp target for ambient temp
Degrees
Feedback control
Degrees
Power
Tube heater
Gas
Gas temperature sensor
Gas
Vent
HME
Patient

FIG. 22

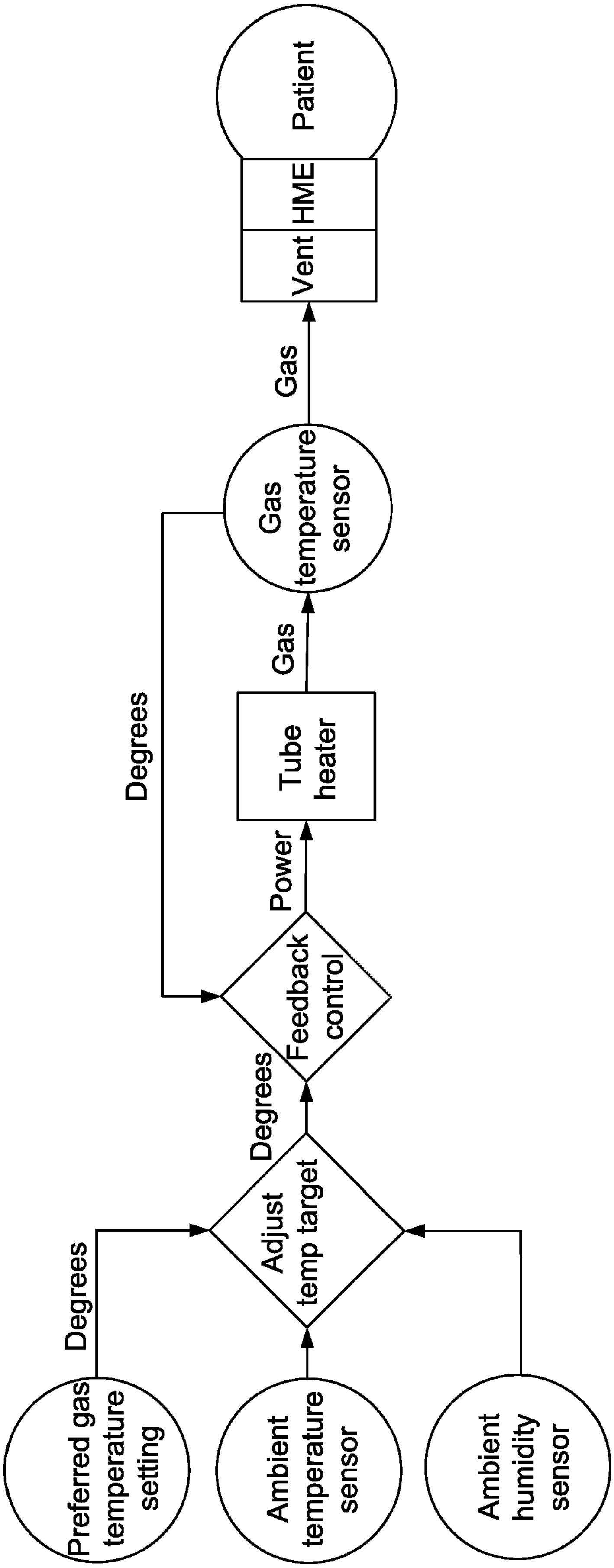
Preferred gas temperature setting
Ambient temperature sensor
Ambient humidity sensor
Degrees
Adjust temp target
Degrees
Feedback control
Degrees
Power
Tube heater
Gas
Gas temperature sensor
Gas
Vent
HME
Patient

FIG. 23

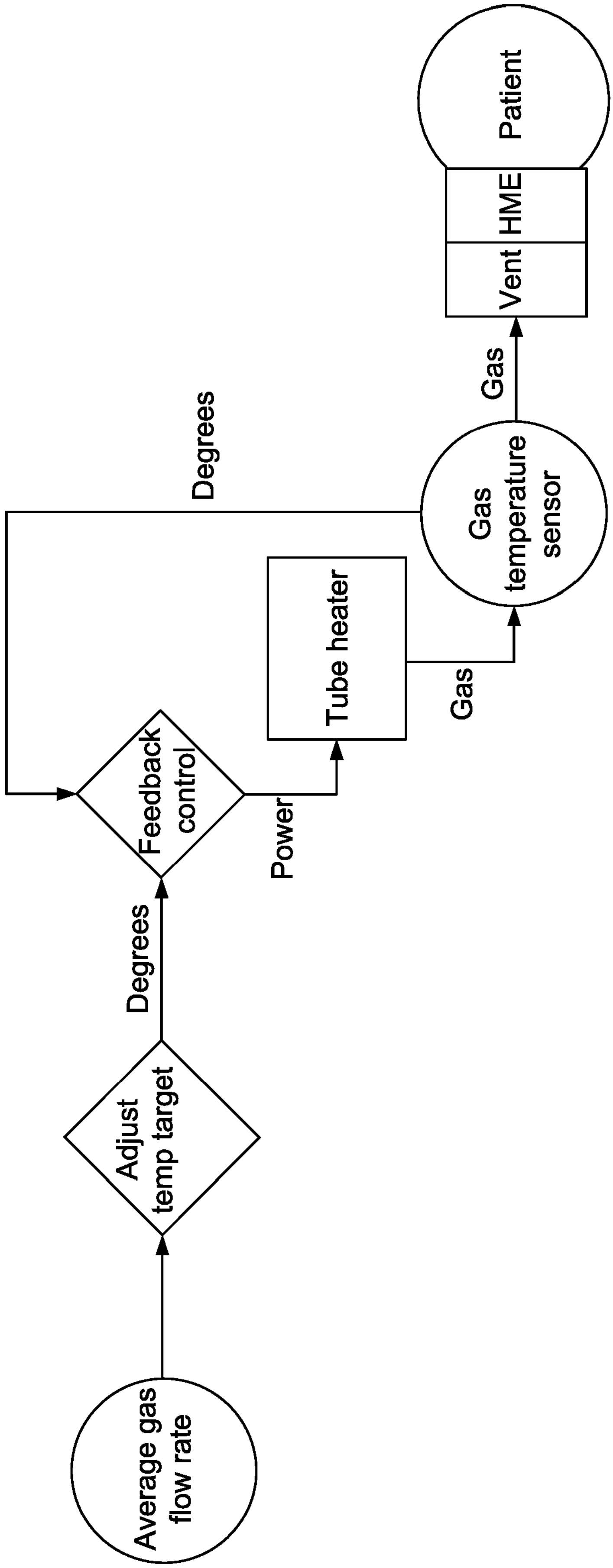
Average gas flow rate
Adjust temp target
Degrees
Feedback control
Degrees
Power
Tube heater
Gas
Gas temperature sensor
Gas
Vent
HME
Patient

FIG. 24

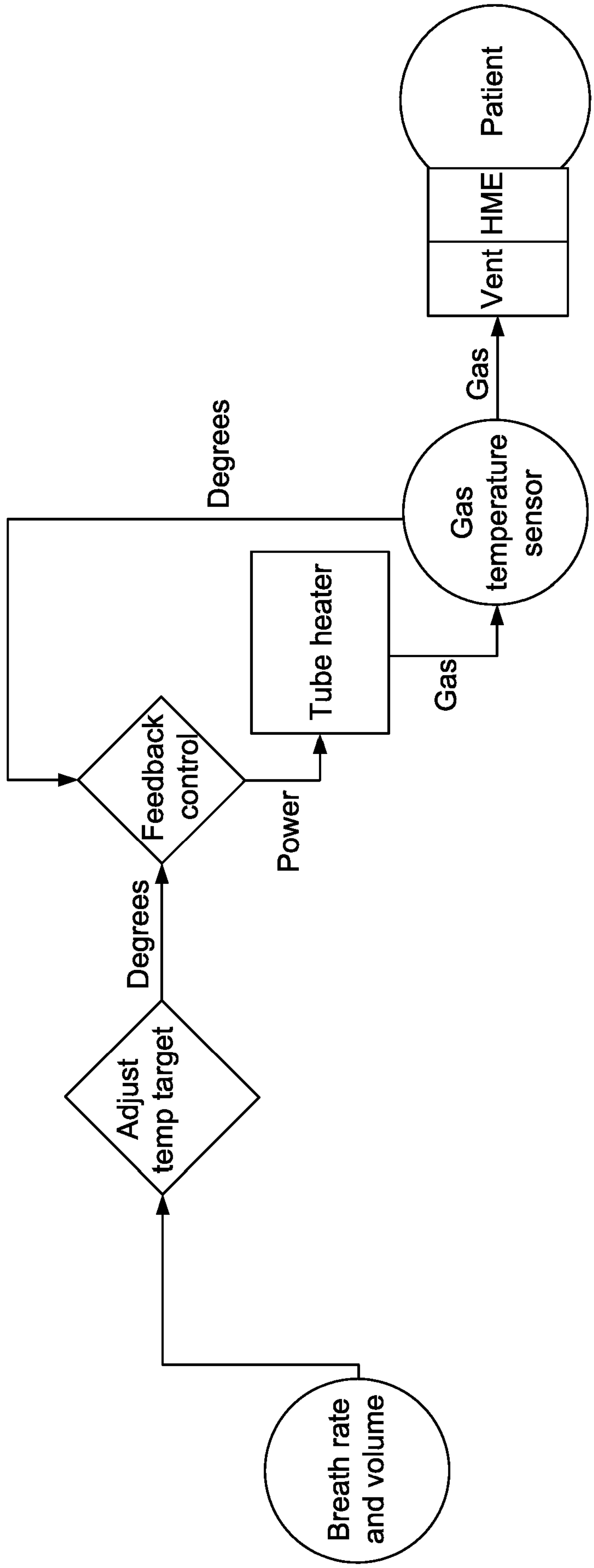
Patient
HME
Vent
Gas
Degrees
Gas temperature sensor
Tube heater
Gas
Feedback control
Power
Degrees
Adjust temp target
Breath rate and volume

FIG. 25

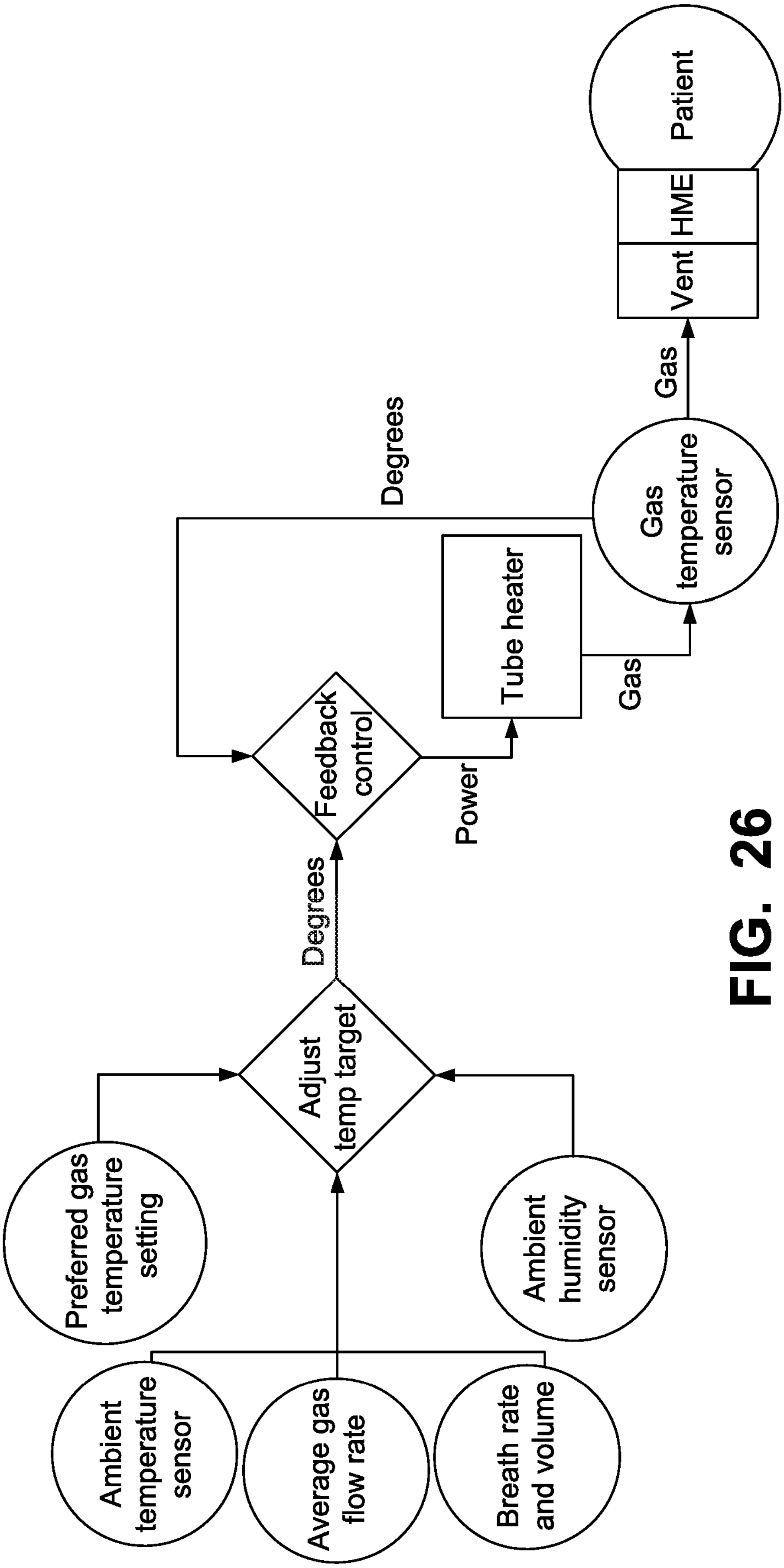
Ambient temperature sensor
Average gas flow rate
Breath rate and volume
Preferred gas temperature setting
Ambient humidity sensor
Adjust temp target
Degrees
Feedback control
Degrees
Power
Tube heater
Gas
Gas temperature sensor
Gas
Vent
HME
Patient

FIG. 26

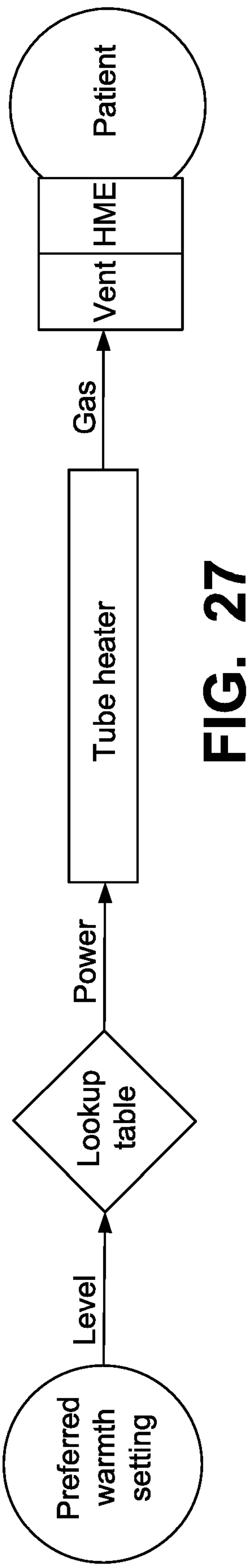
Preferred warmth setting
Level
Lookup table
Power
Tube heater
Gas
Vent
HME
Patient

FIG. 27

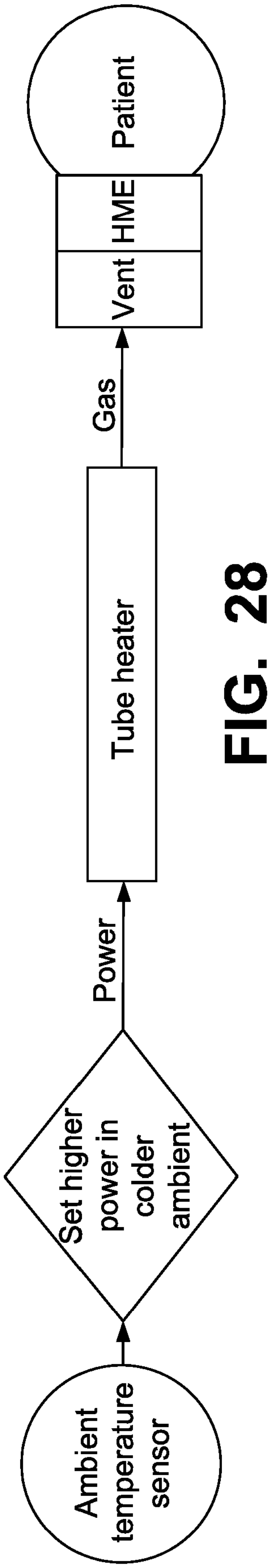
Ambient temperature sensor
Set higher power in colder ambient
Power
Tube heater
Gas
Vent
HME
Patient

FIG. 28

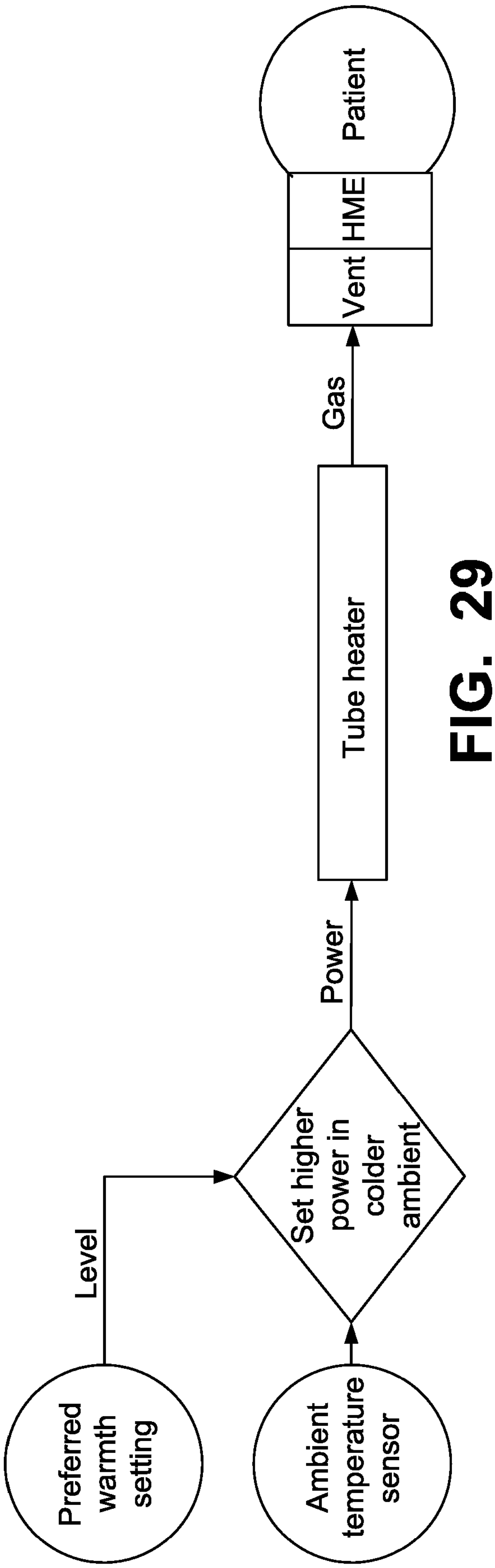
Preferred warmth setting
Level
Ambient temperature sensor
Set higher power in colder ambient
Power
Tube heater
Gas
Vent
HME
Patient

FIG. 29

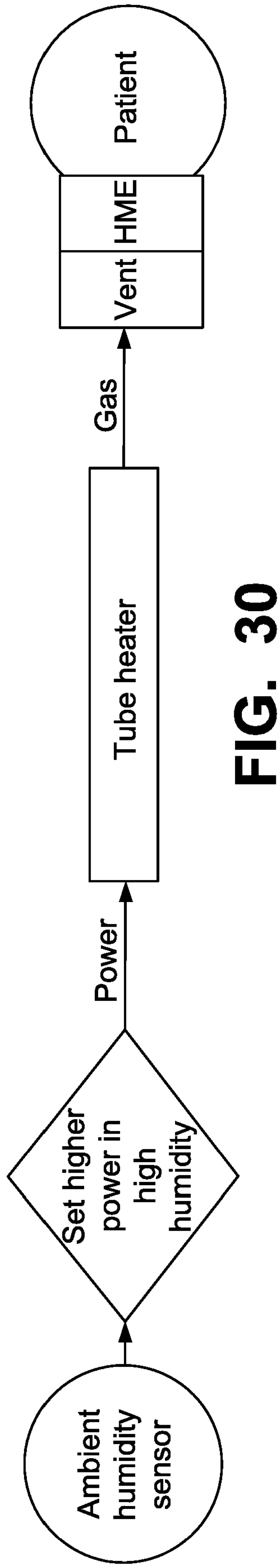
Ambient humidity sensor
Set higher power in high humidity
Power
Tube heater
Gas
Vent
HME
Patient

FIG. 30

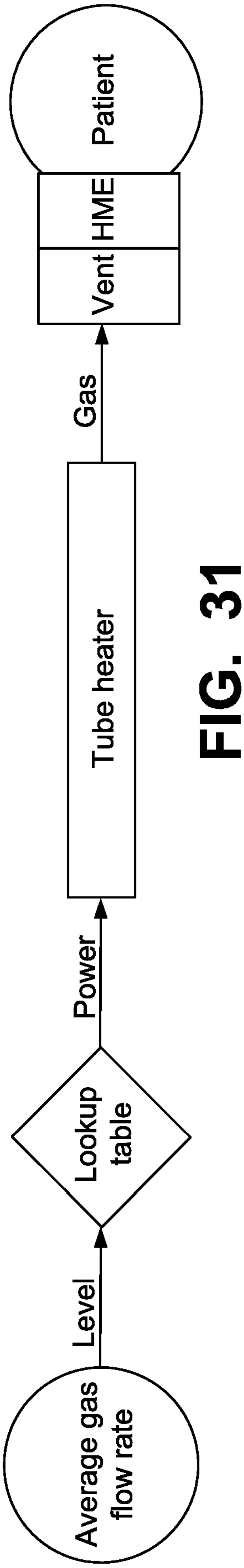
Average gas flow rate
Level
Lookup table
Power
Tube heater
Gas
Vent
HME
Patient

FIG. 31

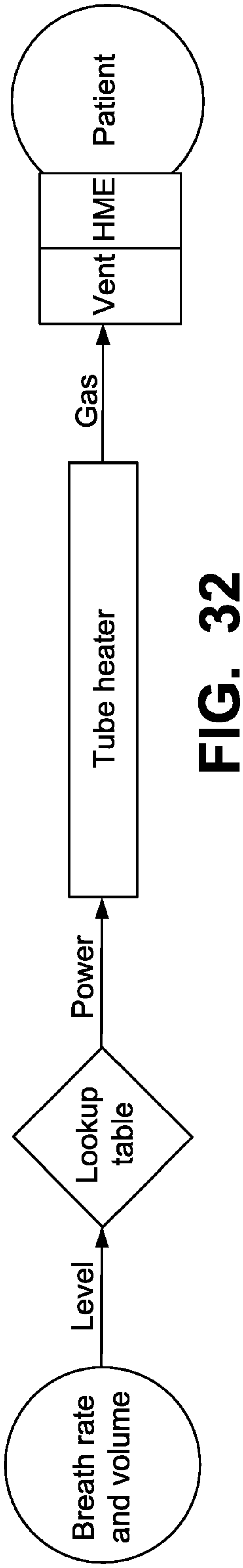
Breath rate and volume
Level
Lookup table
Power
Tube heater
Gas
Vent
HME
Patient

FIG. 32

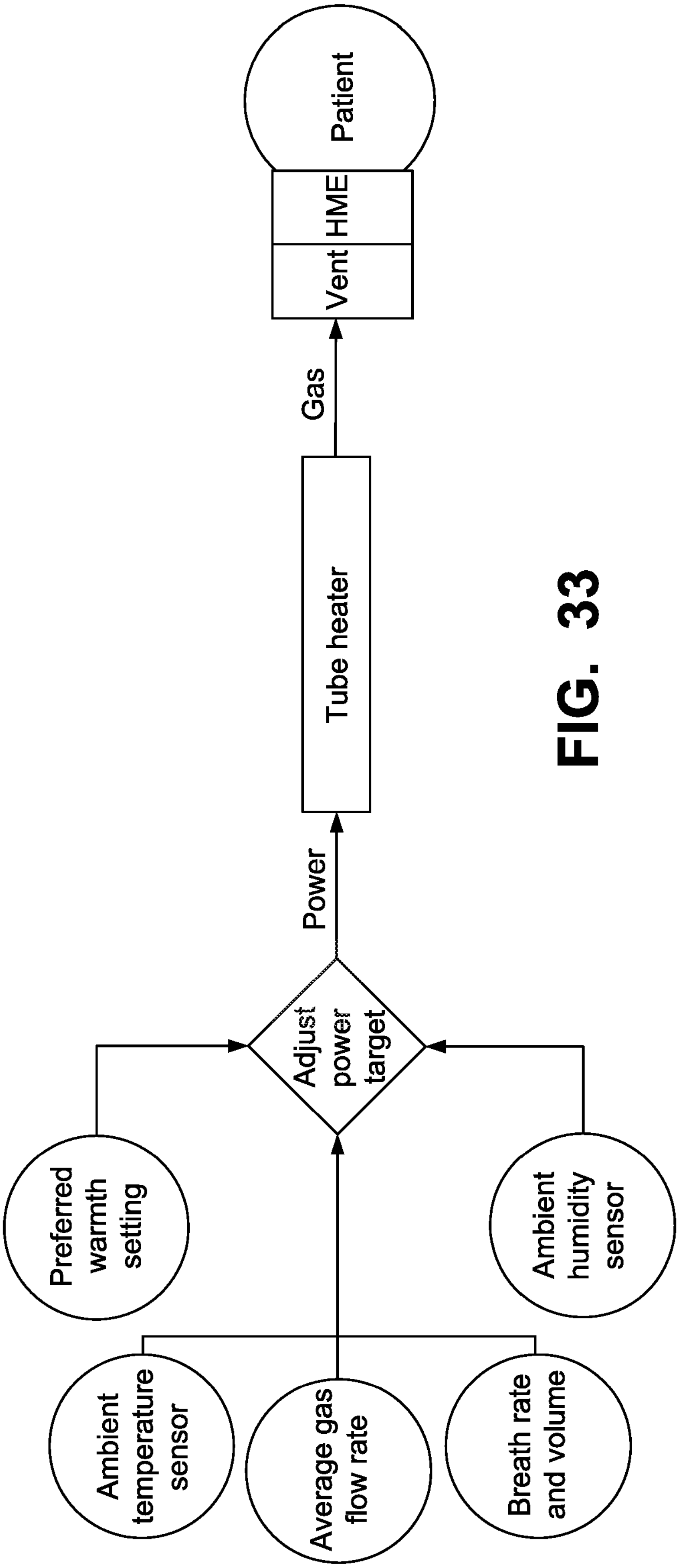
Ambient temperature sensor
Average gas flow rate
Breath rate and volume
Preferred warmth setting
Ambient humidity sensor
Adjust power target
Power
Tube heater
Gas
Vent
HME
Patient

FIG. 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4944310 A, Sullivan
- US 6532959 B, Berthon-Jones
- WO 1998004310 A
- WO 2006074513 A
- WO 2010135785 A
- US 4782832 A, Trimble
- WO 2004073778 A
- US 20090044808 A
- WO 2005063328 A
- WO 2006130903 A
- WO 2009052560 A
- US 20100000534 A
- WO 1998034665 A
- WO 2000078381 A
- US 6581594 B
- US 20090050156 A
- US 20090044808
- WO 2017004664 A1
- EP 3360594 A1
- US 2012304985 A1
- GB 2277689 A
- WO 2018035579 A1
- WO 2015089582 A
- US 7866944 B
- US 8638014 B
- US 8636479 B
- WO 2013020167 A
- US 8733349 B
- US 9903371 B
- WO 2018126295 A
- WO 2015013761 A
- WO 201217072 A

### Non-patent literature cited in the description

- **JOHN B. WEST**. Respiratory Physiology. Lippincott Williams & Wilkins, 2012